# EUROPEAN PATENT APPLICATION

(11) **EP 1 577 316 A1**
(43) Date of publication of application: **21.09.2005**
(21) Application number: 03768216.8
(22) Date of filing: 25.12.2003
(51) Int. Cl.: C07H 15/26, C08B 37/00, G01N 30/48

(54) **SUGAR-CHAIN ASPARAGINE DERIVATIVES AND PROCESSES FOR THE PREPARATION THEREOF**

(30) Priority: 26.12.2002 JP 2002377819
(71) Applicant: Kajihara, Yasuhiro, Yokohama-shi, Kanagawa 224-0014 (JP); OTSUKA CHEMICAL COMPANY, LIMITED, Osaka 540-0021 (JP)
(72) Inventor: KAJIHARA, Yasuhiro, Yokohama-shi, Kanagawa 224-0014 (JP); KAKEHI, Kazuaki, Nara-shi, Nara 630-8113 (JP); FUKAE, Kazuhiro, c/o OTSUKA CHEMICAL CO., LTD., Tokushima-shi, Tokushima 771-0193 (JP)
(74) Representative: Barz, Peter
(86) International application number: PCT/JP2003/016682
(87) International publication number: WO 2004/058789

(57) **Abstract**

An asparagine-linked oligosaccharide of the formula (1) given below having undeca- to tri-saccharides 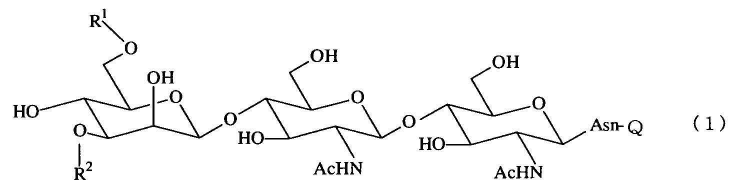 wherein R¹ and R² are each a hydrogen atom or a group of the formulae (2) to (6) disclosed in the specification and may be the same or different, and Q is a biotin group or FITC group,
an asparagine-linked oligosaccharide derivative containing at least one fucose in N-acetylglucosamine on the nonreducing terminal side of an asparagine-linked oligosaccharide wherein the amino group of asparagine is modified with a biotin group or FITC group,
a microplate having immobilized thereto a biotinated asparagine-linked oligosaccharide, and
an affinity column having immobilized thereto a biotinated asparagine-linked oligosaccharide.

## Description

### TECHNICAL FIELD

The present invention relates to biotinated asparagine-linked oligosaccharides, fluoresceinisothiocyanated (FITC-bonded) asparagine-linked oligosaccharides, a processs for proparing such a compound, and the use thereof.

### BACKGROUND ART

In recent years, molecules of oligosaccharides have attracted attention as third chain life molecules following nucleic acids (DNA) and proteins. The human body is a huge cell society comprising about 60 trillion cells, and the surfaces of all the cells are covered with oligosaccharide molecules. For example, ABO blood groups are determined according to the difference of oligosaccharides over the surfaces of cells.

Oligosaccharides function in connection with the recognition of cells and interaction of cells and are key substances for the establishment of the cell society. Disturbances in the cell society lead, for example, to cancers, chronic diseases, infectious diseases and aging.

For example, it is known that when cells cancerate, changes occur in the structure of oligosaccharides. It is also known that *Vibrio cholerae,* influenza virus, etc. ingress into cells and cause infection by recognizing and attaching to a specific oligosaccharide.

Oligosaccharides are much more complex than DNA or proteins in structure because of the diversity of arrangements of monosaccharides, modes or sites of linkages, lengths of chains, modes of branches and overall structures of higher order. Accordingly, biological information derived from the structures thereof is more diversified than is the case with DNA and proteins. Although the importance of research on oligosaccharides has been recognized, the complexity and variety of structures thereof have delayed progress in the research on oligosaccharides unlike the studies on DNA and proteins.

To clarify whether a protein has ability to recognize and bond to a specific oligosaccharide leads to the development of pharmaceuticals and food products based on a novel principle, contributing to the prevention or treatment of diseases and expectedly entailing wide application.

Accordingly, utilizing the specificity of biotin-avidin bond, oligosaccharide microchips can be produced merely by reacting a plurality of biotinated oligosaccharides on an avidinated microplate. This makes it possible to clarify proteins having ability to bond to a specific oligosaccharide.

In order to isolate and purify a specific protein, a specific biotinated oligosaccharide is bonded and immobilized to an avidinated affinity column, and a mixture containing a protein having ability to specifically bond to the biotinated oligosaccharide is passed through the column, whereby the desired protein only can be isolated.

An FITC-bonded asparagine-linked oligosaccharide can be isolated as by capillary electrophoresis even in a very small amount. Furthermore, the asparagine-linked oligosaccharide itself can be isolated by removing the FITC.

An object of the present invention is to provide an asparagine-linked oligosaccharide wherein the amino group of asparagine is biotinated or bonded to FITC, a process for preparing the same and the use of the same.

### DISCLOSURE OF THE INVENTION

The present invention provides the following inventions.
1. An asparagine-linked oligosaccharide of the formula (1) given below having undeca- to tri-saccharides 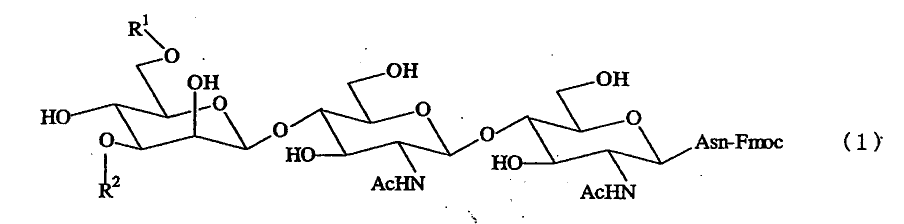 wherein R¹ and R² are each a hydrogen atom or a group of the formulae (2) to (6) and may be the same or different, and Q is a biotin group or FITC group. 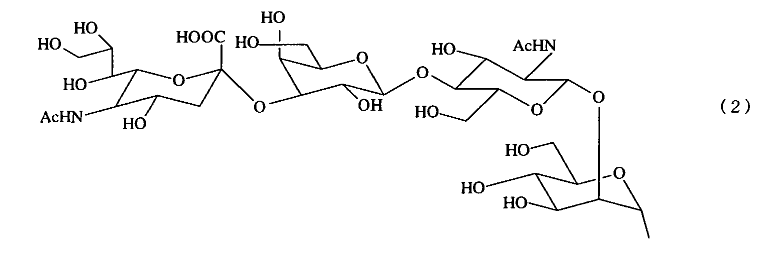 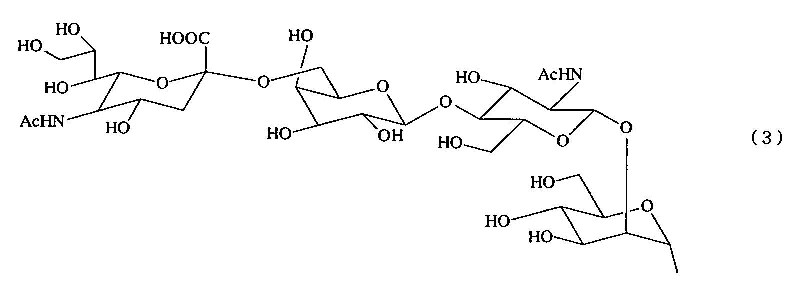 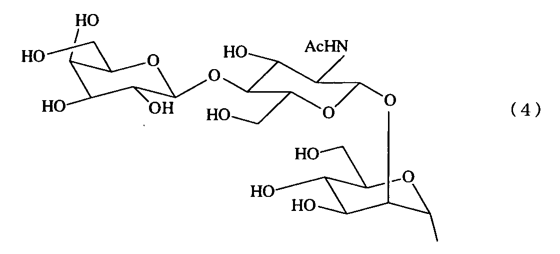 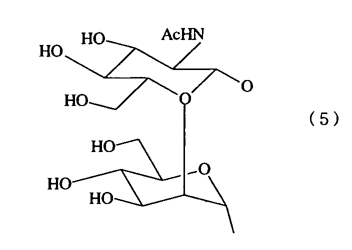 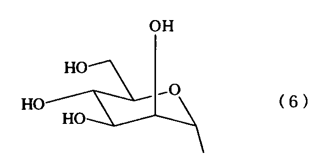
2. An asparagine-linked oligosaccharide derivative containing at least one fucose in N-acetylglucosamine on the nonreducing terminal side of an asparagine-linked oligosaccharide wherein the amino group of asparagine is modified with a biotin group or FITC group.
3. A process for preparing a biotinated asparagine-linked oligosaccharide characterized in that an asparagine-linked oligosaccharide of the formula (9) having undeca- to tri-saccharides is biotinated 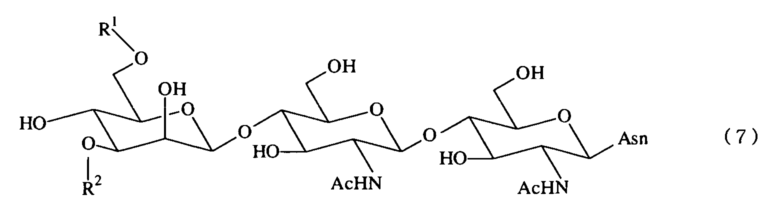 wherein R¹ and R² are as defined above.
4. A process for preparing a FITC-bonded asparagine-linked oligosaccharide characterized in that an asparagine-linked oligosaccharide of the formula (9) having undeca- to tri-saccharides is fluoresceinisothiocyanated (FITC-bonded).
5. A microplate having immobilized thereto the above biotinated asparagine-linked oligosaccharide.
6. An affinity column having immobilized thereto the above biotinated asparagine-linked oligosaccharide.

The present inventor has already developed, as disclosed in Japanese Patent Application No. 2001-185685 (hereinafter referred to as the "prior application"), processes for preparing asparagine-linked oligosaccharides derivative, asparagine-linked oligosaccharides and oligosaccharides which processes are capable of producing various isolated asparagine-linked oligosaccharides derivative with greater ease and in larger quantities than conventionally, and further novel asparagine-linked oligosaccharides derivative, asparagine-linked oligosaccharides and oligosaccharides, wherein oligosaccharides deficient in sugar moieties as desired are linked.

The processes of the prior application include:
(1) a process for preparing an asparagine-linked oligosaccharide derivative derived from an asparagine-linked oligosaccharide which process includes the steps of:
   (a) introducing a lipophilic (hydrophobic) protective group into an asparagine-linked oligosaccharide or at least two asparagine-linked oligosaccharides included in a mixture comprising the oligosaccharide or said at least two oligosaccharides to obtain an asparagine-linked oligosaccharide derivative mixture, and
   (b) hydrolyzing the asparagine-linked oligosaccharide derivative mixture or asparagine-linked oligosaccharides derivative included in this mixture and subjecting the resulting mixture to chromatography to separate off asparagine-linked oligosaccharides derivative,
(2) a process for preparing an asparagine-linked oligosaccharide derivative according to (1) which further includes the step (b') of hydrolyzing the asparagine-linked oligosaccharides derivative separated off by the step (b) with a glycosidase,
(3) a process for preparing an asparagine-linked oligosaccharide derivative according to (1) or (2) wherein the mixture comprising the oligosaccharide or said at least two oligosaccharides includes a compound of the formula (A) below and/or a compound corresponding to said compound wherein at least one sugar moiety is deficient,
(4) a process for preparing an asparagine-linked oligosaccharide derivative according to any one of (1) to (3) wherein the lipophilic protective group is a fluorenylmethoxycarbonyl (Fmoc) group,
(5) a process for preparing an asparagine-linked oligosaccharide derivative according to any one of (1) to (3) wherein the step (a) is the step of introducing Fmoc group into the asparagine-linked oligosaccharide or said at least two asparagine-linked oligosaccharides having a sialic moiety at a nonreducing terminal and included in the mixture, and introducing benzyl group into the sialic moiety to obtain the asparagine-linked oligosaccharide derivative mixture,
(6) A process for preparing an asparagine-linked oligosaccharide including the steps of:
   (a) introducing a lipophilic protective group into an asparagine-linked oligosaccharide or at least two asparagine-linked oligosaccharides included in a mixture comprising the oligosaccharide or said at least two oligosaccharides to obtain an asparagine-linked oligosaccharide derivative mixture,
   (b) hydrolyzing the asparagine-linked oligosaccharide derivative mixture or asparagine-linked oligosaccharides derivative included in this mixture and subjecting the resulting mixture to chromatography to separate off asparagine-linked oligosaccharides derivative, and
   (c) removing the protective group from the asparagine-linked oligosaccharides derivative separated off in the step (b) to obtain asparagine-linked oligosaccharides,
(7) a process for preparing an asparagine-linked oligosaccharide according to (6) which further includes:
   the step (b') of hydrolyzing the asparagine-linked oligosaccharides derivative separated off by the step (b) with a glycosidase, and/or
   the step (c') of hydrolyzing the asparagine-linked oligosaccharides obtained by the step (c) with a glycosidase,
(8) a process for preparing an asparagine-linked oligosaccharide according to (6) or (7) wherein the mixture comprising the oligosaccharide or said at least two oligosaccharides includes a compound of the formula (A) below and/or a compound corresponding to said compound wherein at least one sugar moiety is deficient,
(9) a process for preparing an asparagine-linked oligosaccharide according to any one of (6) to (8) wherein the lipophilic protective group is Fmoc group.
(10) a process for preparing an asparagine-linked oligosaccharide according to any one of (6) to (8) wherein the step (a) is the step of introducing Fmoc group into the asparagine-linked oligosaccharide or said at least two asparagine-linked oligosaccharides having a sialic moiety at a nonreducing terminal and included in the mixture, and introducing benzyl group into the sialic moiety to obtain the asparagine-linked oligosaccharide derivative mixture, etc.

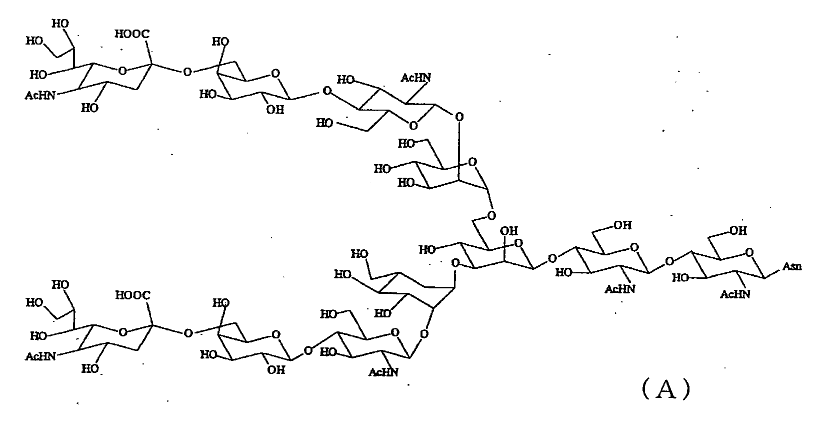

Since a detailed description is given in the prior application about the preparation of these asparagine-linked oligosaccharide derivatives and asparagine-linked oligosaccharides, reference will be made to the application. However, what is disclosed in the prior application will be described to some extent. The process of the prior application for preparing asparagine-linked oligosaccharides derivative is distinctly characterized in that a lipophilic protective group is introduced into (linked with) a asparagine-linked oligosaccharide derived from a naturally occurring glycoprotein, preferably asparagine-linked oligosaccharides included in a mixture of asparagine-linked oligosaccharides obtained from oligosaccharides capable of linking to asparagine, to obtain a mixture of asparagine-linked oligosaccharides derivative, followed by separation of the mixture into individual asparagine-linked oligosaccharides derivative. The term an "asparagine-linked oligosaccharide" as used herein refers to an oligosaccharide having asparagine linked thereto. Further the term "oligosaccharides capable of linking to asparagine" refers to a group of oligosaccharides wherein N-acetylglucosamine present at a reducing terminal is attached by N-glucoside linkage to the acid amino group of asparagine (Asn) in the polypeptide of a protein and which has Man(β1-4)GlcNac(β1-4)GlcNac as the core structure. The term an "asparagine-linked oligosaccharide derivative" refers to an asparagine-linked oligosaccharide wherein a lipophilic protective group is attached to asparagine moiety. Further "AcHN" in the structural formulae of compounds refers to an acetamido group.

As described previously, oligosaccharides derived from naturally occurring glycoproteins are a mixture of oligosaccharides which are randomly deficient in the sugar moiety at the nonreducing terminal. The present inventors have unexpectedly found that the introduction of a lipophilic protective group into an oligosaccharide derived from a naturally occurring glycoprotein, preferably into asparagine-linked oligosaccharides included in a mixture thereof, makes it possible to readily separate a mixture of asparagine-linked oligosaccharides derivative having the protective group introduced therein into individual asparagine-linked oligosaccharides derivative by a known chromatographic procedure. Consequently, asparagine-linked oligosaccharides derivative having different structures can be prepared individually in large quantities. For example, asparagine-linked oligosaccharides derivative which resemble in structure and which are conventionally difficult to separate can be separated from one another, and these compounds can be prepared easily in large quantities. Further a glycosidase can be caused to act on the resulting asparagine-linked oligosaccharides derivative and thereby prepare various asparagine-linked oligosaccharides derivative.

Thus, introducing a lipophilic protective group into asparagine-linked oligosaccharides provides derivatives and makes it possible to separate the asparagine-linked oligosaccharides derivative from one another. Presumably this is attributable to the fact that the introduction of the lipophilic protective group gives improved lipophilicity (hydrophobicity) to the whole asparagine-linked oligosaccharides derivative to ensure remarkably improved interaction between the oligosaccharide and the reverse-phase column to be used favorably, consequently separating the asparagine-linked oligosaccharides derivative from one another by reflecting the difference of structure between the oligosaccharides with high sensitivity.

Further by removing the protective group from the asparagine-linked oligosaccharides derivative obtained, various asparagine-linked oligosaccharides can be artificially prepared easily in large amounts according to the prior application.

The asparagine-linked oligosaccharide derivative, the asparagine-linked oligosaccharide and the oligosaccharide obtained by the invention of the above-mentioned prior application are all α2,6-bonded compounds.

Furthermore, the asparagine-linked oligosaccharide derivative, asparagine-linked oligosaccharide and oligosaccharide obtained by the invention of the above-mentioned prior application are all compounds wherein the oligosaccharide has no fucose linked thereto.

The difference between α2,3-bonded compounds and α2,6-bonded compounds will be described below.

The α2,3-bonded compound and the α2,6-bonded compound represent modes of bonding between sialic acid and galactose. The former refers to a compound wherein the carbon at the 2-position of sialic acid and the carbon at the 3-position of galactose are linked by α-bonding. The latter refers to a compound wherein the carbon at the 2-position of sialic acid and the carbon at the 6-position of galactose are linked by α-bonding. Thus, the difference resides in the difference in the carbon-to-carbon bond between sialic acid and galactose.

According to the process of the invention, an asparagine-linked oligosaccharide (nonasaccharide-Asn-Fmoc) protected with a lipophilic protective group and serving as the starting material is reacted with sialic acid transferase to transfer sialic acid or a sialic acid derivative to the oligosaccharide, and the resulting asparagine-linked oligosaccharide protected with the lipophilic protective group is subjected to chromatography for separation to obtain an asparagine-linked disialooligosaccharide derivative protected with the lipophilic protective group and two kinds of asparagine-linked monosialooligosaccharide derivatives.

The asparagine-linked disialooligosaccharide derivative and two kinds of asparagine-linked monosialooligosaccharide derivatives obtained are then subjected to sugar hydrolysis to obtain asparagine-linked nona- to hepta-saccharide derivatives having sialic acid or a sialic acid derivative.

The asparagine-linked undeca- to hepta-saccharide derivative or asparagine-linked disialooligosaccharide (α2,6-undecasaccharide-Asn-Fmoc) obtained above are subjected to sugar hydrolysis as starting materials to obtain asparagine-linked deca- to hexa-saccharide derivaties, to which fucose is transferred using sugar transferase to obtain asparagine-linked trideca- to hepta-saccharide derivatives containing fucose.

The protecting group is not particularly limited, and there can be used, for instance, a carbonate-based or amide-based protecting group, such as Fmoc group, t-butyloxycarbonyl (Boc) group, benzyl group, allyl group, allyloxycarbonate group, or acetyl group. From the viewpoint that the resulting asparagine-linked oligosaccharide derivative can be immediately used in the synthesis of a desired glycopeptide, the above protecting group is preferably Fmoc group, Boc group or the like, more preferably Fmoc group. The Fmoc group is especially effective when there exists in the oligosaccharide a sugar, such as sialic acid, which is relative unstable under acidic conditions. The introduction of the protecting group may be carried out according to a known process (for instance, Protecting Groups in Organic Chemistry, John Wiley & Sons INC., New York 1991, ISBN 0-471-62301-6).

For instance, when Fmoc group is used, an appropriate amount of acetone is added to the mixture containing asparagine-linked oligosaccharides, 9-fluorenylmethyl-N-succinimidyl carbonate and sodium hydrogencarbonate are further added thereto and dissolved, and thereafter the resulting mixture is subjected to a binding reaction of Fmoc group to an asparagine moiety at 25 °C, whereby the Fmoc group can be introduced into the asparagine moiety of the above asparagine-linked oligosaccharide.

According to the procedures described above, asparagine-linked oligosaccharide derivatives into which a lipophilic protecting group is introduced are obtained.

The sialic acid to be used is one generally available commercially, or one prepared by chemical synthesis.

Examples of sialic acid derivatives usable are those generally available commercially, or those prepared by chemical synthesis. More specific examples of such derivatives are those wherein the hydroxyl group attached to the carbon atom at the 7-position, 8-position or 9-position of sialic acid is substituted with a hydrogen atom or halogen atom. Examples of halogen atoms are fluorine, chlorine, bromine, etc., among which fluorine is preferred.

Examples of sialic acid transferases usable are those generally available commercially, those naturally occurring and those prepared by genetic recombination. A suitable transferase can be selected in accordance with the kind of sialic acid or sialic acid derivative to be transferred. A more specific example is one derived from a rat recombinant which is an α2,3-transferase, and one derived from rat liver which is an α2,6-transferase. Alternatively, sialic acid or a sialic acid derivative may be transferred using sialydase to shift equilibrium as by pH adjustment.

The separation of each of asparagine-linked oligosaccharide derivatives by chromatography can be carried out by appropriately using known chromatographies, singly or in a combination of plural chromatographies.

For instance, the resulting mixture of asparagine-linked oligosaccharide derivatives is purified by a gel filtration column chromatography, and then purified by using HPLC. The column which can be used in HPLC is preferably a reverse phase column, for instance, ODS, phenyl-based, nitrile-based, or anion exchange-based column, and concretely, a mono Q column manufactured by Pharmacia, Iatro-beads column manufactured by Iatron can be utilized. The separation conditions and the like may be adjusted by referring to a known condition. According to the above procedures, each of the desired asparagine-linked oligosaccharide derivatives can be obtained from the mixture of asparagine-linked oligosaccharide derivatives.

Furthermore, the asparagine-linked oligosaccharide derivative having a desired oligosaccharide structure can be efficiently obtained by hydrolyzing the asparagine-linked oligosaccharide derivatives separated in the above step. For instance, in the stage of separating the asparagine-linked oligosaccharide derivatives, the asparagine-linked oligosaccharide derivatives can be roughly separated by limiting the kinds of the asparagine-linked oligosaccharide derivatives contained in the mixture, and thereafter the asparagine-linked oligosaccharide derivatives are subjected to hydrolysis, for instance, hydrolysis with a glycosidase, whereby the asparagine-linked oligosaccharide derivatives having the desired oligosaccharide structures can be efficiently obtained. Here, the hydrolysis can be carried out in the same manner as described above. Especially, it is preferable that the hydrolysis is carried out with a glycosidase of which cleavage mode of the oligosaccharide moieties is clear, from the viewpoint of more efficiently obtaining the asparagine-linked oligosaccharide derivatives having the desired oligosaccharide structures.

For instance, the removal of the galactose moieties can be accomplished by dissolving the compounds to be hydrolysed in a buffer (for instance, phosphate buffer, acetate buffer, Good's buffer or the like), and carrying out cleavage reaction of the galactose moieties with a galactosidase in accordance with a known condition. The compounds to be hydrolysed may be individually isolated compounds or a mixture of these compounds. It is preferable that a commercially available known exo-type enzyme is utilized for the galactosidase used in this reaction. Also, the enzyme may be a newly isolated enzyme or an enzyme generated by genetic engineering, as long as the enzyme has a similar activity. Next, in the same manner as described above, the reaction solution obtained after the reaction (a mixture of asparagine-linked oligosaccharide derivatives of which sugar moieties are cleaved) may be subjected to chromatography to give each of asparagine-linked oligosaccharide derivatives. For instance, it is preferable that the separation is carried out by HPLC (ODS column, eluent being a 50 mM aqueous ammonium acetate: acetonitrile=82:18).

The removal of the N-acetylglucosamine moieties can be accomplished by dissolving the compounds to be hydrolysed in a buffer (for instance, phosphate buffer, acetate buffer, Good's buffer or the like), and carrying out cleavage reaction of the N-acetylglucosamine moieties with an N-acetylglucosaminidase in accordance with a known condition. Also, an N-acetylhexosaminidase can be used. The compounds to be hydrolysed may be individually isolated compounds or a mixture of these compounds. It is preferable that a commercially available known exo-type enzyme is utilized for each enzyme used in this reaction. Also, the enzyme may be a newly isolated enzyme or an enzyme generated by genetic engineering, as long as the enzyme has a similar activity. Next, in the same manner as described above, the reaction solution obtained after the reaction (a mixture of asparagine-linked oligosaccharide derivatives of which oligosaccharide moieties are cleaved) is subjected to chromatography to give each of asparagine-linked oligosaccharide derivatives. For instance, it is preferable that the separation is carried out by HPLC (ODS column, eluent being a 50 mM aqueous ammonium acetate:methanol=65:35 or a 50 mM aqueous ammonium acetate:acetonitrile=82:18).

The removal of the mannose moieties can be accomplished by dissolving the compounds to be hydrolysed in a buffer (for instance, phosphate buffer, acetate buffer, Good's buffer or the like), and carrying out cleavage reaction of the mannose moieties with a mannosidase under a known condition. The compounds to be hydrolysed may be individually isolated compounds or a mixture of these compounds. It is preferable that a commercially available known exo-type enzyme is utilized for the mannosidase used in this reaction. Also, the enzyme may be a newly isolated enzyme or an enzyme generated by genetic engineering, as long as the enzyme has a similar activity. Next, in the same manner as described above, the reaction solution obtained after the reaction (a mixture of asparagine-linked oligosaccharide derivatives of which oligosaccharide moieties are cleaved) is subjected to chromatography to give each of asparagine-linked oligosaccharide derivatives. For instance, it is preferable that the separation is carried out by HPLC (ODS column, eluent: there can be used, for instance, a mixed solution of a buffer such as an about 10 to about 200 mM ammonium acetate and a water-soluble organic solvent with lipophilicity such as acetonitrile, or ethanol, or methanol, or butanol, or propanol in appropriate amounts; when exemplified herein, it is preferable that the eluent is a 50 mM aqueous ammonium acetate:acetonitrile=82:18.).

It is possible to prepare novel asparagine-linked oligosaccharide derivatives containing at least one fucose in N-acetylglucosamine on the nonreducing terminal side of an asparagine-linked oligosaccharide wherein the asparagine has amino group protected with a lipophilic protective group, by obtaining various asparagine-linked oligosaccharide derivatives in this way and thereafter causing the transfer of fucose.

The fucose to be used is one generally available commercially, or one prepared by chemical synthesis.

Examples of fucose transferases usable are those generally available commercially, those naturally occurring and those prepared by genetic recombination. A suitable fucos transferase can be selected in accordance with the kind of fucose to be transferred. A more specific example is Fucosyltransferase V (human recombinant, plasma-derived, serum-derived, milk-derived or liver-derived) which is an enzyme for transferring fucose to N-acetylglucosamine on the nonreducing terminal of asparagine-linked oligosaccharides. Alternatively, fucose can be transferred using fucosidase and shifting equilibrium as by pH adjustment.

The separation of each of asparagine-linked oligosaccharide derivatives by chromatography can be carried out by appropriately using known chromatographies, singly or in a combination of plural chromatographies.

For instance, the resulting mixture of asparagine-linked oligosaccharide derivatives is purified by a gel filtration column chromatography, and then purified by using HPLC. The column which can be used in HPLC is preferably a reverse phase column, for instance, ODS, phenyl-based, nitrile-based, or anion exchange-based column, and concretely, a mono Q column manufactured by Pharmacia, Iatro-beads column manufactured by Iatron can be utilized. The separation conditions and the like may be adjusted by referring to a known condition. According to the above procedures, each of the desired asparagine-linked oligosaccharide derivatives can be obtained from the mixture of asparagine-linked oligosaccharide derivatives.

As described above, each of the various asparagine-linked oligosaccharide derivatives of which branching structures at the terminals of the oligosaccharides are not uniform, can be obtained as individual isolated compounds by further hydrolyzing the derivatives with various glycosidases and the like to remove the sugar moieties at non-reducing terminals of the oligosaccharides after the obtainment of each of the asparagine-linked oligosaccharide derivatives. Moreover, even a larger number of the kinds of the asparagine-linked oligosaccharide derivatives can be prepared by changing the order or the kind of hydrolysis with various glycosidases.

According to a conventional process, enormous amounts of time and cost for obtaining the asparagine-linked oligosaccharide derivatives having very limited oligosaccharide structures are required even on an analytical scale. On the contrary, according to the present invention, about 1 gram of the asparagine-linked oligosaccharide derivatives having desired oligosaccharide structures can be prepared in an about 2-week period by using a conventional gel filtration column, HPLC column, and at least three kinds of glycosidases (for instance, galactosidase, mannosidase, and N-acetylglucosamidase) without necessitating any particular devices or reagents.

According to the present invention, a desired biotinated or FITC-bonded asparagine-linked oligosaccharide derivative is obtained using various asparagine-linked oligosaccharides obtained in the prior application mentioned, α2,3-bonded asparagine-linked oligosaccharides not disclosed in the prior application, (α2,3) (α 2,6) bonded asparagine-linked oligosaccharides, and fucose-bonded compounds of these asparagine-linked oligosaccharides.

The present invention provides an asparagine-linked oligosaccharide having a biotinated or FITC-bonded amino group.

Examples of biotinated asparagine-linked oligosaccharides of the invention can be the compounds of the following formula 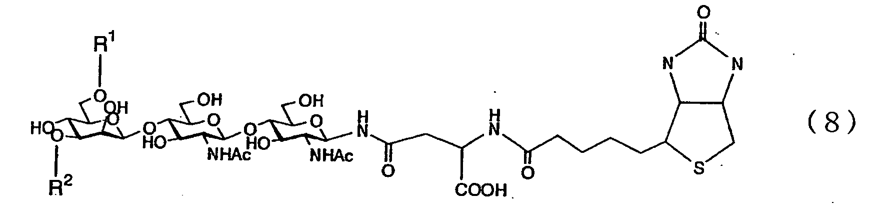 wherein R¹ and R² are each H or one of the groups of the formulae (2) to (6), and may be the same or different.

Examples of FITC-bonded asparagine-linked oligosaccharides can be the compounds of the following formula 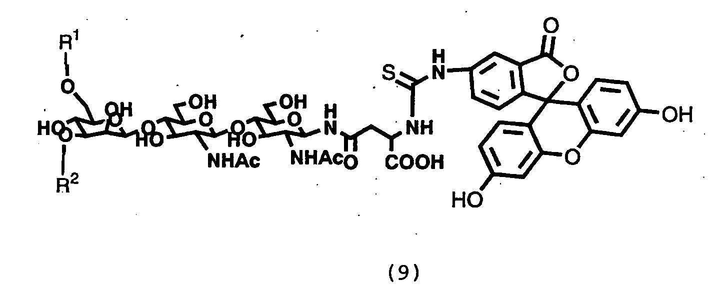 wherein R¹ and R² are as defined above.

The present invention further provides a process for preparing a biotianted or FITC-bonded asparagine-linked oligosaccharide having biotinated or FITC-bonded amino group.

The biotinated or FITC-bonded asparagine-linked oligosaccharide can be prepared by biotinating or FITC-bonding the amino group of asparagine in various isolated asparagine-linked oligosaccharides mentioned above.

Examples of asparagine-linked oligosaccharides for use in the present invention can be the compounds of the formula (7) 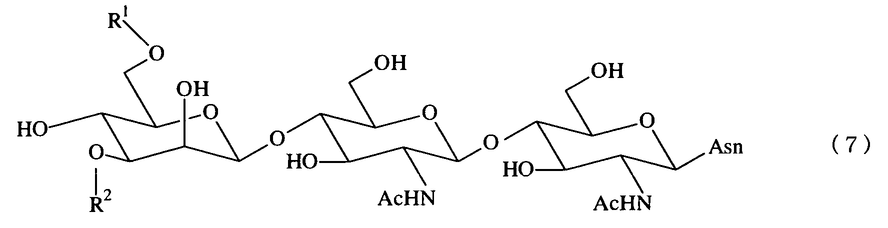 wherein R¹ and R² are as defined above.

More specific examples of asparagine-linked oligosaccharides are compounds disclosed in the prior application. Thus, the α2,6-bonded compounds shown in FIGS. 3 and 4 of the prior application are similarly usable. These compounds as referred to by symbols are herein given in Tables 1 to 3.

The asparagine-linked oligosaccharide derivatives for use in preparing the above asparagine-linked oligosaccharides can also be the α2,6-bonded compounds disclosed in the prior application, i.e., the compounds shown in FIGS. 1 and 2 of the prior application. These compounds as referred to by symbols are herein given in Tables 4 to 6.

In addition to the asparagine-linked oligosaccharides disclosed in the prior application, also usable are α2,3-bonded asparagine-linked oligosaccharides, (α2,3)(α2,6) bonded asparagine-linked oligosaccharides, and fucose-bonded compounds of these asparagine-linked oligosaccharides.

The biotination of the invention can be effected by a known process. A biotinated asparagine-linked oligosaccharide can be obtained, for example, by dissolving an asparagine-linked oligosaccharide in water, adding sodium bicarbonate to the solution, adding dimethylformamide having D-(+)-biotinylsuccinimide dissolved therein to the mixture, reacting the resulting mixture at room temperature for 20 minutes, and purifying the reaction mixture by a gel filtration column or the like.

The present invention provides a microplate having a biotinated asparagine-linked oligosaccharide immobilized thereto. The microplate can be produced by reacting a biotinated asparagine-linked oligosaccharide with an avidinated microplate (e.g., product of Pierce) commercially available.

The invention also provides an affinity column having immobilized thereto a biotinated asparagine-linked oligosaccharide. The column can be produced by reacting a biotinated asparagine-linked oligosaccharide with an avidinated affinity column commercially available.

An asparagine-linked oligosaccharide can be bonded to FITC according to the invention by a known process, for example, by dissolving the asparagine-linked oligosaccharide in water, adding acetone and sodium bicarbonate to the solution, adding fluoresceinisothiocyanate to the mixture, reacting the resulting mixture at room temperature for 2 hours, and purifying the reaction mixture such as by gel filtration column.

The FITC-bonded asparagine-linked oligosaccharide obtained by the invention is useful for the research on acceptors of saccharides in the living body tissues and for the research on the sugar bond specificity of lectin.

### BEST MODE OF CARRYING OUT THE INVENTION

The present invention will be described below with reference to examples, but the invention is in no way limited to these examples. As to ¹H-NMR data, values obtained at 30°C with HOD at 4.8 ppm are given for Examples 1 to 7 and Reference Example 45, and the values obtained at 30°C with the signal of methyl group of acetone serving as an internal standard at 2.225 ppm and with HOD of 4.718 ppm for Reference Examples 1 to 44. For compounds from which Fmoc group is removed, the measurement is done with 50 mM ammonium hydrogencarbonate present in the measurement solvent. Reference Example 1 Preparation of asparagine-linked disialooligosaccharide (Compound 24)

In 100 ml of a tris-hydrochloric acid-calcium chloride buffer (TRIZMA BASE 0.05 mol/l, calcium chloride 0.01 mol/l, pH 7.5) was dissolved 2.6 g of an egg-derived crude SGP (sialyl glycopeptide). 58 mg (772 µmol) of sodium azide and 526 mg of Actinase-E (manufactured by Kaken Pharmaceutical Co., Ltd.) were added to this solution, and the mixture was allowed to stand at 37°C. After 65 hours, 263 mg of Actinase-E was added again, and the mixture was allowed to stand at 37 °C. for additional 24 hours. This solution was freeze-dried, and thereafter the residue was purified twice by gel filtration column chromatography (Sephadex G-25, 2.5 φ × 1 m, eluent: water, flow rate: 1.0 ml/min), to give 1.3 g (555 µmol) of Compound 24.

The physical data for Compound 24 are as follows.
¹H-NMR (D₂O, 30°C)
5.15(1H, s, Man4-H1), 5.06(1H, d, GlcNAc1-H1), 4.95(1H, s, Man4'-H1), 4.82(1H, s, Man3-H1), 4.69(1H, d, GlcNAc2-H1), 4.67(2H, d, GlcNAc5,5'-H1), 4.53(2H, d, Ga16,6'-H1), 4.34(1H, d, Man3-H2), 4.27(1H, d, Man4'-H2), 4.19(1H, d, Man4-H2), 3.03(1H, dd, Asn-βCH), 3.00(1H, dd, Asn-βCH), 2.76(2H, dd, NeuAc7,7'-H3eq), 2.15(18H, s × 6, -Ac), 1.79(2H, dd, NeuAc7,7'-H3ax)

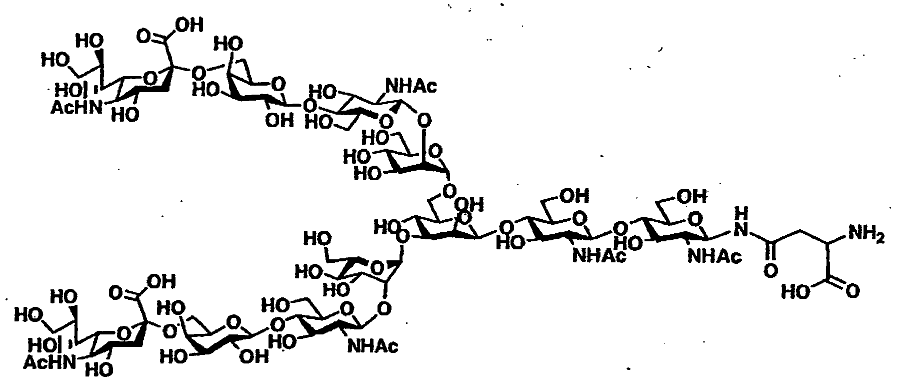

### Reference Example 2 Preparation of Compounds 1, 2, 6 and 10

Compound 24 (609 mg, 261 µmol) obtained in Reference Example 1 was dissolved in 20.7 ml of water, and 13.8 ml of 0.1 N hydrochloric acid was added thereto. Immediately after heating this solution at 70 °C for 35 minutes, the solution was cooled on ice, and a saturated aqueous sodium hydrogencarbonate was added thereto to adjust its pH 7. The solution was freeze-dried, and thereafter the residue was purified by gel filtration column chromatography (Sephadex G-25, 2.5 φ× 1 m, eluent: water, flow rate: 1.0 ml/min), to give 534 mg of a mixture of Compounds 24, 25, 29 and 33. These four components were proceeded to the next step without being isolated from each other.

The physical data for the resulting oligosaccharides mixture are as follows.
¹H-NMR (D₂O, 30°C)
5.13(s, Man4-H1), 5.12(s, Man4-H1), 5.01(d, GlcNAc1-H1), 4.94(s, Man4'-H1), 4.93(s, Man4'-H1), 4.82(s, Man3-H1), 4.60(d, GlcNAc2-H1), 4.58(d, GlcNAc5,5'-H1), 4.47(dd, Ga16,6'-H1), 4.44(d, Gal6,6'-H1), 4.24(d, Man3-H2), 4.19(d, Man4'-H2), 4.11(d, Man4-H2), 2.97(bdd, Asn-βCH), 2.72(dd, NeuAc7-H3eq, NeuAc7-H3eq), 2.64(bdd, Asn-βCH), 2.15 (s× 5, -Ac), 1.79(dd, NeuAc7-H3ax, NeuAc7'-H3ax)

A 429-mg quantity of the mixture of the resulting oligosaccharides was dissolved in 16.3 ml of acetone and 11.2 ml of water. To this solution were added 9-fluorenyl methyl-N-succinimidyl carbonate (155.7 mg, 461.7 µmol) and sodium hydrogencarbonate (80.4 mg, 957 µmol), and the mixture was stirred at room temperature for 2 hours. This solution was applied to an evaporator to remove acetone, and the remaining solution was purified by gel filtration column chromatography (Sephadex G-25, 2.5 φ × 1 m, eluent: water, flow rate: 1.0 ml/min), to give 309 mg of a mixture of Compound 1, Compounds 2 and 6, and Compound 10. This mixture was purified by HPLC (ODS column, eluent: 50 mM aqueous ammonium acetate:methanol=65:35, 2.0 φ × 25 cm, flow rate: 3 ml/min). As a result, Compound 1 was eluted after 51 minutes, a mixture of Compounds 2 and 6 was eluted after 67 minutes, and Compound 10 was eluted after 93 minutes. Each of the fractions were collected and freeze-dried, and thereafter desalted by gel filtration column chromatography (Sephadex G-25, 2.5 φ × 30 cm, eluent: water, flow rate: 1.0 ml/min), thereby giving 150 mg of a desired mixture of Compounds 2 and 6.

The physical data for the resulting Compound 1 are as follows.
¹H-NMR (D₂O, 30°C)
7.99(2H, d, Fmoc), 7.79(2H, d, Fmoc), 7.55(4H, m, Fmoc), 5.15(1H, s, Man4-H1), 5.06(1H, d, GlcNAc1-H1), 4.95(1H, s, Man4'-H1), 4.82(1H, s, Man3-H1), 4.69(1H, d, GlcNAc2-H1), 4.67(2H, d, GlcNAc5,5'-H1), 4.53(2H, d, Ga16,6'-H1), 4.34(1H, d, Man3-H2), 4.27(1H, d, Man4'-H2), 4.19(1H, d, Man4-H2), 3.03(1H, bdd, Asn-βCH), 3.00(1H, bdd, Asn-βCH), 2.76(2H, dd, NeuAc7,7'-H3eq), 2.15(18H, s ×6, -Ac), 1.79(2H, dd, NeuAc7,7'-H3ax) ; HRMS Calcd for C₁₀₃H₁₅₄N₈NaO₆₆ [M+Na+] 2581.8838, found, 2581.8821

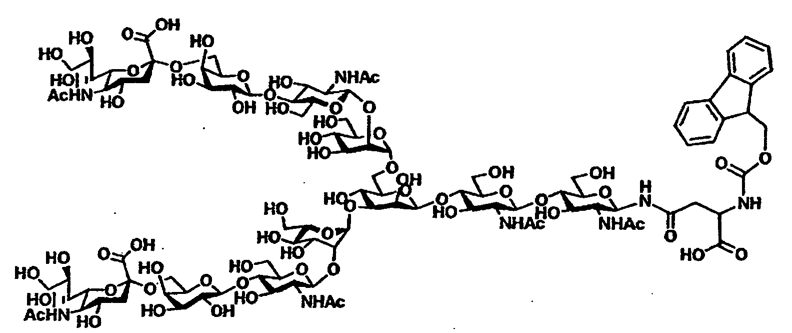

The structure of the above oligosaccharide is shown below. NeuAc: sialic acid, Gal: D-galactose, GlcNAc: N-acetylglucosamine, Man: D-mannose, Asn: asparagine

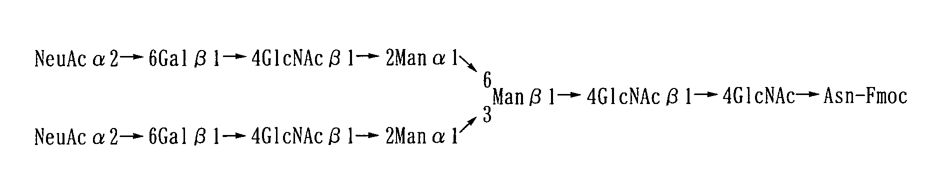

The physical data for the resulting mixture of Compounds 2 and 6 are as follows.
¹H-NMR(D₂O, 30°C)
7.99(d, Fmoc), 7.79(d, Fmoc), 7.55(m, Fmoc), 5.14(s, Man4-H1), 5.12(s, Man4-H), 5.00(d, GlcNAc1-H1), 4.94(s, Man4'-H1), 4.93(s, Man4'-H1), 4.82(s, Man3-H1), 4.60(d, GlcNAc2-H1), 4.58(d, GlcNAc5,5'-H1), 4.46(dd, Gal6,6'-H1), 4.44(d, Gal6,6'-H1), 4.24(d, Man3-H2), 4.19(d, Man4'-H2), 4.11(d, Man4-H2), 2.97(bdd, Asn-βCH), 2.72(dd, NeuAc7-H3eq, NeuAc7-H3eq), 2.64(bdd, Asn-βCH), 2.15 (s ×5, -Ac), 1.79(dd, NeuAc7-H3ax, NeuAc7'-H3ax)

The physical data for the resulting Compound 10 are as follows.
¹H - NMR (D₂O , 30°C)
7.99(2H, d, Fmoc), 7.79(2H, d, Fmoc), 7.55(4H, m, Fmoc), 5.12(1H, s, Man4-H1), 5.06(1H, d, GlcNAc1-H1), 4.93(1H, s, Man4'-H1), 4.82(1H, s, Man3-H1), 4.69(1H, d, GlcNAc2-H1), 4.67(2H, d, GlcNAc5,5'-H1), 4.53(2H, d, Gal6,6'-H1), 4.34(1H, d, Man3-H2), 4.27(1H, d, Man4'-H2), 4.19(1H, d, Man4-H2), 3.03(1H, bdd, Asn-βCH), 3.00(1H, bdd, Asn- βCH), 2.15(12H, s×4, -Ac) ; HRMS Calcd for C₈₁H₁₂₀N₆NaO₅₀[M+Na+] 1999.6930, found, 1999.6939

### Reference Example 3 Preparation of Compounds 3 and 7

The mixture (224 mg, 97 µmol) of Compounds 2 and 6 obtained in Reference Example 2 and 24 mg of bovine serum albumin were dissolved in 22 ml of HEPES buffer (50 mM, pH 6.0), and Diplococcus pneumoniae-derived β-galactosidase (1.35 U) was added thereto. This solution was allowed to stand at 37 °C for 15 hours, and thereafter freeze-dried. The residue was purified by HPLC (ODS column, 2.0 φ×25 cm, eluent: 50 mM aqueous ammonium acetate:acetonitrile=85:15, flow rate: 3 ml/min), and Compound 3 was eluted after 129 minutes, and Compound 7 was eluted after 134 minutes. Each of the fractions was collected and freeze-dried. Subsequently, the fraction was desalted by HPLC (ODS column, 2.0 φ × 25 cm, eluent:water for a first 15 minutes, and applied to a gradient of water:acetonitrile of from 10:0 to 85:15 (volume ratio) for a period of from 16 to 30 minutes, and then to a gradient of water: acetonitrile from 85:15 to 80:20 for a period of from 31 to 45 minutes; flow rate: 3.0 ml/min), to give a desired Compound 3 in an amount of 81 mg and Compound 7 in an amount of 75 mg.
The physical data for the resulting compound 3 are as follows.
¹H-NMR (D₂O, 30°C)
7.99(2H, d, Fmoc), 7.79(2H, d, Fmoc), 7.55(4H, m, Fmoc), 5.15(1H, S, Man4-H1), 5.06(1H, d, GlcNAc1-H1), 4.95(1H, s, Man4'-H1), 4.82(1H, s, Man3-H1), 4.69(1H, d, GlcNAc2-H1), 4.67(2H, d, GlcNAc5,5'-H1), 4.53(1H, d, Ga16'-H1), 4.34(1H, d, Man3-H2), 4.27(1H, d, Man4'-H2), 4.19(1H, d, Man4-H2), 2.97(1H, bdd, Asn-βCH), 2.76(1H, dd, NeuAc7'-H3eq), 2.61(1H, bdd, Asn-βCH), 2.15(15H, s× 5, -Ac), 1.79 (1H, dd, NeuAc7' -H3ax) ; HRMS Calcd for C₈₆H₁₂₇N₇NaO₅₃ [M+Na+] 2128.7356, found, 2128.7363

The physical data for the resulting Compound 7 are as follows.
¹H-NMR (D₂O, 30°C)
7.99(2H, d, Fmoc), 7.79(2H, d, Fmoc), 7.55(4H, m, Fmoc), 5.15(1H, S, Man4-H1), 5.06(1H, d, GlcNAc1-H1), 4.95(1H, s, Man4'-H1), 4.82(1H, s, Man3-H1), 4.69(1H, d, GlcNAc2-H1), 4.67(2H, d, GlcNAc5,5'-H1), 4.53(1H, d, Ga16-H1), 4.34(1H, d, Man3-H2), 4.27(1H, d, Man4'-H2), 4.19(1H, d, Man4-H2), 2.97(1H, bdd, Asn-βCH), 2.76(1H, dd, NeuAc7-H3eq), 2.60(1H, bdd, Asn-βCH), 2.15(15H, s×5, -Ac), 1.79(1H, dd, NeuAc7-H3ax) ; HRMS Calcd for C₈₆H₁₂₅N₇Na₃O₅₃ [M+Na+] 2172.6995, found, 2172.7084

### Reference Example 4 Preparation of Compounds 4 and 8

A mixture (90 mg, 47.3 µmol) of Compounds 3 and 7 obtained in Reference Example 3 was dissolved in 8.1 ml of HEPES buffer (50 mM, pH 6.0) together with 8 mg of bovine serum albumin without separating the compounds from each other, and 2.88 U of a bovine kidney-derived β-glucosaminidase (manufactured by Sigma-Aldrich Corporation, from bovine kidney) was added thereto. This solution was allowed to stand at 37°C for 18 hours, and thereafter freeze-dried. The residue was purified by HPLC (ODS column, 2.0 φ ×25 cm, eluent: 50 mM aqueous ammonium acetate:methanol=65:35, flow rate: 3 ml/min), and Compound 4 was eluted after 117 minutes, and Compound 8 was eluted after 127 minutes. Each of the fractions was collected and freeze-dried. Subsequently, the fraction was desalted by HPLC (ODS column, 2.0 φ × 25 cm, eluent: water for a first 15 minutes, and applied to a gradient of water:acetonitrile of from 10:0 to 85:15 for a period of from 16 to 30 minutes, and then to a gradient of water: acetonitrile of from 85:15 to 80:20 for a period of from 31 to 45 minutes; flow rate: 3.0 ml/min), to give a desired Compound 4 in an amount of 40 mg and Compound 8 in an amount of 37 mg. The physical data for the resulting Compound 4 are as follows.
¹H-NMR (D₂O, 30°C)
8.01(2H, d, Fmoc), 7.80(2H, d, Fmoc), 7.56(4H, m, Fmoc), 5.22(1H, s, Man4-H1), 5.08(1H, d, GlcNAc1-H1), 4.94(1H, s, Man4'-H1), 4.84(1H, s, Man3-H1), 4.69(1H, d, GlcNAc2-H1), 4.67(1H, d, GlcNAc5-H1), 4.55(1H, d, Ga16-H1), 4.33(1H, dd, Man3-H2), 4.20(1H, dd, Man4-H2), 4.15(1H, dd, Man4'-H2), 2.97(1H, bdd, Asn-β CH), 2.76(2H, dd, NeuAc7,7'-H3eq), 2.62 (1H, bdd, Asn-βCH), 2.15(12H, s ×4, -Ac), 1.79(2H, dd, NeuAc7,7'-H3ax) ; HRMS Calcd for C₇₈H₁₁₄N₆NaO₄₈ [M+Na+] 1925.6562, found, 1925.6539

The physical data for the resulting Compound 8 are as follows.
¹H - NMR (D₂O, 30°C)
7.99(2H, d, Fmoc), 7.79(2H, d, Fmoc), 7.55(4H, m, Fmoc), 5.15(1H, S, Man4-H1), 5.06(1H, d, GlcNAc1-H1), 4.95(1H, s, Man4'-H1), 4.82(1H, s, Man3-H1), 4.69(1H, d, GlcNAc2-H1), 4.67(2H, d, GlcNAc5,5'-H1), 4.53(2H, d, Ga16,6'-H1), 4.34(1H, d, Man3-H2), 4.27(1H, d, Man4'-H2), 2.97(1H, bdd, Asn-βCH2), 2.76(1H, dd, NeuAc7'-H3eq), 2.61(1H, bdd, Asn-βCH2), 2.15(12H, s× 4, -Ac), 1.79(1H, dd, NeuAc7'-H3ax); HRMS Calcd for C₇₈H₁₁₄N₆NaO₄₈ [M+Na+] 1925.6562, found, 1925.6533

### Reference Example 5 Preparation of Compound 5

Compound 4 (30 mg, 473 µmol) obtained in Reference Example 4 and 3 mg of bovine serum albumin were dissolved in 6 ml of HEPES buffer (50 mM, pH 6.0), and 10 U of Jack Beans-derived α-mannosidase was added thereto. This solution was allowed to stand at 37°C for 21 hours, and then freeze-dried. Subsequently, the residue was purified by HPLC (ODS column, 2.0 φ × 25 cm, eluent:water for a first 15 minutes, and applied to a gradient of water:acetonitrile of from 10:0 to 85:15 for a period of from 16 to 30 minutes, and then to a gradient of water: acetonitrile of from 85:15 to 80:20 for a period of from 31 to 45 minutes; flow rate: 3.0 ml/min), to give 20 mg of a desired Compound 5.
The physical data for the resulting Compound 5 are as follows.
¹H-NMR (D₂O, 30°C)
8.01(2H, d, Fmoc), 7.80(2H, d, Fmoc), 7.56(4H, m, Fmoc), 5.00(1H, d, GlcNAc1-H1), 4.95(1H, s, Man4'-H1), 4.84 (1H, s, Man3-H1), 4.67(1H, d, GlcNAc2-H1), 4.56(1H, d, GlcNAc5-H1), 4.44(1H, d, Gal6-H1), 4.11(1H, dd, Man4'-H2), 4.07(1H, dd, Man3-H2), 2.97(1H, bdd, Asn-β CH), 2.76(1H, dd, NeuAc7'-H3eq), 2.62(1H, bdd, Asn-βCH), 2.15(12H, s × 4, -Ac), 1.79(2H, dd, NeuAc7'-H3ax) ; HRMS Calcd for C₇₂H₁₀₄N₆NaO₄₃ [M+Na+] 1763.6034, found, 1763.6074

### Reference Example 6 Preparation of Compound 9

Compound 8 (40 mg, 630 µmol) obtained in Reference Example 4 and 5 g of bovine serum albumin were dissolved in 7.8 ml of HEPES buffer (50 mM, pH 6.0), and 38 U of a Jack Beans-derived α-mannosidase was added thereto. This solution was allowed to stand at 37 °C for 63 hours, and then freeze-dried. Subsequently, the residue was purified by HPLC (ODS column, 2.0φ × 25 cm, eluent: water for a first 15 minutes, and applied to a gradient of water: acetonitrile of from 10:0 to 85:15 for a period of from 16 to 30 minutes, and then to a gradient of water: acetonitrile of from 85:15 to 80:20 for a period of from 31 to 45 minutes; flow rate: 3.0 ml/min), to give 30 mg of a desired Compound 9.
The physical data for the resulting Compound 9 are as follows.
¹H - NMR (D₂O, 30°C)
8.01(2H, d, Fmoc), 7.80(2H, d, Fmoc), 7.56(4H, m, Fmoc), 5.23(1H, s, Man4-H1), 5.08(1H, d, GlcNAc1-H1), 4.53(1H, d, Ga16-H1), 4.32(1H, dd, Man3-H2), 4.28(1H, dd, Man4-H2), 2.81(1H, bdd, Asn-βCH), 2.76(1H, dd, NeuAc7-H3eq), 2.59(1H, bdd, Asn-βCH), 2.13(12H, s× 4, -Ac), 1.80 (1H, dd, NeuAc7H3ax) ; HRMS Calcd for C₇₂H₁₀₄N₆NaO₄₃ [M+Na+] 1763.6034, found, 1763.6041

### Reference Example 7

### Deprotection of Fmoc group (Preparation of Compound 33)

Compound 10 (10.5 mg, 5.27 µmoles) obtained in Reference Example 2 was dissolved in 1.4 ml of 50% morpholine/N,N-dimethylformamide solution , and the solution was reacted at room temperature in an argon atmosphere for 2 hours. To the reaction mixture was added 3 ml of toluene, and the mixture was placed into an evaporator at 35°C. This procedure was repeated three times to remove the reaction solvent. The residue was purified by gel filtration column chromatography (Sephadex G-25, 2.5 cm (diam.) × 30 cm, eluent: water, flow rate: 1.0 ml/min.), giving 7 mg of the desired product, i.e., Compound 33 (yield 76%). The compound obtained matched to Compound 33 obtained in Reference Example 2 in ¹H-NMR spectrum and therefore had the same structure as Compound 33.

Given below is the physical data of Compound 33.
¹H-NMR(30°C)
δ5.12(s, 1H, Man4-H-1), 5.07(d, 1H, J=9.7Hz, GlcNAc1-H-1), 4.92(s, 1H, Man4'-H-1), 4.76(s, 1H, Man3-H-1), 4.62(d, 1H, J=8.0Hz, GlcNAc2-H-1), 4.58(d, 2H, J=7.8Hz, GlcNAc5,5'-H-1), 4.47(d, 2H,J =7.9Hz, Ga16,6'-H-1), 4.24(bd, 1H, Man3-H-2), 4.19(bdd, 1H, J=3.2Hz, 1.4Hz, Man4'-H-2), 4.12(bdd, 1H, J=3.2Hz, 1.4Hz, Man4-H-2), 2.93(dd, 1H, J=4.5Hz, 17.0Hz, Asn-βCH), 2.93(dd, 1H, J=6.8Hz, 17.0Hz Asn-β CH), 2.08(s, 3H, Ac), 2.05(s, 6H, Ac ×2), 2.01(s, 3H, Ac)

### Reference Example 8 Preparation of Compound 14

Compound 3 (28 mg, 21.3 µmol) and 1.0 mg of bovine serum albumin were dissolved in HEPES buffer (50 mM, pH 5.0, 454 µL), and neuraminidase (manufactured by Sigma-Aldrich Corp., from Viblio Cholerae, 198 mU) was added thereto. This solution was allowed to stand at 37°C for 20 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 14 (17 mg, yield: 70%).
The physical data for the resulting compound are as follows.
¹H-NMR (30°C)
δ7.91(d, 2H, J=7.5Hz, Fmoc), 7.71(d, 2H, J=7.5Hz, Fmoc), 7.51(dd, 2H, J=7.5Hz, Fmoc), 7.43(dd, 2H, J=7.5Hz, Fmoc), 5.12(s, 1H, Man4-H-1), 4.99(d, 1H, J=9.5Hz, GlcNAc1-H-1), 4.92(s, 1H,Man4'-H-1), 4.76(s, 1H, Man3-H-1), 4.58(d, 1H, J=8.0Hz, GlcNAc2-H-1), 4.55(d, 1H, J=8.4Hz, GlcNAc5'-H-1), 4.47(d, 1H, J=7.8Hz, Ga16'-H-1), 4.34(t, 1H, Fmoc), 4.24(bd, 1H, J=1.9Hz, Man3-H-2), 4.18(bdd, 1H, J=1.4Hz ,3.3Hz, Man4-H-2), 4.11(bdd, 1H, J=1.4Hz, 3.5Hz, Man4'-H-2), 2.72(bdd, 1H, J=3.0Hz, 15.7Hz, Asn-βCH), 2.52(bdd, 1H, J=8.7Hz, 15.7Hz, Asn-βCH), 2.06, 2.05, 2.04, 1.89(each s, each 3H, Ac) ; HRMS Calcd for C₇₅H₁₁₀N₆NaO₄₅ [M+Na+] 1837.6402, found 1837.6471

### Reference Example 9 Preparation of Compound 19

Compound 7 (20 mg, 9.4 µmol) and 1.6 mg of bovine serum albumin were dissolved in HEPES buffer (50 mM, pH 5.0, 323 µL), and neuraminidase (manufactured by Sigma-Aldrich Corp., from Viblio Cholerae, 141 mU) was added thereto. This solution was allowed to stand at 37 °C for 18 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 19 (13 mg, yield: 76%). The structure of the resulting compound was confirmed from the finding that its ¹H-NMR was identical to that of the standard compound.

### Reference Example 10 Preparation of Compound 15

Compound 4 (45 mg, 24 µmol) and 1.7 mg of bovine serum albumin were dissolved in HEPES buffer (50 mM, pH 5.0, 820 µL), and neuraminidase (manufactured by Sigma-Aldrich Corp., from Viblio Cholerae, 134 mU) was added thereto. This solution was allowed to stand at 37 °C for 14 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 15 (28 mg, yield: 74%). The physical data for the resulting compound are as follows.
¹H-NMR (30°C)
δ7.92(d, 2H, J=7.5Hz, Fmoc), 7.71(d, 2H, J=7.5Hz, Fmoc), 7.51(dd, 2H, J=7.5Hz, Fmoc), 7.44(dd, 2H, J=7.5Hz, Fmoc), 5.10(s, 1H, Man4-H-1), 4.99(d, 1H, J=9.5Hz, GlcNAc1-H-1), 4.92(s, 1H, Man4'-H-1), 4.76(s, 1H, Man3-H-1), 4.58(d, 2H, GlcNAc2,5'-H-1), 4.47(d, 1H, J=8.0Hz, Ga16'-H-1), 4.35(t, 1H, Fmoc), 4.24(bd, 1H, J=1.9Hz, Man3-H-2), 4.11(bs, 1H, Man4'-H-2), 4.07(bs, 1H, Man4-H-2), 2.72(bd, 1H, J=15.5Hz, Asn-βCH), 2.52(bdd, 1H, J=8.7Hz, 15.5Hz, Asn-βCH), 2.06, 2.04, 1.89(each s, each 3H, Ac) ; HRMS Calcd for C₆₇H₉₇N₅NaO₄₀[M+Na+ 1634.5608, found, 1634.5564

### Reference Example 11 Preparation of Compound 70

Compound 15 (11 mg, 6.8 µmol) and 1.5 mg of bovine serum albumin were dissolved in HEPES buffer (50 mM, pH 5.0, 269 µL), and β -galactosidase (manufactured by Seikagaku Corp., from Jack Beans, 11 µL, 275 mU) was added thereto. This solution was allowed to stand at 37 °C for 14 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 70 (6.3 mg, yield: 64%). The physical data for the resulting compound are as follows.
¹H-NMR (30°C)
δ 7.91(d, 2H, J=7.5Hz, Fmoc), 7.70(d, 2H, J=7.5Hz, Fmoc), 7.50(dd, 2H, J=7.5Hz, Fmoc), 7.43(dd, 2H, J=7.5Hz, Fmoc), 5.10(s, 1H, Man4-H-1), 4.99(d, 1H, J=9.5Hz, GlcNAc1-H-1), 4.91(s, 1H, Man4'-H-1), 4.76(s, 1H, Man3-H-1), 4.55(d, 2H, GlcNAc2,5'-H-1), 4.32(t, 1H, Fmoc), 4.24(bs, 1H, Man3-H-2), 4.10(bs, 1H, Man4-H-2), 4.06(bs, 1H, J=1.3Hz, Man4'-H-2), 2.72(bd, 1H, J=14.0Hz, Asn-βCH), 2.52(bdd, 1H, J=9.5Hz, 14.8Hz, Asn-βCH), 2.06, 2.05, 1.89(each s, each 3H, Ac) ; MS(Fab), Calcd for C₆₁H₈₈N₅O₃₅[M+H+] 1450.5, found, 1450.4

### Reference Example 12 Preparation of Compound 20

Compound 8 (47 mg, 25 µmol) and 1.9 mg of bovine serum albumin were dissolved in HEPES buffer (50 mM, pH 5.0, 840 µL), and neuraminidase (manufactured by Sigma-Aldrich Corp., from Viblio Cholerae, 369 mU) was added thereto. This solution was allowed to stand at 37 °C for 37 hours, and thereafter the termination of the reaction was confirmed by HPLC analysis. The reaction solution was freeze-dried, and the freeze-dried product was subsequently purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 20 (26 mg, yield: 65%). The physical data for the resulting compound are as follows.
¹H-NMR(30°C)
δ7.92(d, 2H, J=7.5Hz, Fmoc), 7.71(d, 2H, J=7.5Hz, Fmoc), 7.51(dd, 2H, J=7.5Hz, Fmoc), 7.43(dd, 2H, J=7.5Hz, Fmoc), 5.12(s, 1H, Man4-H-1), 4.99(d, 1H, J=9.4Hz, GlcNAc1-H-1), 4.91(s, 1H, Man4'-H-1), 4.77(s, 1H, Man3-H-1), 4.57(bd, 2H, GlcNAc2,5'-H-1), 4.46(d, 1H, J=7.5Hz, Ga16'-H-1), 4.34(t, 3H, Fmoc), 4.24(bs, 1H, Man4'-H-2), 4.19(bs, 1H, Man4-H-2), 2.72(bd, 1H, J=15.5Hz, Asn-βCH), 2.52(bdd, 1H, J=9.2Hz, 15.5Hz, Asn-βCH), 2.06, 2.05, 1.89(each s, each 3H, Ac) ; HRMS Calcd for C₆₇H₉₇N₅NaO₄₀ [M+Na+] 1634.5608, found, 1634.5644

### Reference Example 13 Preparation of Compound 71

Compound 20 (12 mg, 7.4 µmol) and 1.0 mg of bovine serum albumin were dissolved in HEPES buffer (50 mM, pH 5.0, 330 µL), and β-galactosidase (manufactured by Seikagaku Corp., from Jack Beans, 12 µL, 297 mU) was added thereto. This solution was allowed to stand at 37°C for 46 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 71 (6.6 mg, yield: 61%). The physical data for the resulting compound are as follows.
¹H-NMR(30°C)
δ7.90(d, 2H, J=7.5Hz, Fmoc), 7.70(d, 2H, J=7.5Hz, Fmoc), 7.49(dd, 2H, J=7.5Hz, Fmoc), 7.42(dd, 2H, J=7.5Hz, Fmoc), 5.11(s, 1H, Man4-H-1), 4.99(d, 1H, J=9.4Hz, GlcNAc1-H-1), 4.91(s, 1H, Man4'-H-1), 4.76(s, 1H, Man3-H-1), 4.55(d, 2H, GlcNAc2,5-H-1), 4.31(b, 1H, Fmoc), 4.24(bs, 1H, Man3-H-2), 4.18(bs, 1H, Man4-H-2), 3.97(dd, 1H, J=1.8Hz, 3.3Hz, Man4'-H-2), 2.72(bd, 1H, J=15.5Hz, Asn-βCH), 2.52(bdd, 1H, J=8.0Hz, 15.5Hz, Asn-βCH), 2.06, 2.05, 1.88(each s, each 3H, Ac) ; MS (Fab), Calcd for C₆₁H₈₈N₅O₃₅ [M+H+] 1450.5, found, 1450.3

### Reference Example 14 Preparation of Compound 16

Compound 5 (32 mg, 18.4 µmol) and 2.5 mg of bovine serum albumin were dissolved in HEPES buffer (50 mM, pH 5.0, 713 µL), and neuraminidase (manufactured by Sigma-Aldrich Corp., from Viblio Cholerae, 134 mU) was added thereto. This solution was allowed to stand at 37 °C for 17 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 16 (13 mg, yield: 52%). The physical data for the resulting compound are as follows.
¹H-NMR (30°C)
δ7.92(d, 2H, J=7.5Hz, Fmoc), 7.71(d, 2H, J=7.5Hz, Fmoc), 7.51(dd, 2H, J=7.5Hz, Fmoc), 7.44(dd, 2H, J=7.5Hz, Fmoc), 5.00(d, 1H, J=9.9Hz, GlcNAc1-H-1), 4.92(s, 1H, Man4'-H-1), 4.75(s, 1H, Man3-H-1), 4.58(d, 2H, J=7.5Hz, GlcNAc2,5'-H-1), 4.47(d, 1H, J=7.8Hz, Ga16'-H-1), 4.34(t, 1H, Fmoc), 4.10(bd, 1H, Man3-H-2), 4.07(bs, 1H, Man4'-H-2), 2.72(bdd, 1 H, J=15.5Hz, Asn-βCH), 2.52(bdd, 1H, J=9.2Hz, 15.5Hz, Asn-βCH), 2.07, 2.05, 1.89(each s, each 3H, Ac) ; MS (Fab), Calcd for C₆₁H₈₈N₅O₃₅ [M+H+] 1450.5, found, 1450.3

### Reference Example 15 Preparation of Compound 17

Compound 16 (9 mg, 6.2 µmol) and 1.6 mg of bovine serum albumin were dissolved in HEPES buffer (50 mM, pH 5.0, 613 µL), and β -galactosidase (manufactured by Seikagaku Corp., from Jack Beans, 186 mU) was added thereto. This solution was allowed to stand at 37 °C for 32 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 17 (5.4 mg, yield: 68%). The physical data for the resulting compound are as follows.
¹H-NMR (30°C)
δ7.89(d, 2H, J=7.5Hz, Fmoc), 7.68(d, 2H, J=7.5Hz, Fmoc), 7.49(dd, 2H, J=7.5Hz, Fmoc), 7.42(dd, 2H, J=7.5Hz, Fmoc), 4.99(d, 1H, J=9.7Hz, GlcNAc1-H-1), 4.91(s, 1H, Man4'-H-1), 4.55(d, 1H, J=8.1Hz, GlcNAc2,5'-H-1), 4.09, 4.07(s, 1H, Man4'-H-2, Man3-H-2), 2.72(bd, 1H, J=15.5Hz, Asn-βCH), 2.56(bdd, 1H, J=8.1Hz, 15.5Hz, Asn-βCH), 2.07, 2.05, 1.89(each s,each 3H, Ac) ; MS(Fab), Calcd for C₅₅H₇₇N₅NaO₃₀ [M+Na+] 1310.5, found, 1310.2

### Reference Example 16 Preparation of Compound 18

Compound 17 (3.4 mg, 2.6 µmol) and 1.1 mg of bovine serum albumin were dissolved in HEPES buffer (50 mM, pH 5.0, 257 µL), and N-acetylglucosamidase (manufactured by Sigma-Aldrich Corp., from Jack Beans, 144 mU) was added thereto. This solution was allowed to stand at 37 °C for 24 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 18 (2.1 mg, yield: 75%). The physical data for the resulting compound are as follows.
¹H-NMR (30°C)
δ7.91(d, 2H, J=7.5Hz, Fmoc), 7.71(d, 2H, J=7.5Hz, Fmoc), 7.51(dd, 2H, J=7.5Hz, 7.5Hz, Fmoc), 7.43(dd, 2H, J=7.5Hz, 7.5Hz, Fmoc), 5.00(d, 1H, J=9.7Hz, GlcNAc1-H-1), 4.91(d, 1H, J=1.6Hz, Man4'-H-1), 4.76(s, 1H, Man3-H-1), 4.58(d, 1H, J=7.8Hz, GlcNAc2-H-1), 4.34(t, 1H, Fmoc), 4.07(d, 1H, J=2.7Hz, Man4'-H-2), 3.97(dd, 1H, J=1.6Hz, 3.7Hz, Man3-H-2), 2.72(bdd, 1H, J=3.2Hz, 15.1Hz, Asn-β CH), 2.52(bdd, 1H, J=8.9Hz, 15.1Hz, Asn-βCH), 2.07, 1.89(each s, each 3H, Ac) ; MS (Fab), Calcd for C₄₇H₆₅N₄O₂₅ [M+Na+] 1085.4, found, 1085.3

### Reference Example 17 Preparation of Compound 21

Compound 9 (28 mg, 16 µmol) and 1.7 mg of bovine serum albumin were dissolved in HEPES buffer (50 mM, pH 5.0, 624 µL), and neuraminidase (manufactured by Sigma-Aldrich Corp., from Viblio Cholerae, 117 mU) was added thereto. This solution was allowed to stand at 37°C for 17 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 21 (14.6 mg, yield: 68%). The physical data for the resulting compound are as follows.
¹H-NMR (30°C)
δ7.92(d, 2H, J=7.5Hz, Fmoc), 7.71(d, 2H, J=7.5Hz, Fmoc), 7.50(dd, 2H, J=7.5Hz, Fmoc), 7.43(dd, 2H, J=7.5Hz, Fmoc), 5.12(s, 1H, Man4-H-1), 4.99(d, 1H, J=9.5Hz, G1cNAc1-H-1), 4.77(s, 1H, Man3-H-1), 4.57(d, 2H, J=7.2Hz, G1cNAc2-H-1), 4.46(d, 1H, J=7.8Hz, Ga16-H-1), 4.34(t, 1H, Fmoc), 4.22(bd, 1H, J=2.7Hz, Man3-H-2), 4.19(b, 1H, Man4-H-2), 2.72(bdd, 1H, J=15.5Hz, Asn-βCH), 2.52(bdd, 1H, J=9.8Hz, 15.5Hz, Asn-βCH), 2.05(s, 6H, Ac ×2), 1.89(s, 3H, Ac) ; MS(Fab), Calcd for C₆₁H₈₈N₅O₃₅[M+H+] 1450.5, found, 1450.3

### Reference Example 18 Preparation of Compound 22

Compound 21 (10 mg, 6.9 µmol) and 1.6 mg of bovine serum albumin were dissolved in HEPES buffer (50 mM, pH 5.0, 672 µL), and β-galactosidase (manufactured by Seikagaku Corp., from Jack Beans, 205 mU) was added thereto. This solution was allowed to stand at 37 °C for 20 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 22 (5.6 mg, yield: 64%). The physical data for the resulting compound are as follows.
¹H-NMR(30°C)
δ7.87(d, 2H, J=7.5Hz, Fmoc), 7.67(d, 2H, J=7.5Hz, Fmoc), 7.48(dd, 2H, J=7.5Hz, Fmoc), 7.41(dd, 2H, J=7.5Hz, Fmoc), 5.12(s, 1H, Man4-H-1), 4.99(d, 1H, J=9.7Hz, GlcNAc1-H-1), 4.76(s, 1H, Man3-H-1), 4.55(d, 2H, J=8.6Hz, GlcNAc2,5-H-1), 4.26(t, 1H, moc), 4.22(d, 1H, J=2.2Hz, Man3-H-2), 4.18(bdd, 1H, J=1.3Hz, 3.3Hz, Man4-H-2), 2.72(bd, 1H, J=15.5Hz, Asn-βCH), 2.54 (bdd, 1H,J=9.5Hz,15.5Hz,Asn-β CH),2.05(s,6H,Ac ×2), 1.88(s, 3H, Ac) ; MS(Fab), Calcd for C₅₅H₇₈N₅O₃₀[M+H+] 1288.5, found, 1288.3

### Reference Example 19 Preparation of Compound 23

Compound 22 (3.6 mg, 2.8 µmol) and 1.2 mg of bovine serum albumin were dissolved in HEPES buffer (50 mM, pH 5.0, 277 µL), and N-acetylglucosamidase (manufactured by Sigma-Aldrich Corp., from Jack Beans, 195 mU) was added thereto. This solution was allowed to stand at 37 °C for 24 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 23 (2.3 mg, yield: 77%). The physical data for the resulting compound are as follows.
¹H-NMR (30°C)
δ 7.91(d, 2H, J=7.5Hz, Fmoc), 7.70(d, 2H, J=7.5Hz, Fmoc), 7.50(dd, 2H, J=7.5Hz, Fmoc), 7.43(dd, 2H, J=7.5Hz, Fmoc), 5.11(s, 1H, Man4-H-1), 4.99(d, 1H, J=9.7Hz, GlcNAc1-H-1), 4.77(s, 1H, Man3-H-1), 4.57(d, 1H, J=6.5Hz, GlcNAc-H-1), 4.33(t, 1H, Fmoc), 4.22(d, 1H, J=3.0Hz, Man3-H-2), 4.07(bdd, 1H, J=2.1Hz, Man4-H-2), 2.72(bdd, 1H, J=15.5Hz, Asn-βCH), 2.52(bdd, 1H, J=8.9Hz, 15.5Hz, Asn-βCH), 2.05, 1.89 (each s, each 3H, Ac); MS (Fab), Calcd for C₄₇H₆₅N₄O₂₅ [M+H+] 1085.4, found, 1085.3

### Reference Example 20 Preparation of Compound 11

Compound 10 (123 mg, 62 µmol) and 1.1 mg of bovine serum albumin were dissolved in HEPES buffer (50 mM, pH 5.0, 2.5 mL), and β -galactosidase (manufactured by Seikagaku Corp., from Jack Beans, 24 µL, 612 mU) was added thereto. This solution was allowed to stand at 37 °C for 61 hours, and thereafter the termination of the reaction was confirmed by HPLC analysis. The reaction solution was freeze-dried, and the freeze-dried product was subsequently purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on ODS column (Cosmosil 75C18-OPN, 15 × 100 mm, eluted first with 50 mL of H₂O and then with 25% acetonitrile), to give a desired Compound 11 (71 mg, yield: 70%). The physical data for the resulting compound are as follows.
¹H-NMR (30°C)
δ 7.91(d, 2H, J=7.5Hz, Fmoc), 7.71(d, 2H, J=7.5Hz, Fmoc), 7.50(dd, 2H, J=7.5Hz, Fmoc), 7.43(dd, 2H, J=7.5Hz, Fmoc), 5.11(s, 1H, Man4-H-1), 4.99(1H, d, J=9.9Hz, GlcNAc1-H-1), 4.91(s, 1H, Man4'-H-1), 4.76(s, 1H, Man3-H-1), 4.55(d, 2H, J=8.6Hz, GlcNAc2,5-H-1), 4.34(t, 1H, Fmoc), 4.24(s, 1H, Man3-H-2), 4.18(s, 1H, Man4-H-2), 4.10(s, 1H, Man4'-H-2), 2.72(bd, 1H, J=15.5Hz, Asn-βCH), 2.51(bdd, 1H, J=9.0Hz, 15.5Hz, Asn-βCH), 2.06(s, 3H, Ac), 2.05(s, 6H, Ac× 2), 1.88 (s, 3H, Ac) ; HRMS Calcd for C₆₉H₁₀₀N₆NaO₄₀[M+Na+] 1675.5873, found, 1675.5841

### Reference Example 21 Preparation of Compound 12

Compound 11 (50 mg, 30 µmol) and 2.0 mg of bovine serum albumin were dissolved in HEPES buffer (50 mM, pH 5.0, 920 µL), and N-acetylglucosamidase (manufactured by Sigma-Aldrich Corp., from Jack Beans, 2.1 U) was added thereto. This solution was allowed to stand at 37 °C for 48 hours, and thereafter the termination of the reaction was confirmed by HPLC analysis. The reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min) and then freeze-dried. The residue was desalted on an ODS column (Cosmosil 75C18-OPN, 15 × 100 mm, eluted first with 50 mL of H₂O and then with 25% acetonitrile), to give a desired Compound 12 (25 mg, yield: 66%). The physical data for the resulting compound are as follows.
¹H-NMR (30°C)
δ 7.91(d, 2H, J=7.5Hz, Fmoc), 7.70(d, 2H, J=7.5Hz, Fmoc), 7.50(dd, 2H, J=7.5Hz, Fmoc), 7.43(dd, 2H, J=7.5Hz, Fmoc), 5.10(s, 1H, Man4-H-1), 4.99(d, 1H, J=9.7Hz, GlcNAc1-H-1), 4.91(bd, 1H, J=1.6Hz, Man4'-H-1), 4.77(s, 1H, Man3-H-1), 4.58~4.52(b, 1H, G1cNAc2-H-1), 4.33(t, 1H, Fmoc), 4.24(bs, 1H, Man3-H-2), 4.06(dd, 1H, J=1.6Hz, 3.2Hz, Man4-H-2), 3.97(dd, 1H, J=1.6Hz, 3.5Hz, Man4'-H-2), 2.72(bd, 1H, J=15.5Hz, Asn-βCH), 2.53(bdd, 1H, J=9.0Hz, 15.5Hz, Asn-βCH), 2.05, 1.88(each s, each 3H, Ac)

### Reference Example 22 Preparation of Compound 13

Compound 12 (10 mg, 11 µmol) and 0.9 mg of bovine serum albumin were dissolved in HEPES buffer (50 mM, pH 5.0, 440 µL), and α-mannosidase (manufactured by Sigma-Aldrich Corp., from Jack Beans, 30 µL, 3.2 U) was added thereto. This solution was allowed to stand at 37°C for 21 hours, and thereafter the termination of the reaction was confirmed by HPLC analysis. Subsequently, the reaction solution was purified by HPLC (YMC Packed Column D-ODS-5 S-5 120A ODS No. 2020178, 20 × 250 mm, eluent: 50 mM aqueous ammonium acetate:acetonitrile=80:20, flow rate: 4 mL/min). Further, the residue was desalted on an ODS column (Cosmosil 75C18-OPN, 15 × 100 mm, eluted first with 50 mL of H₂O and then with 25% acetonitrile), to give a desired Compound 13 (3 mg, yield: 43%). The physical data for the resulting compound are as follows.
¹H-NMR (30°C)
δ7.92(d, 2H, J=7.5Hz, Fmoc), 7.71(d, 2H, J=7.5Hz, Fmoc), 7.51(dd, 2H, J=7.5Hz, Fmoc), 7.43(dd, 2H, J=7.5Hz, Fmoc), 4.99(d, 1H, J=9.5Hz, GlcNAc1-H-1), 4.76(s, 1H, Man3-H-1), 4.57(1H, GlcNAc2-H-1), 4.06(d, 1H, J=3.2Hz, Man3-H-2), 2.72(bd, 1H, J=15.5Hz, Asn-βCH), 2.52(bdd, 1H, J=8.3Hz, 15.5Hz, Asn-βCH), 2.05, 1.89(each s, each 3H, Ac),

### (Deprotection of Fmoc group of asparagine-linked oligosaccharide derivative)

Fmoc group was deprotected by the following procedure for all the asparagine-linked oligosaccharide derivatives. First, 240 µL of N,N-dimethylformamide and 160 µL of morpholine were added to the asparagine-linked oligosaccharide Fmoc compound per µmole of the latter, and the mixture was reacted at room temperature in an argon atmosphere. The completion of reaction was confirmed by TLC (eluent: 1 M ammonium acetate:isopropanol=8:5), and the reaction mixture was cooled with ice water. To the reaction mixture was added diethyl ether in 10 times the amount of the mixture, followed by stirring for 15 minutes, and the precipitate separating out was filtered off. The residue obtained was dissolved in water, and the solution was evaporated at 35°C. With addition of 3 mL of toluene, the resulting residue was further evaporated. This procedure was repeated three times. The resulting residue was purified by reverse-phase column chromatography (Cosmosil 75C18-OPN, 15 × 100 mm, eluent: water).

### Reference Example 23 Preparation of Compound 33

Compound 10 (10.5 mg, 5.3 µmoles) was reacted for 7 hours by the deprotection procedure described above, giving desired Compound 33 (7 mg, yield 76%). For identification, the compound obtained was found to match to an authentic product in ¹H-NMR.

### Reference Example 24 Preparation of Compound 26

Compound 3 (8.0 mg, 3.8 µmoles) was reacted for 21 hours by the deprotection procedure described above, giving desired Compound 26 (6.3 mg, yield 88%). The physical data for the resulting compound are as follows.
¹H-NMR (30°C)
δ5.13(s, 1H, Man4-H-1), 5.07(d, 1H, J=9.9Hz, GlcNAc1-H-1), 4.95(s, 1H, Man4'-H-1), 4.78(s, 1H, Man3-H-1), 4.62(2H, GlcNAc2,5'-H-1), 4.56(d, 1H, J=8.1Hz, GlcNAc5-H-1), 4.52(d, 1H, J=7.8Hz, Gal6'-H-1), 4.25(bs, 1H, Man3-H-2), 4.19(bs, 1H, Man4'-H-2), 4.12(bs, 1H, Man4-H-2), 2.94(dd, 1H, J=4.5Hz, 17.0Hz,Asn-βCH), 2.85(dd, 1H, J=6.8Hz, 17.0Hz, Asn-βCH), 2.68(dd, 1H, J=4.6Hz, 12.4Hz, NeuAc7'-H-3eq), 2.08, 2.07, 2.06, 2.04, 2.02(each s, each 3H, Ac), 1.72(dd, 1H, J=12.1Hz, 12.1Hz, NeuAc7'-H-3ax) ; MS(Fab), Calcd for C₇₁H₁₁₈N₇O₅₁ [M+H+] 1884.7, found, 1884.5

### Reference Example 25 Preparation of Compound 27

Compound 4 (11.0 mg, 5.8 µmoles) was reacted for 23 hours by the deprotection procedure described above, giving desired Compound 27 (8.5 mg, yield 88%). The physical data for the resulting compound are as follows.
¹H-NMR (30°C)
δ 5.11(s, 1H, Man4-H-1), 5.08(d, 1H, J=9.7Hz, GlcNAc1-H-1), 4.95(s, 1H, Man4'-H-1), 4.78(s, 1H, Man3-H-1), 4.62(d, 2H, GlcNAc2,5'-H-1), 4.45(d, 1H, J=7.6Hz, Gal6'-H-1), 4.26(bd, 1H, Man3-H-2), 4.12(bd, 1H, Man4'-H-2), 4.08(bdd, 1H, J=1.6Hz, 3.3Hz, Man4-H-2), 2.94(dd, 1H, J=4.0Hz, 17.2Hz, Asn-βCH), 2.85(dd, 1H, J=7.2Hz, 17.2Hz, Asn-βCH), 2.68(dd, 1H, J=4.1Hz, 12.1Hz, NeuAc7'-H-3eq), 2.09, 2.07, 2.04, 2.02(each s, each 3H, Ac), 1.72(dd, 1H, J=12.1Hz, 12.1Hz, NeuAc7'-H-3ax), ; MS(Fab), Calcd for C₆₃H₁₀₄N₆NaO₄₆ [M+Na+] 1703.6, found, 1703.1

### Reference Example 26 Preparation of Compound 28

Compound 5 (7.0 mg, 4.0 µmoles) was reacted for 21 hours by the deprotection procedure described above, giving desired Compound 28 (5.3 mg, yield 87%). The physical data for the resulting compound are as follows.
¹H-NMR (30°)
δ 5.07 (d, 1H, J=9.4Hz, GlcNAc1-H-1), 4.94(s, 1H, Man4'-H-1), 4.76(s, 1H, Man3-H-1), 4.61, 4.59(each d, each 1H, GlcNAc2,5'-H-1), 4.44(d, 1H, J=7.8Hz, Ga16'-H-1), 4.10, 4.07(each 1H, Man4', 3-H-2), 2.93(dd, 1H, J=4.6Hz, 17.5Hz, Asn-βCH), 2.85(dd, 1H, J=7.0Hz, 17.5Hz, Asn-βCH), 2.67(dd, 1H, J=4.6Hz, 12.2Hz, NeuAc7'-H-3eq), 2.08, 2.06, 2.02, 2.01(each s, each 3H, Ac), 1.71(2H, dd, J=12.2Hz, 12.2Hz, NeuAc7'-H-3ax) ; MS (Fab), Calcd for C₅₇H₉₄N₆NaO₄₁ [M+Na+] 1541.5, found, 1541.3

### Reference Example 27 Preparation of Compound 30

Compound 7 (13.9 mg, 6.6 µmoles) was reacted for 7 hours by the deprotection procedure described above, giving desired Compound 30 (8.0 mg, yield 64%). The physical data for the resulting compound are as follows.
¹H - NMR (30°C)
δ 5.13(s, 1H, Man4-H-1), 5.06(d, 1H, J=9.9Hz, GlcNAc1-H-1), 4.91(s, 1H, Man4'-H-1), 4.77(s, 1H, Man3-H-1), 4.61, 4.60(each d, each 1H, J=8.0Hz, GlcNAc2,5-H-1), 4.55(d, 1H, J=8.4Hz, GlcNAc5'-H-1), 4.44(d, 1H, J=8.0Hz, Gal6-H-1), 4.24(bd, 1H, Man3-H-2), 4.19(bdd, 1H, J=1.3Hz, 3.2Hz, Man4'-H-2), 4.10(bdd, 1H, J=1.4Hz, 3.2Hz, Man4-H-2), 2.90(dd, 1H, J=4.5Hz, 16.7Hz, Asn-βCH), 2.80(dd, 1H, J=7.5Hz, 16.7Hz, A sn-*β*CH), 2.66(dd, 1H, J=4.6Hz, 12.4Hz, NeuAc7-H-3eq), 2.07, 2.06, 2.05, 2.02, 2.01(each s, each 3H, Ac), 1.71(dd, 1H, J=12.4Hz, 12.4Hz, NeuAc7-H-3ax) ; MS(Fab), Calcd for C₇₁H₁₁₇N₇NaO₅₁ [M+Na+] 1906.7, found, 1906.1

### Reference Example 28 Preparation of Compound 31

Compound 8 (8.0 mg, 4.2 µmoles) was reacted for 12 hours by the deprotection procedure described above, giving desired Compound 31 (6.0 mg, yield 86%). The physical data for the resulting compound are as follows.
¹H-NMR (30°C)
δ5.12(s, 1H, Man4-H-1), 5.06(d, 1H, J=9.5Hz, GlcNAc1-H-1), 4.91(s, 1H, Man4'-H-1), 4.77(s, 1H, Man3-H-1), 4.61, 4.59(each d, each 1H, GlcNAc2,5-H-1), 4.43(d, 1H, J=8.0Hz, Ga16-H-1), 4.24(bd, 1H, Man3-H-2), 4.18(bdd, 1H, Man4'-H-2), 2.91(bd, 1H, J=17.0Hz, Asn-βCH), 2.81(dd, 1H, J=6.5Hz, 17.0Hz, Asn-β CH), 2.66(dd, 1H, J=4.6Hz, 12.6Hz, NeuAc7-H-3eq), 2.06, 2.06, 2.02, 2.00(each s, each 3H, Ac), 1.70(dd, 1H, J=12.6Hz, 12.6Hz, NeuAc7-H-3ax) ; MS(Fab), Calcd for C₆₃H₁₀₄N₆NaO₄₆ [M+Na+] 1703.6, found, 1703.0

### Reference Example 29 Preparation of Compound 32

Compound 9 (7.7 mg, 4.4 µmoles) was reacted for 23 hours by the deprotection procedure described above, giving desired Compound 32 (5.2 mg, yield 78%). The physical data for the resulting compound are as follows.
¹H-NMR (30^{°C})
δ5.14(s, 1H, Man4-H-1), 5.07(d, 1H, J=9.4Hz, GlcNAc1-H-1), 4.78(s,1H, Man3-H-1), 4.61, 4,60(each d, each 1H, GlcNAc2,5-H-1), 4.44(d, 1H, J=8.0Hz, Gal6-H-1), 4.23(d, 1H, J=3.0Hz, Man3-H-2), 4.19(bdd, 1H, J=1.3Hz, 2.9Hz, Man4-H-2), 2.92(dd, 1H, J=4.1Hz, 17.2Hz, Asn-βCH), 2.83(dd, 1H, J=7.5Hz, 12.7Hz, Asn-βCH), 2.67(dd, 1H, J=4.6Hz, 12.7Hz, NeuAc7-H-3eq), 2.06(s, 6H, Ac ×2), 2.03, 2.01(each s, each 3H, Ac), 1.71(dd, 1H, J=12.7Hz, 12.7Hz, NeuAc7-H-3ax) ; MS (Fab), Calcd for C₅₇H₉₄N₆NaO₄₁ [M+Na+] 1541.5, found, 1541.2

### Reference Example 30 Preparation of Compound 37

Compound 14 (9.1 mg, 5.0 µmoles) was reacted for 13 hours by the deprotection procedure described above, giving desired Compound 37 (6.5 mg, yield 77%). The physical data for the resulting compound are as follows.
¹H-NMR(30°C)
β.11(s, 1H, Man4-H-1), 5.06(d, 1H, J=9.5Hz, GlcNAc1-H-1), 4.92(s, 1H, Man4'-H-1), 4.75(s, 1H, Man3-H-1), 4.61, 4.57, 4.55(each d, each 1H, J=7.5Hz, GlcNAc2,5,5'-H-1), 4.46(d, 1H, J=7.3Hz, Gal6'-H-1), 4.23(bs, 1H, Man3-H-2), 4.18(bs, 1H, Man4'-H-2), 4.10(bs, 1H, Man4-H-2), 2.87(dd, 1H, J=4.8Hz, 17.0Hz, Asn-βCH), 2.76(dd, 1H, J=7.2Hz, 17.0Hz, Asn-βCH), 2.07(s, 3H, Ac), 2.04(s, 6H, Ac ×2), 2.00(s, 3H, Ac) ; MS (Fab), Calcd for C₆₀H₁₀₀N₆NaO₄₃ [M+Na+] 1615.6, found, 1615.0

### Reference Example 31 Preparation of Compound 42

Compound 19 (9.8 mg, 5.4 µmoles) was reacted for 13 hours by the deprotection procedure described above, giving desired Compound 42 (8.0 mg, yield 88%). The physical data for the resulting compound are as follows.
¹H-NMR (30°C)
δ5.11(s, 1H, Man4-H-1), 5.06(d, 1H, J=9.5Hz, GlcNAc1-H-1), 4.91(s, 1H, Man4'-H-1), 4.76(s, 1H, Man3-H-1), 4.60, 4.57, 4.55(each d, each 1H, GlcNAc2,5,5'-H-1), 4.46(d, 1H, J=7.8Hz, Gal6-H-1), 4.28(s, 1H, Man3-H-2), 4.18(s, 1H, Man4'-H-2), 4.10(s, 1H, Man4-H-2), 2.88(dd, 1H, J=4.0Hz, 16.6Hz, Asn-βCH), 2.77(dd, 1H, J=7.5Hz,1 6.6Hz, Asn-βCH), 2.07(s, 3H, Ac), 2.04(s, 6H, Ac ×2), 2.00(s, 3H, Ac) ; MS (Fab), Calcd for C₆₀H₁₀₁N₆O₄₃ [M+H+] 1593.6, found, 1593.8

### Reference Example 32 Preparation of Compound 38

Compound 15 (5.1 mg, 3.2 µmoles) was reacted for 11 hours by the deprotection procedure described above, giving desired Compound 38 (4.0 mg, yield 91%). The physical data for the resulting compound are as follows.
¹H-NMR(30°C)
δ5.10(s, 1H, Man4-H-1), 5.07(d, 1H, J=9.4Hz, G1cNAc1-H-1), 4.92(s, 1H, Man4'-H-1), 4.76(s, 1H, Man3-H-1), 4.61, 4.57(each d, each 1H, J=7.8Hz, GlcNAc2,5'-H-1), 4.47(d, 1H, J=7.8Hz, Ga16'-H-1), 4.24(d, 1H, J=2.3Hz, Man3-H-2), 4.10, 4.06(each bd, each 1H, Man4',4-H-2), 2.90(dd, 1H, J=4.2Hz, 16.8Hz, Asn-βCH), 2.81(dd, 1H, J=7.3Hz, 16.8Hz, Asn-βCH), 2.07, 2.04, 2.01(each s, each 3H, Ac) ; MS(Fab), Calcd for C₅₂H₈₈N₅O₃₈ [M+H+] 1390.5, found, 1390.1

### Reference Example 33 Preparation of Compound 72

Compound 70 (4.0 mg, 2.8 µmoles) was reacted for 13 hours by the deprotection procedure described above, giving desired Compound 72 (2.9 mg, yield 85%). The physical data for the resulting compound are as follows.
¹H-NMR (30°C)
δ5.09(s, 1H, Man4-H-1), 5.06(d, 1H, J=9.8Hz, GlcNAc1-H-1), 4.91(s, 1H, Man4'-H-1), 4.76(s, 1H, Man3-H-1), 4.61, 4.54(each d, each 1H, GlcNAc2,5-H-1), 4.24(s, 1H, Man3-H-2), 4.10, 4.06(each bs, each 1H, Man4,4'-H-2), 2.87(dd, 1H, J=17.2Hz, Asn-βCH), 2.76(dd, 1H, J=6.5Hz, 17.2Hz, Asn-βCH), 2.07, 2.04, 2.00(each s, each 3H, Ac) ; MS (Fab), Calcd for C₄₆H₇₈N₅O₃₃ [M+H+] 1228.5, found, 1228.3

### Reference Example 34 Preparation of Compound 43

Compound 20 (5.4 mg, 3.3 µmoles) was reacted for 11 hours by the deprotection procedure described above, giving desired Compound 43 (4.1 mg, yield 87%). The physical data for the resulting compound are as follows.
¹H-NMR(30^{°C})
δ 5.11(s, 1H, Man4-H-1), 5.07(d, 1H, J=9.5Hz, GlcNAc1-H-1), 4.91(s, 1H, Man4'-H-1), 4.77(s, 1H, Man3-H-1), 4.61, 4.57(each d, each 1H, GlcNAc2,5-H-1), 4.46(d, 1H, Gal6-H-1), 4.24(s, 1H, Man3-H-2), 4.18(bs, 1H, Man4-H-2), 2.90(dd, 1H, J=4.0Hz, 17.0Hz, Asn-βCH), 2.80(dd, 1H, J=7.3Hz, 17.0Hz, Asn-βCH), 2.07, 2.04, 2.01(each s, each 3H, Ac) ; MS (Fab), Calcd for C₅₂H₈₈N₅O₃₈[M+H+] 1390.5, found, 1390.2

### Reference Example 35 Preparation of Compound 73

Compound 71 (4.0 mg, 2.8 µmoles) was reacted for 13 hours by the deprotection procedure described above, giving desired Compound 73 (2.9 mg, yield 85%). The physical data for the resulting compound are as follows.
¹H-NMR (30°C)
δ5.11(s, 1H, Man4-H-1), 5.06(d, 1H, J=9.9Hz, GlcNAc1-H-1), 4.91(s, 1H, Man4'-H-1), 4.77(s, 1H, Man3-H-1), 4.60, 4.54(each d, each 1H, J=7.9Hz, GlcNAc2,5-H-1), 4.24(s, 1H, Man3-H-2), 4.18(dd, 1H, J=1.6Hz, 1.6Hz, Man4-H-2), 3.96(1H, dd, J=1.6Hz, 1.6Hz, Man4-H-2), 2.88(dd, 1H, J=4.3Hz, 16.8Hz, Asn-βCH), 2.77(dd, 1H, J=7.2Hz, 16.8Hz, Asn-βCH), 2.06, 2.04, 2.00(each s, each 3H, Ac) ; MS(Fab), Calcd for C₄₆H₇₈N₅O₃₃ [M+H+] 1228.5, found, 1228.3

### Reference Example 36 Preparation of Compound 39

Compound 16 (2.2 mg, 1.5 µmoles) was reacted for 7 hours by the deprotection procedure described above, giving desired Compound 39 (1.6 mg, yield 84%). The physical data for the resulting compound are as follows.
¹H- NMR (30°C)
δ5.07(d, 1H, J=9.7Hz, GlcNAc1-H-1), 4.92(s, 1H, Man4'-H-1), 4.75(s, 1H, Man3-H-1), 4.62, 4.58(each d, each 1H, GlcNAc2,5-H-1), 4.09, 4.08(each s, each 1H, Man3,4'-H-2), 2.91(dd, 1H, J=4.1Hz, 16.9Hz, Asn-βCH), 2.81(dd, 1H, J=6.8Hz, 16.9Hz, Asn-βCH), 2.08, 2.04, 2.01 (each s, each 3H, Ac) ; MS (Fab), Calcd for C₄₆H₇₇N₅NaO₃₃ [M+Na+] 1250.4, found, 1250.3

### Reference Example 37 Preparation of Compound 40

Compound 17 (1.5 mg, 1.2 µmoles) was reacted for 14 hours by the deprotection procedure described above, giving desired Compound 40 (1.1 mg, yield 89%). The physical data for the resulting compound are as follows.
¹H-NMR (30°C)
δ5.07(d, 1H, J=9.5Hz, GlcNAc1-H-1), 4.91(s, 1H, Man4'-H-1), 4.76(s, 1H, Man3-H-1), 4.62, 4.55(each d, each 1H, GlcNAc2,5-H-1), 4.10, 4.07(each s, each 1H, Man4', 3-H-2), 2.89(dd, 1H, J=3.7Hz, 17.0Hz, Asn-βCH), 2.79(dd, 1H, J=7.0Hz, 17.0Hz, Asn-βCH), 2.07, 2.05, 2.01 (each s, each 3H, Ac) ; MS (Fab), Calcd for C₄₀H₆₇N₅NaO₂₈ [M+Na+] 1088.4, found, 1088.2

### Reference Example 38 Preparation of Compound 41

Compound 18 (1.3 mg, 1.2 µmoles) was reacted for 14 hours by the deprotection procedure described above, giving desired Compound 41 (0.8 mg, yield 80%). The physical data for the resulting compound are as follows.
¹H-NMR (30°C)
δ 5.07 (d, 1H, J=9.5Hz, GlcNAc1-H-1), 4.91(s, 1H, Man4'-H-1), 4.76(s, 1H, Man3-H-1), 4.62(d, 1H, J=7.8Hz, GlcNAc2-H-1), 4.08(d, 1H, J=2.9Hz, Man3-H-2), 2.92(dd, 1H, J=3.9Hz, 17.3Hz, Asn-βCH), 2.83(dd, 1H, J=7.0Hz, 17.3Hz, Asn-βCH), 2.07, 2.01(each s, each 3H, Ac) ; MS(Fab), Calcd for C₃₂H₅₅N₄O₂₇ [M+H+] 863.3, found 863.2

### Reference Example 39 Preparation of Compound 44

Compound 21 (2.3 mg, 1.6 µmoles) was reacted for 7 hours by the deprotection procedure described above, giving desired Compound 44 (1.6 mg, yield 84%). The physical data for the resulting compound are as follows.
¹H-NMR(30°C)
δ5.11(s, 1H, Man4-H-1), 5.06(d, 1H, J=9.8Hz, GlcNAc1-H-1), 4.77(s, 1H, Man3-H-1), 4.61, 4.57(each d, each 1H, GlcNAc2,5-H-1), 4.46(d, 1H, J=7.8Hz, Gal-H-1), 4.22, 4.18(each bs, each 1H, Man3,4-H-2), 2.91(dd, 1H, J=4.1Hz, 17.3Hz, Asn-βCH), 2.82(dd, 1H, J=7.0Hz, 17.3Hz, Asn-βCH), 2.05, 2.04, 2.01(each s, each 3H, Ac) ; MS(Fab), Calcd for C₄₆H₇₈N₅O₃₃ [M+H+] 1228.5, found, 1228.3

### Reference Example 40 Preparation of Compound 45

Compound 22 (1.6 mg, 1.3 µmoles) was reacted for 14 hours by the deprotection procedure described above, giving desired Compound 45 (1.1 mg, yield 85%). The physical data for the resulting compound are as follows.
¹H-NMR (30°C)
δ5.12(s, 1H, Man4-H-1), 5.07(d, 1H, J=9.7Hz, G1cNAc1-H-1), 4.77(s, 1H, Man3-H-1), 4.61,4 .54(each d, each 1H, GlcNAc2,5-H-1), 4.22(d, 1H, J=2.5Hz, Man3-H-2), 4.18(dd, 1H, J=1.4Hz, 3.0Hz, Man4'-H-2), 2.89(dd, 1H, J=4.3Hz, 16.9Hz, Asn-βCH), 2.78(dd, 1H, J=7.5Hz, 16.9Hz, Asn-βCH), 2.06, 2.05, 2.01(each s, each 3H, Ac) ; MS(Fab), Calcd for C₄₀H₆₇N₅NaO₂₈ [M+Na+] 1088.4, found, 1088.3

### Reference Example 41 Preparation of Compound 46

Compound 23 (1.6 mg, 1.5 µmoles) was reacted for 14 hours by the deprotection procedure described above, giving desired Compound 46 (1.1 mg, 6.4 µmoles, yield 85%). The physical data for the resulting compound are as follows.
¹H - NMR (30°C)
δ5.10(s, 1H, Man4-H-1), 5.06(d, 1H, J=9.5Hz, GlcNAc1-H-1), 4.77(s, 1H, Man3-H-1), 4.61(d,1 H, J=7.3Hz, GlcNAc2-H-1), 4.22(d, 1H, J=2.4Hz, Man3-H-2), 4.07(dd, 1H, J=1.6Hz, 3.0Hz, Man4'-H-2), 2.90(dd, 1H, J=4.3Hz, 17.0Hz, Asn-βCH), 2.80(dd, 1H, J=7.0Hz, 17.2Hz, Asn-βCH), 2.05, 2.01(each s, each 3H, Ac); MS(Fab), Calcd for C₃₂H₅₅N₄O₂₃ [M+H+] 863.3, found 863.3

### Reference Example 42 Preparation of Compound 34

Compound 11 (12.4 mg, 7.5 µmoles) was reacted for 11 hours by the deprotection procedure described above, giving desired Compound 34 (9.2 mg, yield 86%). The physical data for the resulting compound are as follows.
¹H-NMR (30°C)
δ5.11(s, 1H, Man4-H-1), 5.07(d, 1H, J=10.0Hz, GlcNAc1-H-1), 4.91(s, 1H, Man4'-H-1), 4.77(s, 1H, Man3-H-1), 4.61(d, 1H, J=6.8Hz, GlcNAc2-H-1), 4.55(d, 2H, GlcNAc5,5'-H-1), 4.24(bs, 1H, Man3-H-2), 4.18(bs, 1H, Man4'-H-2), 4.10(bs, 1H, Man4-H-2), 2.80(dd, 1H, J=3.8Hz, 15.6Hz, Asn-βCH), 2.63(dd, 1H, J=8.2Hz, 15.6Hz, Asn-βCH), 2.07(s, 3H, Ac), 2.05(s, 6H, Ac ×2), 2.01(s, 3H, Ac) ; MS(Fab), Calcd for C₅₄H₉₀N₆Na0₃₈ [M+Na+] 1453.5, found, 1453.2

### Reference Example 43 Preparation of Compound 35

Compound 12 (12.0 mg, 8.4 µmoles) was reacted for 11 hours by the deprotection procedure described above, giving desired Compound 35 (7.0 mg, yield 81%). The physical data for the resulting compound are as follows.
¹H -NMR (30°C)
δ5.10(s, 1H, Man4-H-1), 5.07(d, 1H, J=9.7Hz, GlcNAc1-H-1), 4.91(s, 1H, Man4'-H-1), 4.78(s, 1H, Man3-H-1), 4.61(d, 1H, J=8.0Hz, GlcNAc2-H-1), 4.25(bs, 1H, Man3-H-2), 4.06(bs, 1H, Man4'-H-2), 3.97(bs, 1H, Man4-H-2), 2.79(dd, 1H, J=5.0Hz, 17.0Hz, Asn-βCH), 2.61(dd, 1H, J=7.3Hz, 17.0Hz, Asn-βCH), 2.07, 2.01 (each s, each 3H, Ac) ; MS (Fab), Calcd for C₃₈H₆₅N₄O₂₈ [M+H+] 1025.4, found, 1025.2

### Reference Example 44 Preparation of Compound 36

Compound 13 (8.4 µmoles) was reacted for 11 hours by the deprotection procedure described above, giving desired Compound 36.

### Reference Example 45

### Preparation and isolation of Compounds 76 and 77

The mixture of Compounds 2 and 6 (5.0 mg, 2.2 µmol) were dissolved in 220 µL of water, and 100 µL of a 22 mM aqueous cesium carbonate was added thereto to adjust its pH 7.0. This solution was freeze-dried. Four-hundred and thirty microliters of N,N-dimethylformamide was added to the solid obtained after drying, and further 20 µL of a 6.6 µmol benzyl bromide/N,N-dimethylformamide solution was added thereto. This solution was stirred under argon atmosphere. After 48 hours, the disappearance of the starting material was confirmed by TLC (eluent: 1M NH₄OAc:isopropanol=1:2), and thereafter 4.4 mL of diethyl ether was added to the solution to allow the compound to precipitate therefrom. The precipitated oligosaccharides were filtered, and the residual oligosaccharide was dissolved in water and freeze-dried. The residue after the lyophilization was purified by fractional HPLC (YMC Packed Column D-ODS-5 S-5 120A ODS No. 2020178, 20 × 250 mm, eluent: 50 mM aqueous ammonium acetate : acetonitrile=78:22, flow rate: 4 mL/min), and Compound 77 was eluted after 88 minutes and Compound 76 was eluted after 91 minutes. The fractions were collected, and further desalted on an ODS column (Cosmosil 75C18-OPN, 15 × 100 mm, eluted first with 50 mL of H₂O and then with 25% acetonitrile), to give a benzyl derivative of Compound 2 in an amount of 1.6 mg and a benzyl derivative of Compound 6 in an amount of 1.8 mg.

A benzyl compound of Compound 2 (decasaccharide, 13.6 mg, 5.8 mmoles) was dissolved in 1.4 ml of NaOH aq. (pH=12) with ice cooling. The solution was stirred for about 8 hours while monitoring the reaction by HPLC. On completion of progress of the reaction, the reaction mixture was adjusted to a PH of 7.0 with 40 mM of HCl. The mixture resulting from neutralization was filtered by a membrane filter, followed by concentration, and fractionation and purification by HPLC (YMC-pack ODS-AM, SH-343-5AM, 20 × 250 mm, AN/25 mM AcONH₄ buffer=20/80, 7.0 ml/min., wave length: 274 nm). The fraction obtained was concentrated and passed through an ODS column (Cosmoseal 75C₁₈-OPN, product of NACALAI TESQUE, INC.) for a desalting treatment, followed by concentration and freeze-drying to obtain Compound 2 (6.2 mg, 47.4%) as a pure product.

To Compound 2 (4.0 mg, 1.76 mmoles) obtained was added 282 ml of DMSO to obtain a solution. The solution was stirred at room temperature with addition of 282 ml of morpholine. The mixture was reacted for about 1 hour while the reaction was being minitored by TLC. The disappearance of the starting material was recognized, and 5 ml of a solution of acetone and diisopropyl ether (IPE) in the ratio of 1:1 was thereafter added to the reaction mixture. The resulting slurry was filtered with a membrane filter, and the residue was eluted with purified water. The aqueous layer was concentrated, and the concentrate was purified by gel column chromatography (Sephadex G-25, H₂O). The fractions containing the desired product were collected and concentrated, and the concentrate was freeze-dried, giving desired asparagine-linked monosialooligosaccharide (Compound 76)(3.3 mg, yield 91.5%). Given below is the NMR data as to Compound 76.
¹H-NMR (400MHz, D₂O, 30°C, HOD=4.81)
δ 5.22(s, 1H, Man4-H1), 5.15(d, 1H, J=10.0, GlcNAc1-H1), 5.01(s, 1H, Man4'-H-1), 4.85(s, 1H), 4.65-4.75(m, 3H), 4.54(dd, 2H, Ja=10.8, Jb=7.6), 4.33(s, 1H), 4.27(bd, 1H, J=2.4, Man4-H2), 4.19(bd, 1H, J=2.4), 2.95(dd, 1H, Ja=16.8, Jb=4.4, Asn-βCH), 2.82(dd, 1H, Ja=16.8, Jb=7.2, Asn-βCH), 2.74(dd, 1H, Ja=12.4, Jb=4.4, NeuAc7-H3eq), 2.16, 2.15, 2.13, 2.11, 2.09(eachs, 15H, Acx5), 1.83(dd, 1H, Ja=12.4, Jb=12.0, NeuAc7-H3ax).

A benzyl compound of Compound 6 (decasaccharide, 5.0 mg, 2.1 mmoles) was dissolved in 2.0 ml of NaOH aq. (pH=12) with ice cooling. The solution was stirred for about 5 hours while monitoring the reaction by HPLC. On completion of progress of the reaction, the reaction mixture was adjusted to a PH of 7.0 with 40 mM of HCl. The mixture resulting from neutralization was filtered by a membrane filter, followed by concentration, and fractionation and purification by HPLC (YMC- pack ODS-AM, SH-343-5AM, 20 × 250 mm, AN/25 mM AcONH₄ buffer=20/80, 7.0 ml/min., wave length: 274 nm). The fraction obtained was concentrated and passed through an ODS column (Cosmoseal 75C₁₈-OPN, product of NACALAI TESQUE, INC.) for a desalting treatment, followed by concentration and freeze-drying to obtain the desired product, i.e., Compound 6 (2.5 mg, 52.0%) as a pure product.

To Compound 6 (1.5 mg, 0.67 mmoles) obtained was added 105 ml of DMSO to obtain a solution. The solution was stirred at room temperature with addition of 105 ml of morpholine. The mixture was reacted for about 1 hour while the reaction was being minitored by TLC. The disappearance of the starting material was recognized, and 5 ml of a solution of acetone and diisopropyl ether (IPE) in the ratio of 1:1 was thereafter added to the reaction mixture. The resulting slurry was filtered with a membrane filter, and the residue was eluted with purified water. The aqueous layer was concentrated, and the concentrate was purified by gel column chromatography (Sephadex G-25, H₂O). The fractions containing the desired product were collected and concentrated, and the concentrate was freeze-dried, giving desired asparagine-linked monosialooligosaccharide (Compound 77)(1.4 mg, yield 99%). Given below is the NMR data as to Compound 77.
¹H-NMR(400MHz, D₂O, 30°C, HOD=4.81)
δ 5.20(s, 1H, Man4-H1), 5.15(d, 1H, J=9.6, GlcNAc1-H1), 5.02(s, 1H, Man4'-H-1), 4.85(s, 1H), 4.63-4.73(m, 3H), 4.53(dd, 2H, Ja=7.6, Jb=7.2), 4.33(s, 1H), 4.27(bd, 1H, J=2.4, Man4-H2), 4.19(d, 1H, J=2.0), 2.83(dd, 1H, Ja=16.0, Jb=4.0, Asn-βCH), 2.75(dd, 1H, Ja=12.4, Jb=4.8, NeuAc7-H3eq), 2.62(dd, 1H, Ja=16.0, Jb=8.0, Asn-β CH), 2.16, 2.14, 2.13, 2.11, 2.09(eachs, 15H, Acx5), 1.79(dd, 1H, Ja=12.4, Jb=11.6, NeuAc7-H3ax).

### Example 1

Compound 24 (6 mg, 2.58 mmoles) obtained in Reference Example 1 was dissolved in water (300 ml), and sodium bicarbonate (2.1 mg, 24.9 mmoles) was added to the solution. A solution of D-(+)-biotinylsuccinimide (4.2 mg, 12.3 mmoles) in dimethylformamide (300 ml) was added to the mixture, followed by reaction at room temperature for 20 minutes. The disappearance of the starting material was confirmed by TLC (isopropanol:1M ammonium acetate aqueous solution=3:2), and the reaction mixture was concentrated using an evaporator. The residue was purified with a gel filtration column (f20 mm × 300 mm, Sephadex G-25, water), affording the desired compound (6.2 mg, 94%). The physical data as to the compound obtained is given below. 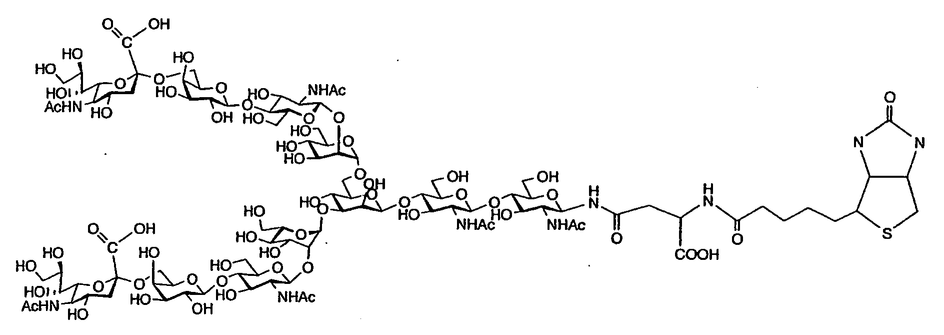 ¹H-NMR(400mHz, D₂O, 30°C, HOD=4.81)
d5.22(s, 1H, Man4-H1), 5.14(d, 1H, GlcNAc1-H1), 5.03(s, 1H, Man4'-H-1), 4.59(dd, 1H), 4.86(s, 1H, Man3-H1), 4.74-4.66(m, 3H, GlcNAc2,5,5'-H1), 4.53(d, 2H, Ga16,6'-H1), 4.34(s, 1H, Man3-H2), 4.28(bs, 1H, Man4-H2), 4.19(bs, 1H, Man4'-H2), 3.09(dd, 2H, NeuAc7,7'-H3eq), 2.94-2.86(m, 2H, biotin), 2.78-2.71(m, 2H, biotin), 2.37(t, 1H, biotin), 2.17, 2.16, 2.13, 2.11(each s, Ac), 1.80(dd, 2H, NeuAc7,7'-H3ax), 1.80-1.67(m, biotin), 1.52-1.47(m, biotin), 1.32(dd, biotin)

### Example 2

Compound 76 obtained in Reference Example 45 was biotinated in the same manner as in Example 1.

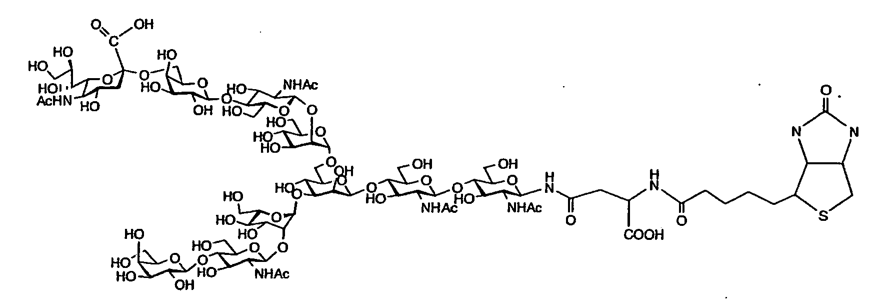

### Example 3

Compound 77 obtained in Reference Example 45 was biotinated in the same manner as in Example 1.

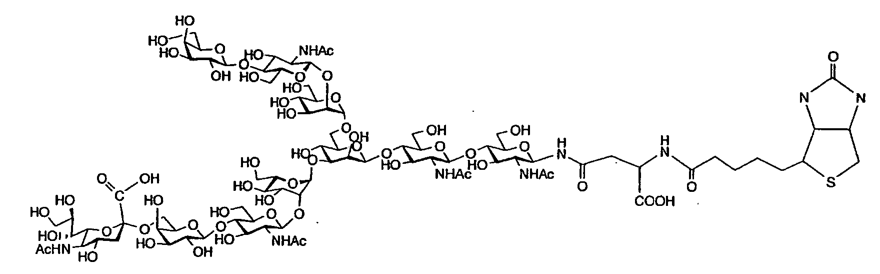

### Example 4

Compound 33 obtained in Reference Example 7 was biotinated in the same manner as in Example 1.

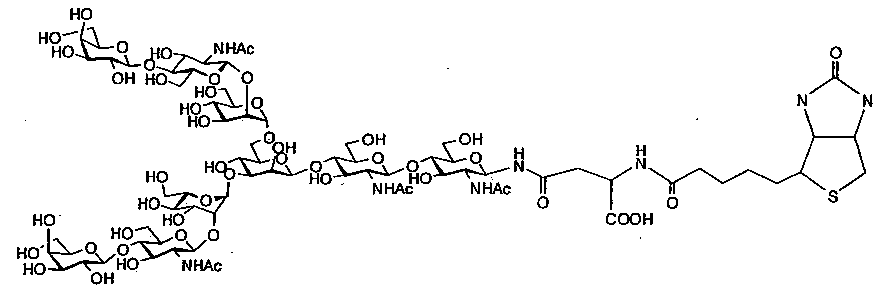

### Example 5

Compound 26 obtained in Reference Example 24 was biotinated in the same manner as in Example 1.

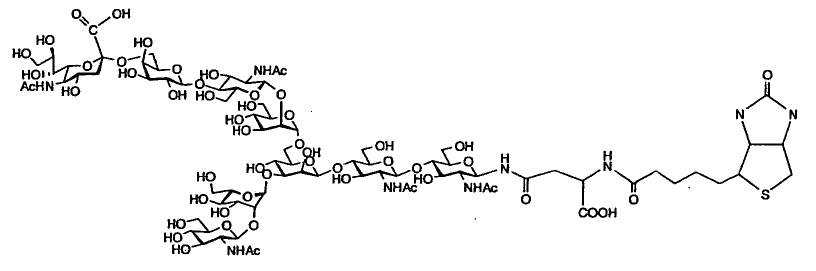

### Example 6

Compound 27 obtained in Reference Example 25 was biotinated in the same manner as in Example 1.

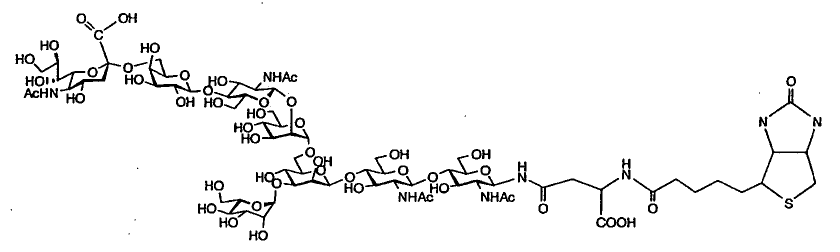

### Example 7

Compound 28 obtained in Reference Example 26 was biotinated in the same manner as in Example 1.

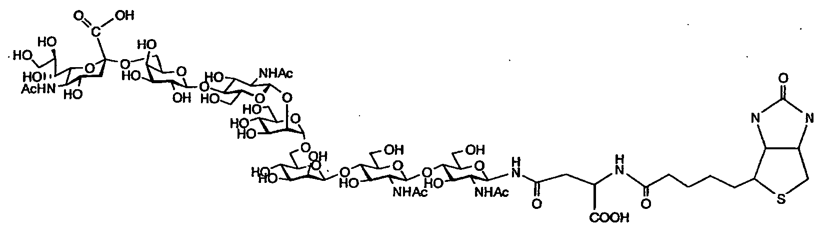

### Example 8

Compound 30 obtained in Reference Example 27 was biotinated in the same manner as in Example 1.

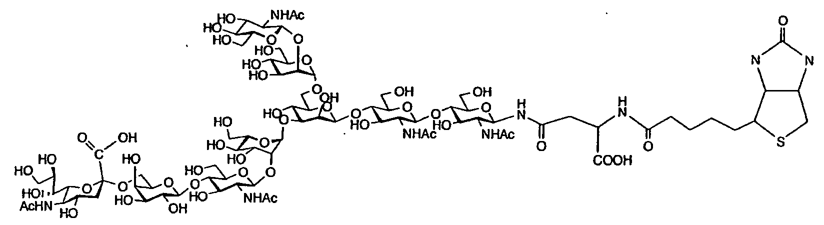

### Example 9

Compound 31 obtained in Reference Example 28 was biotinated in the same manner as in Example 1.

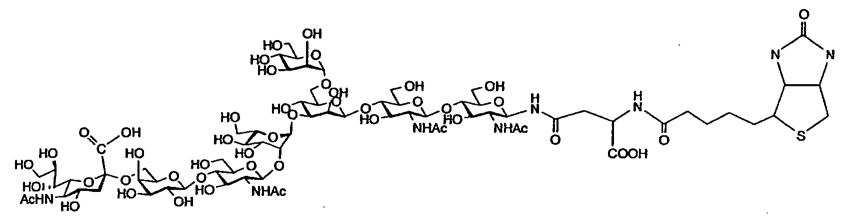

### Example 10

Compound 32 obtained in Reference Example 29 was biotinated in the same manner as in Example 1.

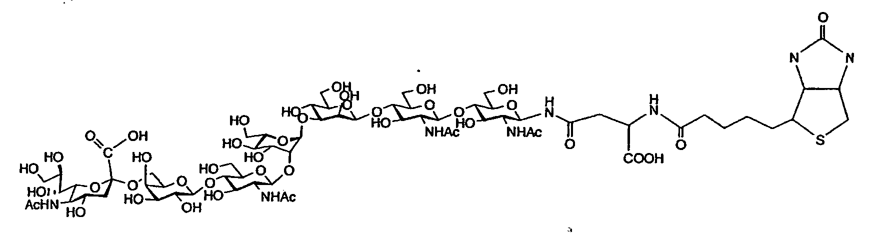

### Example 11

Compound 37 obtained in Reference Example30 was biotinated in the same manner as in Example 1.

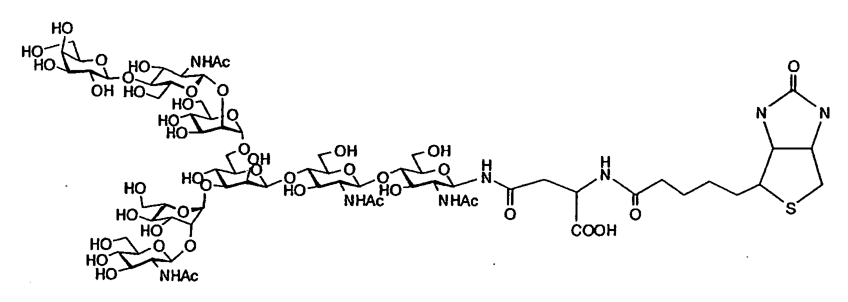

### Example 12

Compound 42 obtained in Reference Example 31 was biotinated in the same manner as in Example 1.

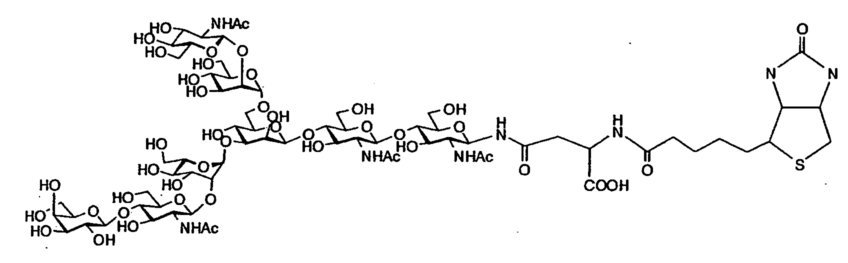

### Example 13

Compound 38 obtained in Reference Example 32 was biotinated in the same manner as in Example 1.

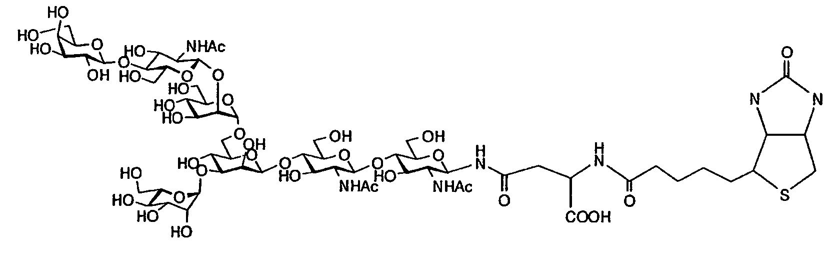

### Example 14

Compound 72 obtained in Reference Example 33 was biotinated in the same manner as in Example 1.

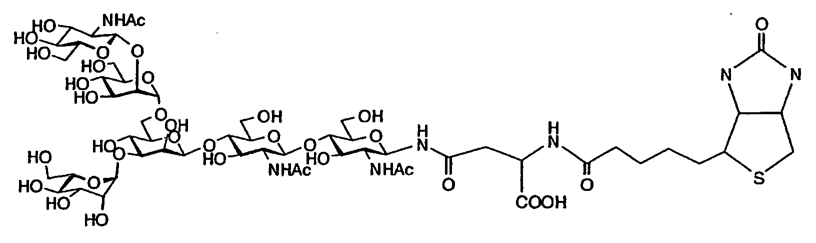

### Example 15

Compound 43 obtained in Reference Example 34 was biotinated in the same manner as in Example 1.

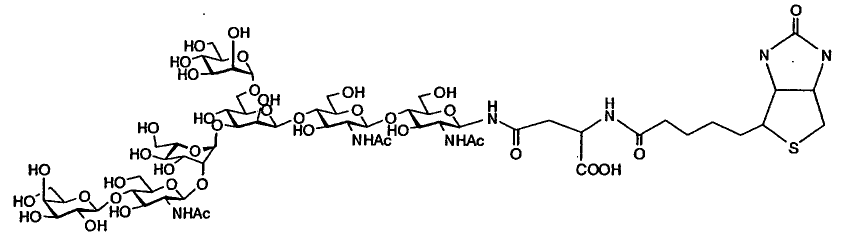

### Example 16

Compound 73 obtained in Reference Example 35 was biotinated in the same manner as in Example 1.

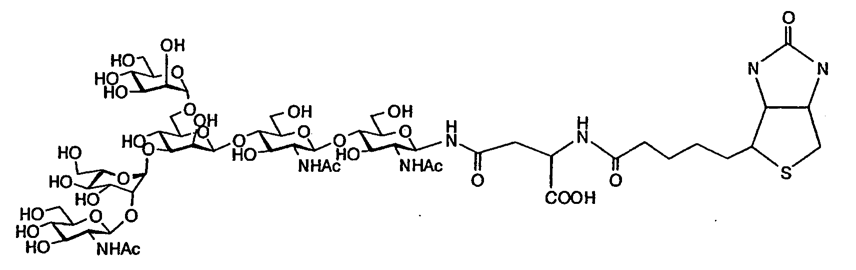

### Example 17

Compound 39 obtained in Reference Example 36 was biotinated in the same manner as in Example 1.

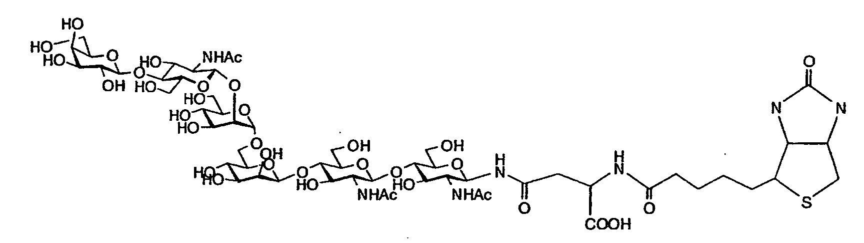

### Example 18

Compound 40 obtained in Reference Example 37 was biotinated in the same manner as in Example 1.

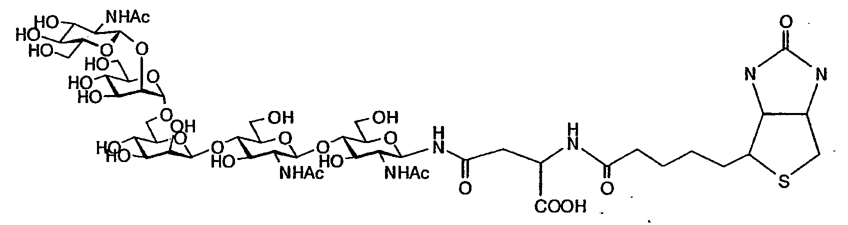

### Example 19

Compound 41 obtained in Reference Example 38 was biotinated in the same manner as in Example 1.

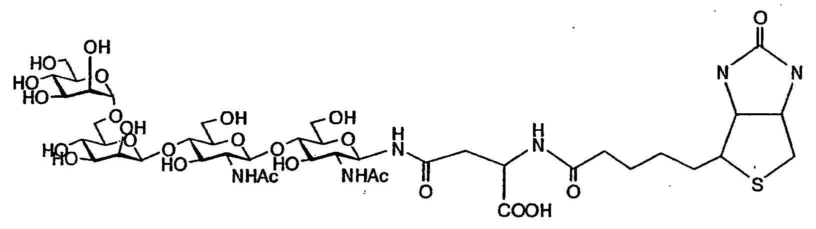

### Example 20

Compound 44 obtained in Reference Example 39 was biotinated in the same manner as in Example 1.

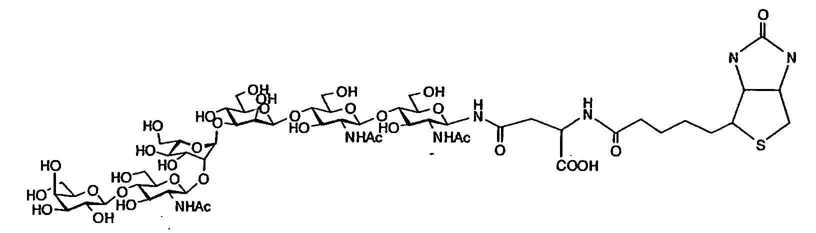

### Example 21

Compound 45 obtained in Reference Example 40 was biotinated in the same manner as in Example 1.

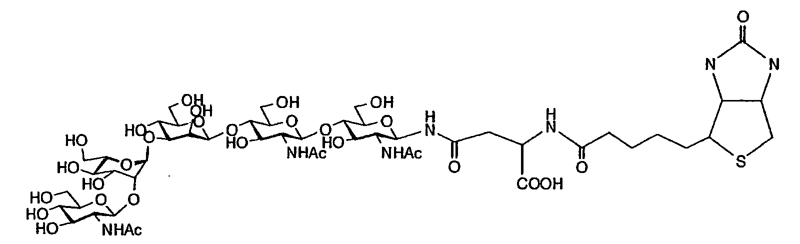

### Example 22

Compound 46 obtained in Reference Example 41 was biotinated in the same manner as in Example 1.

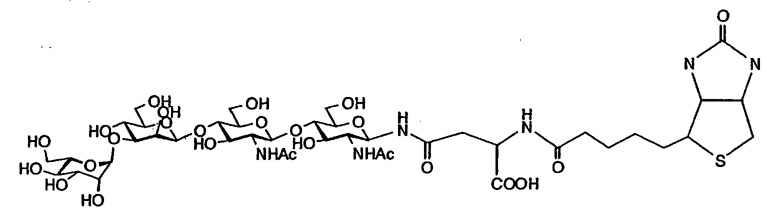

### Example 23

Compound 34 obtained in Reference Example 42 was biotinated in the same manner as in Example 1.

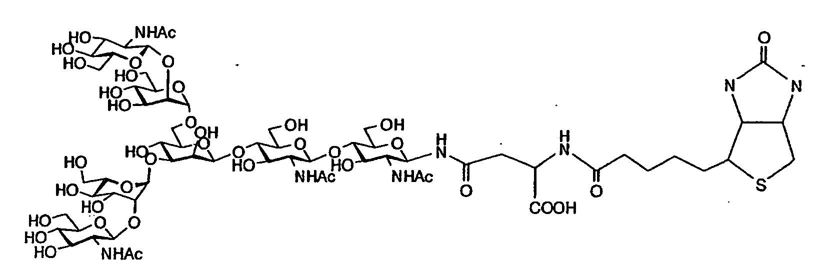

### Example 24

Compound 35 obtained in Reference Example 43 was biotinated in the same manner as in Example 1.

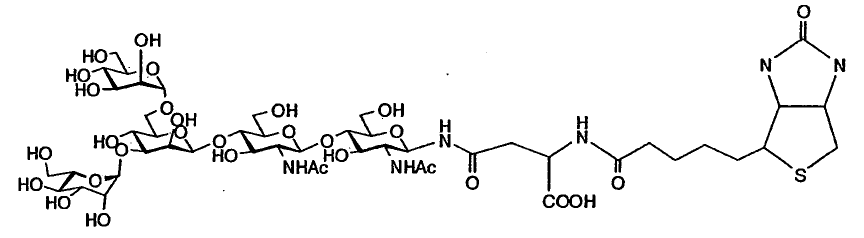

### Example 25

Compound 36 obtained in Reference Example 44 was biotinated in the same manner as in Example 1.

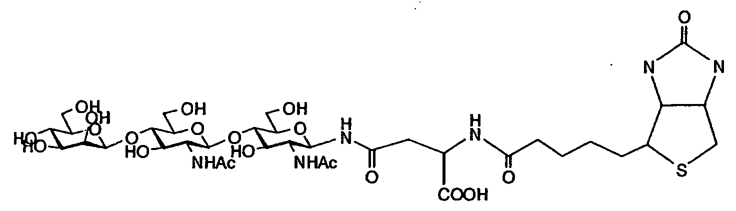

### Example 26

Preparation of asparagine-linked disialo α2,3-oligosaccharide (C1-1) wherein the amino group of the asparagine is protected with Fmoc group and two kinds of asparagine-linked monoasilo α2,3-oligosaccharides (C1-2 and C1-3) wherein the amino group of the asparagine is protected with Fmoc group.

Using sialic acid transferase, CMP-sialic acid was transferred to the asparagine-linked asialooligosaccharide which was obtained in Reference Example 2 and wherein the amino group of the asparagine was protected with Fmoc group.

The sialic acid transferase used was α2,3-transferase which was commercially available and derived from a rat recombinant.

The asialononasaccharide (20 mg, 10.1 µmoles) obtained in Reference Example 2 was dissolved in 50 mM of cacodylic acid buffer (6.0 in pH, 5 ml), and bovine serum albumin (BSA, 5 mg) was then added to the solution. To the mixture were added CMP-sialic acid (26 mg, 40.4 µmoles) and alkanline phosphatase (5 µl, 125 units), and the resulting mixture was stirred uniformly. Finally, α2,3-sialyltransferase (product of CALBIOCHEM, 100 µl) was added to the mixture, and the resulting mixture was allowed to stand at 37°C for 48 hours. The reaction was terminated upon the starting material reducing to the desired quantity while monitoring the reaction by HPLC, and the reaction mixture was filtered with a membrane filter. The filtrate was concentrated to reduce the quantity thereof and thereafter purified by HPLC (YMC-Pack R&D ODS, D-ODS-5-A, 20 × 250 mm, AN/25 mM AcONH₄ buffer = 18/82, 7.5 ml/min., wave length: 274 nm). The eluates obtained were disialoundecasaccharide compound (C1-1) in 25 minutes, and monosialodecasaccharide compounds (C1-2) and (C1-3) in 30 minutes and 34 minutes, respectively. The fractions were collected, desalted and freeze-dried individually, giving Compounds 1, 2 and 3 in respective amounts of 0.7 mg (2.7%), 1.9 mg (8.3%) and 3.5 mg (15.3%). The NMR data as to the compounds is given below.

### Compound (C1-1)

¹H NMR (400MHz, D₂O, 30°C, HOD=4.81)
δ 7.90(d, 2H, Fmoc), 7.69(d, 2H, Fmoc), 7.49(dd, 2H, Fmoc), 7.42(dd, 2H, Fmoc), 5.10(s, 1H, Man4-H1), 4.97(d, 1H, GlcNAc1-H1), 4.91(s, 1H, Man4'-H-1), 4.50-4.60(m, 4H), 4.34(1H, Fmoc), 4.24(bs, 1H, Man3-H2), 4.18(bs, 1H, Man4-H2), 4.10(m, 2H), 2.74(m, 3H, Asn-βCH, NeuAc7,7'-H3eq), 2.40-2.60(m, 1H, Asn-βCH), 2.05, 2.03, 2.02(each s, Ac), 1.77(dd, 2H, NeuAc7,7'-H3ax).

### Compound (C1-2)

¹H NMR(400MHz, D₂O, 30°C, HOD=4.81)
δ 7.90(d, 2H, Fmoc), 7.69(d, 2H, Fmoc), 7.49(dd, 2H, Fmoc), 7.42(dd, 2H, Fmoc), 5.10(s, 1H, Man4-H1), 4.97(d, 1H, GlcNAc1-H1), 4.90(s, 1H, Man4'-H-1), 4.47-4.60(m), 4.43(d, 1H), 4.32(1H, Fmoc), 4.22(bs, 2H), 4.17(bs, 1H, Man4-H2), 4.06-4.13(m, 2H), 2.72(m, 2H, Asn-βCH, NeuAc7-H3eq), 2.50-2.60(m, 1H, Asn-βCH), 2.05, 2.03, 2.01(each s, Ac), 1.77(dd, 1H, NeuAc7-H3ax).

### Compound (C1-3)

¹H NMR (400MHz, D₂O, 30°C, HOD=4.81)
δ 7.90(d, 2H, Fmoc), 7.69(d, 2H, Fmoc), 7.49(dd, 2H, Fmoc), 7.42(dd, 2H, Fmoc), 5.10(s, 1H, Man4-H1), 4.97(d, 1H, GlcNAc1-H1), 4.90(s, 1H, Man4'-H-1), 4.50-4.60(m), 4.45(d, 1H), 4.33(1H, Fmoc), 4.22(m, 2H), 4.17(bs, 1H, Man4-H2), 4.09(m, 2H), 2.74(m, 2H, Asn-β CH, NeuAc7-H3eq), 2.45-2.60(m, 1H, Asn-βCH), 2.05, 2.03, 2.02, 2.00(each s, Ac), 1.77(dd, 1H, NeuAc7-H3ax)

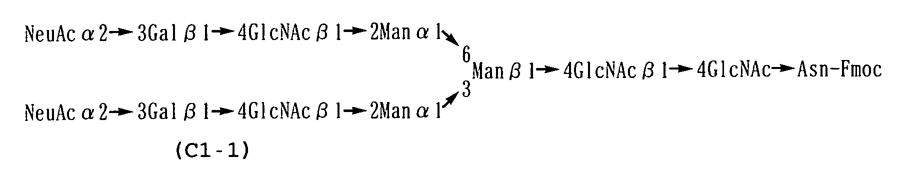

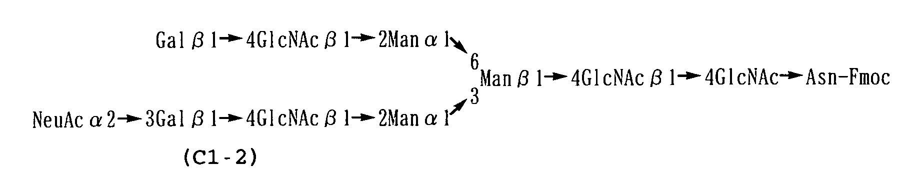

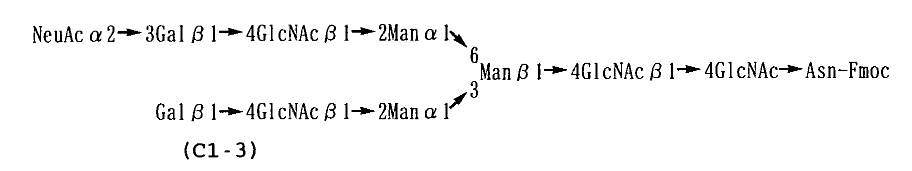

Each of Compounds (C-1)~(C-3) obtained was treated in the same manner as in Reference Example 7 to remove Fmoc group to prepare an asparagine-linked oligosaccharide. The obtained asparagine-linked oligosaccharide was biotinated in the same manner as in Example 1.

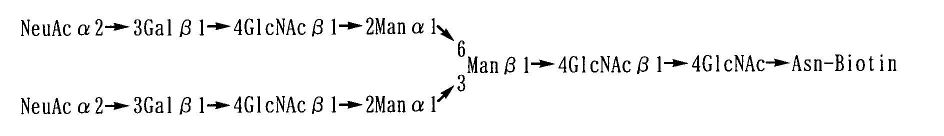

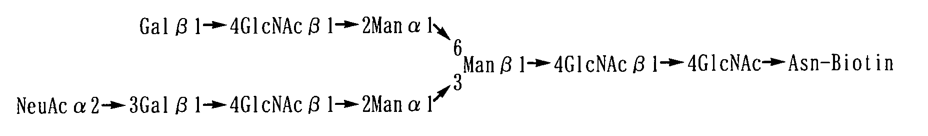

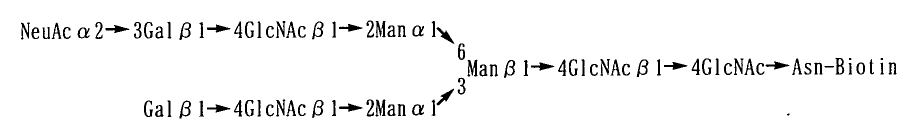

### Example 27

Compound (C1-2) (2 mg, 0.88 µmole) obtained in Example 26 and 1 mg of bovine serum albumin were dissolved in 100 µl of HEPES buffer solution (50 mM, pH 5.0), and β-galactosidase (product of Seikagaku Corp.., from Jack Beans, 5 µl, 100 mU) was added to the solution. The resulting solution was allowed to stand at 37°C for 15 hours, and thereafter filtered with a membrane filter. The filtrate was purified by HPLC [ODS column, 2.0 (diam.) × 25 cm; eluent: 50 mM aqueous solution of ammonium acetate : acetonitrile = 82:18; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.5 µg of the desired Compound (C2). The NMR data is given below.
¹H NMR(400MHz, D₂O, 30°C, HOD=4.81)
δ 7.90(d, 2H, Fmoc), 7.69(d, 2H, Fmoc), 7.49(dd, 2H, Fmoc), 7.42(dd, 2H, Fmoc), 5.10(s, 1H, Man4-H1), 4.98(d, 1H, GlcNAc1-H1), 4.90(s, 1H, Man4'-H-1), 4.50-4.60(m), 4.33(1H, Fmoc), 4.22(m, 2H), 4.17(bs, 1H, Man4-H2), 4.10(m, 2H), 2.74(m, 2H, Asn-β CH, NeuAc7-H3eq), 2.45-2.60(m, 1H, Asn-βCH), 2.05, 2.03, 2.01(each s, Ac), 1.78(dd, 1H, NeuAc7-H3ax)

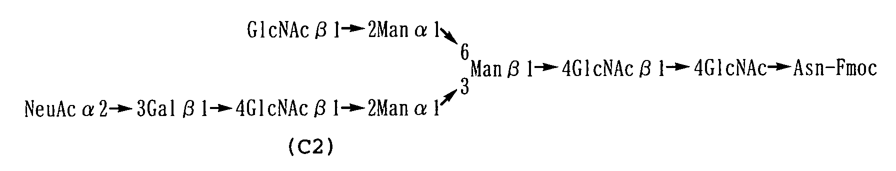

Compound (C2) obtained was treated in the same manner as in Reference Example 7 to remove Fmoc group to prepare an asparagine-linked oligosaccharide. The obtained asparagine-linked oligosaccharide was biotinated in the same manner as in Example 1.

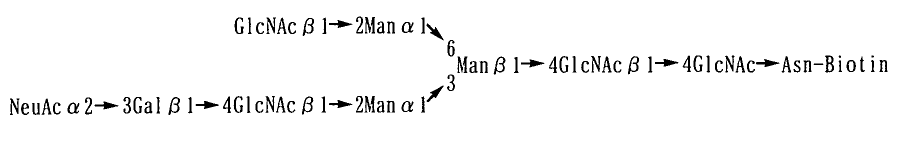

### Example 28

Compound (C2) (1.8 mg, 0.86 µmole) obtained in Example 27 and 1 mg of bovine serum albumin were dissolved in 90 µl of HEPES buffer solution (50 mM, pH 5.0), and 4 µl (250 mU) of N-acetyl-β-glucosamidase (product of Sigma-Aldrich Corp., from Jack Beans) was added to the solution. The resulting solution was allowed to stand at 37°C for 24 hours, and thereafter filtered with a membrane filter. The filtrate was purified by HPLC [ODS column, 2.0 (diam.) × 25 cm; eluent: 50 mM aqueous solution of ammonium acetate : acetonitrile = 82:18; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.9 µg of the desired Compound (C3).
¹H NMR (400MHz, D₂O 30°C, HOD=4.81)
δ 8.01 (d, 2H, J=7.6, Fmoc), 7.80(d, 2H, J=7.6, Fmoc), 7.60(dd, 2H, J=7.6, Fmoc), 7.53(dd, 2H, J=7.6, Fmoc), 5.21(s, 1H, Man4-H1), 5.09(d, 1H, J=8.8, GlcNAc1-H1), 5.00(s, 1H, Man4'-H-1), 4.87(s, 1H), 4.60-4.78(m, 5H), 4.40-4.50(bm, 2H), 4.34(s, 1H), 4.28(bs, 1H, Man4-H2), 4.20(dd, 1H, Ja=3.0, Jb=9.9), 2.80-2.95(m, 2H, Asn-βCH, NeuAc7-H3eq), 2.65-2.75(m, 1H, Asn-βCH), 2.16, 2.14, 2.12(eachs, Acx3), 1.98(s, 3H, Ac), 1.89(dd, 1H, Ja=12.1, Jb=11.9, NeuAc7-H3ax).

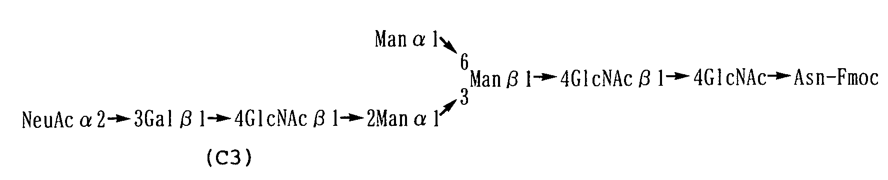

Compound (C3) obtained was treated in the same manner as in Reference Example 7 to remove Fmoc group to prepare an asparagine-linked oligosaccharide. The obtained asparagine-linked oligosaccharide was biotinated in the same manner as in Example 1.

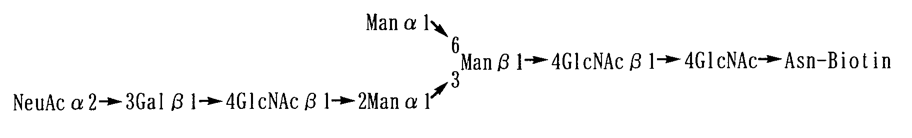

### Example 29

Compound (C3) (0.8 mg, 0.42 µmole) obtained in Example 28 and 1 mg of bovine serum albumin were dissolved in 50 µl of HEPES buffer solution (50 mM, pH 5.0), and 30 µl (2.9 U) of α-mannosidase (product of Sigma-Aldrich Corp., from Jack Beans) was added to the solution. The resulting solution was allowed to stand at 37°C for 63 hours, and thereafter filtered with a membrane filter. The filtrate was purified by HPLC [ODS column, 2.0 (diam.) × 25 cm; eluent: 50 mM aqueous solution of ammonium acetate: acetonitrile = 80:20; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.6 µg of the desired Compound (C4).
¹H NMR (400MHz, D₂O, 30 °C, HOD=4.81)
δ 8.00(d, 2H, J=7.2, Fmoc), 7.79(d, 2H, J=7.2, Fmoc), 7.59(dd, 2H, J=7.2, Fmoc), 7.52(dd, 2H, J=7.2, Fmoc), 5.21(s, 1H, Man4-H1), 5.09(d, 1H, J=10.0, GlcNAc1-H1), 4.60-4.75(m,), 4.40-4.50(m, 2H), 4.32(bd, 1H, J=2.3), 4.28(bs, 1H), 4.22(bdd, 1H, Ja=9.7, Jb=2.8, Man4-H2), 2.80-2.95(m, 2H, Asn-βCH, NeuAc7-H3eq), 2.60-2.75(m, 1H, Asn-βCH), 2.14, 2.14, 2.12(eachs, Acx3), 1.98(s, 3H, Ac), 1.88(dd, 1H, Ja=12.1, Jb=12.0, NeuAc7-H3ax).

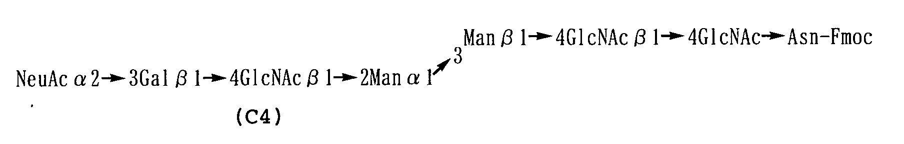

Compound (C4) obtained was treated in the same manner as in Reference Example 7 to remove Fmoc group to prepare an asparagine-linked oligosaccharide. The obtained asparagine-linked oligosaccharide was biotinated in the same manner as in Example 1.

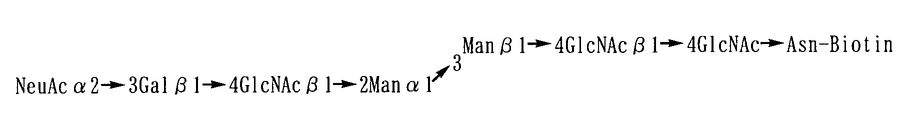

### Example 30

Compound (C1-3) (1 mg, 0.44 µmole) obtained in Example 26 and 1 mg of bovine serum albumin were dissolved in 50 µl of HEPES buffer solution (50 mM, pH 5.0), and β-galactosidase (product of Seikagaku Corp., from Jack Beans, 5 µl, 100 mU) was added to the solution. The resulting solution was allowed to stand at 37°C for 15 hours, and thereafter filtered with a membrane filter. The filtrate was purified by HPLC [ODS column, 2.0 (diam.) × 25 cm; eluent: 50 mM aqueous solution of ammonium acetate : acetonitrile = 82:18; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.3 µg of the desired Compound (C5).
¹H NMR (400MHz, D₂O, 30°C, HOD=4.81)
δ 8.01(d, 2H, J=7.2, Fmoc), 7.81(d, 2H, J=7.2, Fmoc), 7.60(dd, 2H, J=7.2, Fmoc), 7.53(dd, 2H, J=7.2, Fmoc), 5.21(s, 1H, Man4-H1), 5.09(d, 1H, J=9.6, GlcNAc1-H1), 5.02(s, 1H, Man4'-H-1), 4.55-4.70(m), 4.44(1H, Fmoc), 4.30-4.38(bm, 2H), 4.28(bd, 1H, Man4-H2), 4.17-4.25(m, 2H), 2.78-2.95(m, 2H, Asn-βCH, NeuAc7-H3eq), 2.55-2.70(m, 1H, Asn-βCH), 2.16, 2.15, 2.14, 2.12(eachs, 12H, Acx4), 1.98(s, 3H, Ac), 1.89(dd, 1H, Ja=12.2, Jb=12.0, NeuAc7-H3ax).

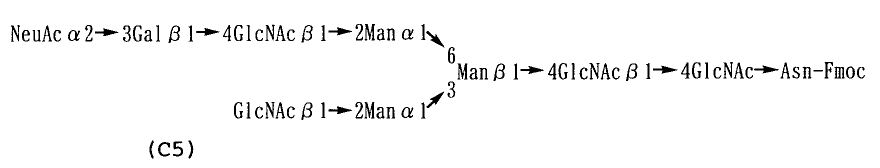

Compound (C5) obtained was treated in the same manner as in Reference Example 7 to remove Fmoc group to prepare an asparagine-linked oligosaccharide. The obtained asparagine-linked oligosaccharide was biotinated in the same manner as in Example 1.

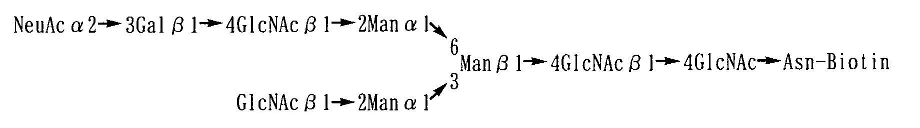

### Example 31

Compound (C5) (1.0 mg, 0.48 µmole) obtained in Example 30 and 1 mg of bovine serum albumin were dissolved in 50 µl of HEPES buffer solution (50 mM, pH 5.0), and 4 µl (250 mU) of N-acetyl-β-glucosamidase (product of Sigma-Aldrich Corp., from Jack Beans) was added to the solution. The resulting solution was allowed to stand at 37°C for 22 hours, and thereafter filtered with a membrane filter. The filtrate was purified by HPLC [ODS column, 2.0 (diam.) × 25 cm; eluent: 50 mM aqueous solution of ammonium acetate : acetonitrile = 82:18; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.6 µg of the desired Compound (C6).
¹H NMR (400MHz, D₂O, 30°C, HOD=4.81)
δ 8.01(d, 2H, J=7.6, Fmoc), 7.80(d, 2H, J=7.6, Fmoc), 7.60(dd, 2H, J=7.6, Fmoc), 7.53(dd, 2H, J=7.6, Fmoc), 5.19(s, 1H, Man4-H1), 5.09(d, 1H, J=9.2, GlcNAc1-H1), 5.02(s, 1H, Man4'-H-1), 4.85(s, 1H), 4.58-4.75(m, 5H), 4.38-4.48(m, 2H, Fmoc), 4.40(bd, J=2.4, 1H), 4.18-4.25(m, 2H), 4.15(m, 1H), 2.80-2.95(m, 2H, Asn-βCH, NeuAc7-H3eq), 2.65-2.75(m, 1H, Asn-βCH), 2.16, 2.13, 2.12(eachs, 9H, Acx3), 1.98(s, 3H, Ac), 1.89(dd, 1H, Ja=12.2, Jb=12.0, NeuAc7-H3ax).

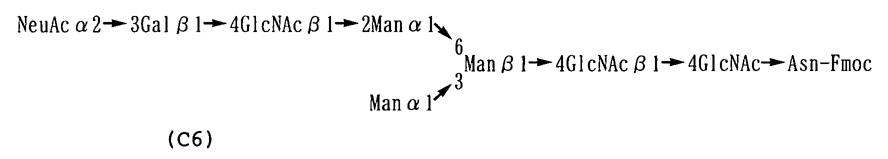

Compound (C6) obtained was treated in the same manner as in Reference Example 7 to remove Fmoc group to prepare an asparagine-linked oligosaccharide. The obtained asparagine-linked oligosaccharide was biotinated in the same manner as in Example 1.

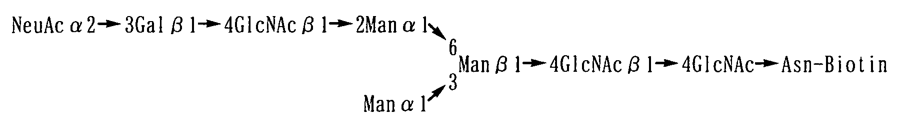

### Example 32

Compound (C6) (1.0 mg, 0.53 µmole) obtained in Example 31 and 1 mg of bovine serum albumin were dissolved in 50 µl of HEPES buffer solution (50 mM, pH 5.0), and 10 µl (0.9 U) of α-mannosidase (product of Sigma-Aldrich Corp., from Jack Beans) was added to the solution. The resulting solution was allowed to stand at 37°C for 20 hours, and thereafter filtered with a membrane filter. The filtrate was purified by HPLC [ODS column, 2.0 (diam.) × 25 cm; eluent: 50 mM aqueous solution of ammonium acetate : acetonitrile = 80:20; flow rate 7.5 ml/min], followed by concentration of the solvent and freeze-drying. The residue was dissolved in 200 µl of water and desalted by ODS-column chromatography (Cosmosil 75C₁₈-opn, washing with water first, subsequent elution with 25% aqueous solution of acetonitrile), giving 0.5 µg of the desired Compound (C7).
¹H NMR (400MHz, D₂O, 30°C, HOD=4.81)
δ 8.01(d, 2H, J=7.6, Fmoc), 7.81(d, 2H, J=7.6, Fmoc), 7.60(dd, 2H, J=7.2, Fmoc), 7.53(dd, 2H, J=7.6, Fmoc), 5.09(d, 1H, J=9.2, GlcNAc1-H1), 5.01(s, 1H, Man4'-H-1), 4.84(s, 1H), 4.55-4.70(m, 5H), 4.44 (t, 1H, J=6.0, Fmoc), 4.30-4.38(bs, 1H), 4.15-4.25(m, 2H), 4.17(s, 1H), 2.80-2.95(m, 2H, Asn-βCH, NeuAc7-H3eq), 2.55-2.70(m, 1H, Asn-βCH), 2.16, 2.13, 2.12(eachs, Acx3), 1.98(s, 3H, Ac) 1.89(dd, 1H, Ja=12.2, Jb=12.3, NeuAc7-H3ax).

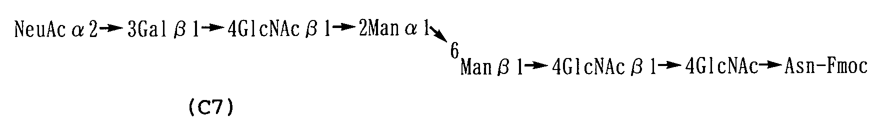

Compound (C7) obtained was treated in the same manner as in Reference Example 7 to remove Fmoc group to prepare an asparagine-linked oligosaccharide. The obtained asparagine-linked oligosaccharide was biotinated in the same manner as in Example 1.

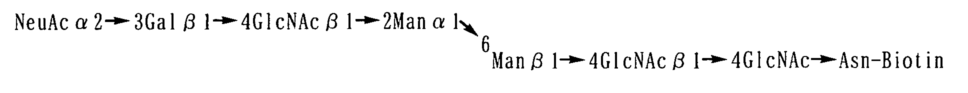

### Example 33

Preparation of asparagine-linked disialo (α 2,6) (α 2,3)oligosaccharide wherein the amino group of the asparagine was protected with Fmoc group

Using sialic acid transferase, CMP-sialic acid was transferred to the asparagine-linked asialooligosaccharide (Compound 2) which was obtained in Reference Example 45 and wherein the amino group of the asparagine was protected with Fmoc group.

The sialic acid transferase used was α2,3-transferase which was commercially available and derived from a rat recombinant.

Compound 2 (1.7 mg, 0.75 µmole) obtained in Reference Example 45 was dissolved in 50 mM of cacodylic acid buffer (5.0 in pH, 85 µl), and bovine serum albumin (BSA, 1 mg) was then added to the solution. To the mixture were added CMP-sialic acid (4.8 mg, 7.5 µmoles) and alkanline phosphatase (1 µl, 75 units), and the resulting mixture was stirred uniformly. Finally, α2,3-sialyltransferase (product of CALBIOCHEM, 75 µl, 34 mU) was added to the mixture, and the resulting mixture was allowed to stand at 37°C for 3.5 hours. The reaction was terminated upon the disappearance of the starting material while monitoring the reaction by HPLC, and the reaction mixture was filtered with a membrane filter. The filtrate was concentrated to reduce the quantity thereof and thereafter purified by HPLC fractionating column (YMC-Pack R&D ODS, D-ODS-5-A, 20 × 250 mm, AN/25 mM AcONH₄ buffer = 18/82, 7.5 ml/min., wave length: 274 nm). Compound (C7A) was obtained as an eluate 25 minutes later. The fraction was collected, desalted and freeze-dried, giving 1.3 mg (67.8%) of Compound (C7A). The NMR data is given below.
¹H NMR (400MHz, D₂O, 30°C, HOD=4.81)
δ 8.00(d, 2H, J=7.2, Fmoc), 7.79(d, 2H, J=7.2, Fmoc), 7.60(dd, 2H, J=7.2, Fmoc), 7.52(dd, 2H, J=7.2, Fmoc), 5.21(s, 1H, Man4-H1), 5.09(d, 1H, J=8.8, GlcNAc1-H1), 5.03(s, 1H, Man4'-H-1), 4.86(s, 1H), 4.58-4.72(m, 5H), 4.54(d, 1H, J=8.0), 4.38-4.48(m, 2H), 4.34(bs, 1H), 4.28(bs, 1H), 4.15-4.25(m, 2H), 2.80-2.86(dd, 1H, Ja=4.4, Jb=12.4, NeuAc7-H3eq), 2.73-2.83(m, dd, 3H, Ja=4.4, Jb=12.4, Asn-β CH, NeuAc7-H3eq), 2.60-2.72(m, 1H, Asn-βCH), 2.16, 2.15, 2.14, 2.12(each s, Ac x5), 1.98(s, 3H, Ac), 1.89(dd, 1H, Ja=12.4, Jb=12.0, NeuAc7-H3ax), 1.81(dd, 1H, Ja=12.4, Jb=12.0, NeuAc7-H3ax).

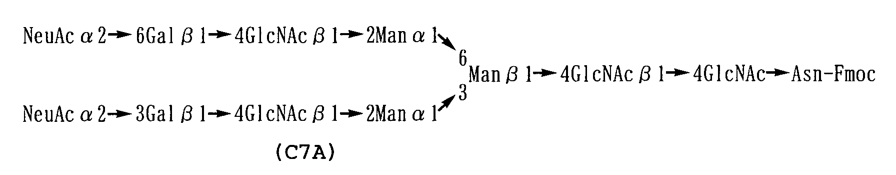

Compound (C7A) obtained was treated in the same manner as in Reference Example 7 to remove Fmoc group to prepare an asparagine-linked oligosaccharide. The obtained asparagine-linked oligosaccharide was biotinated in the same manner as in Example 1.

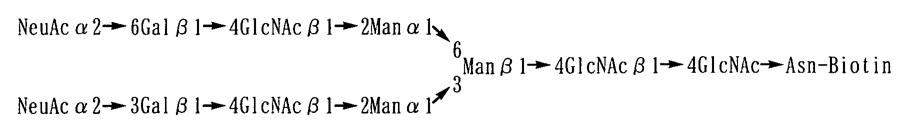

### Example 34

Preparation of asparagine-linked disialo (α2,3) (α 2,6)oligosaccharide wherein the amino group of the asparagine was protected with Fmoc group

Using sialic acid transferase, CMP-sialic acid was transferred to the asparagine-linked monosialooligosaccharide (Compound 6) which was obtained in Reference Example 45 and wherein the amino group of the asparagine was protected with Fmoc group.

The sialic acid transferase used was α2,3-transferase which was commercially available and derived from a rat recombinant.

Compound 6 (1.2 mg, 0.53 µmole) obtained in Reference Example 45 was dissolved in 50 mM of cacodylic acid buffer (5.0 in pH, 60 µl), and bovine serum albumin (BSA, 1 mg) was then added to the solution. To the mixture were added CMP-sialic acid (3.4 mg, 5.3 µmoles) and alkanline phosphatase (1 µl, 75 units), and the resulting mixture was stirred uniformly. Finally, α2,3-sialyltransferase (product of CALBIOCHEM, 52.9 µl, 24 mU) was added to the mixture, and the resulting mixture was allowed to stand at 37°C for 3 hours. The reaction was terminated upon the disappearance of the starting material while monitoring the reaction by HPLC, and the reaction mixture was filtered with a membrane filter. The filtrate was concentrated to reduce the quantity thereof and thereafter purified by HPLC fractionating column (YMC-Pack R&D ODS, D-ODS-5-A, 20 × 250 mm, AN/25 mM AcONH₄ buffer = 18/82, 7.5 ml/min., wave length: 274 nm). Compound (C7B) was obtained as an eluate 23 minutes later. The fraction was collected, desalted and freeze-dried, giving 1.1 mg (81.2%) of Compound (C7B). The NMR data is given below.
¹H NMR(400MHz, D₂O, 30°C, HOD=4.81)
δ 8.00(d, 2H, J=7.6, Fmoc), 7.79(d, 2H, J=7.6, Fmoc), 7.59(dd, 2H, J=7.6, Fmoc), 7.51(dd, 2H, J=7.6, Fmoc), 5.21(s, 1H, Man4-H1), 5.08(d, 1H, J=10.0, G1cNAc1-H1), 5.00(s, 1H, Man4'-H-1), 4.84(s, 1H), 4.60-4.72(m, 5H), 4.52(d, 1H, J=7.6), 4.35-4.45(m, 2H), 4.33(bs, 1H), 4.27(bs, 1H), 4.15-4.25(m, 2H), 2.80-2.86(dd, 1H, Ja=4.8, Jb=12.4, NeuAc7-H3eq), 2.73-2.83(bs, dd, 3H, Ja=4.8, Jb=12.4, Asn-βCH, NeuAc7-H3eq), 2.60-2.72(m, 1H, Asn-βCH), 2.15, 2.12, 2.10(each s, Ac x5), 1.97(s, 3H, Ac), 1.88(dd, 1H, Ja=12.4, Jb=12.4, NeuAc7-H3ax), 1.80(dd, 1H, Ja=12.4, Jb=12.4, NeuAc7-H3ax).

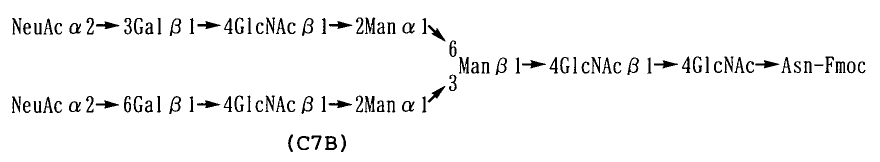

Compound (C7B) obtained was treated in the same manner as in Reference Example 7 to remove Fmoc group to prepare an asparagine-linked oligosaccharide. The obtained asparagine-linked oligosaccharide was biotinated in the same manner as in Example 1.

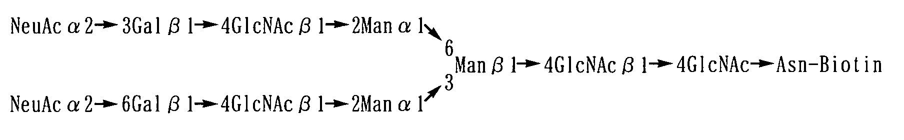

### Examples 35 to 52

Each of the asparagine-linked Fmoc-oligosaccharides prepared in Reference Examples 2, 8 to 10, 12, 14, 17, 45 and Examples 26 to 32 was dissolved, in an amount of 2 nmoles, in about 10 ml of Tris hydrochloric acid buffer. To the solution were added 200 nmoles of GDP-fucose and 0.5 mU of Fucosyltransferase V (human recombinant), and the mixture was allowed to stand at 37°C for about 2 hours for reaction. The reaction mixture was diluted with 20 ml of ultrapure water and thereafter subjected to capillary electrophoresis (fused silica capillary, 50 mm i.d., 60 cm, buffer: Tris-borate, 8.3 in pH, 100 mM heptane sulfonate, applied voltage 27 kV, temp. 25°C, 214 mm) for separation to obtain an asparagine-linked oligosaccharide derivative containing fucose.

The obtained asparagine-linked oligosaccharide derivative was treated in the same manner as in Reference Example 7 to remove Fmoc group to prepare an asparagine-linked oligosaccharide. The obtained asparagine-linked oligosaccharide was biotinated in the same manner as in Example 1. The obtained biotinated asparagine-linked oligosaccharides are shown below.

### Example 35

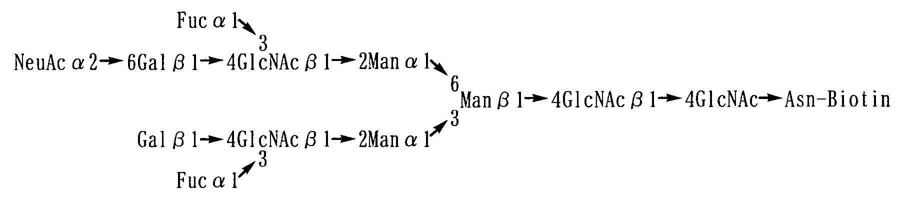

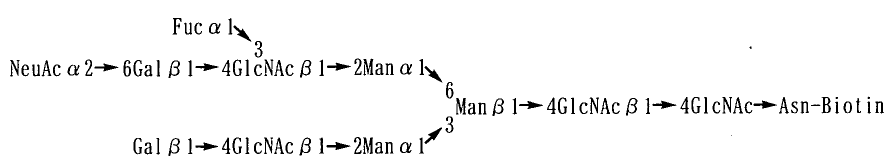

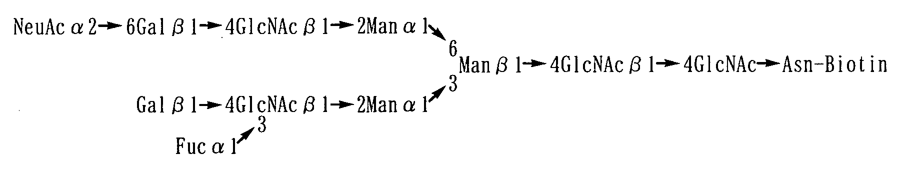

### Example 36

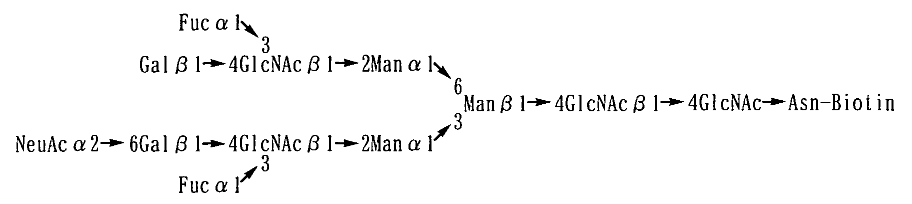

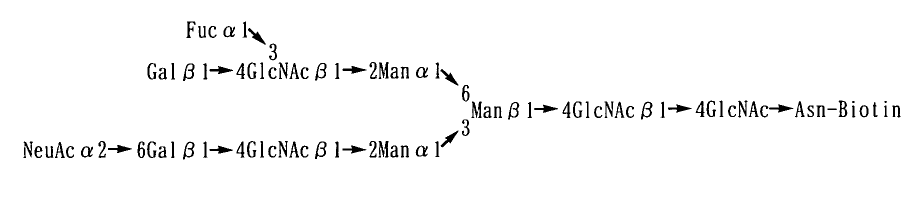

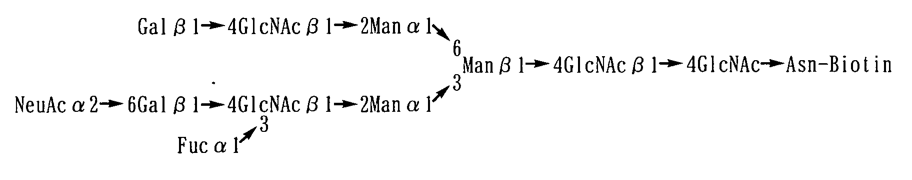

### Example 37

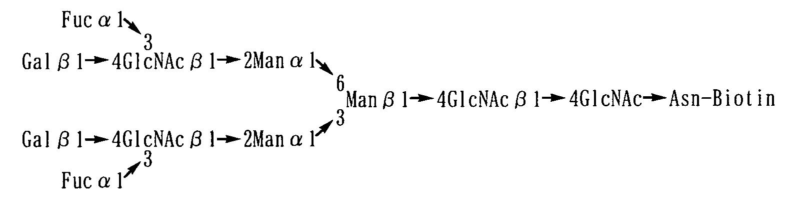

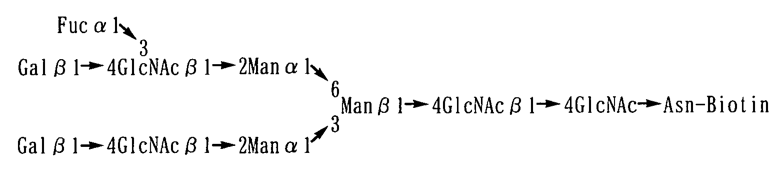

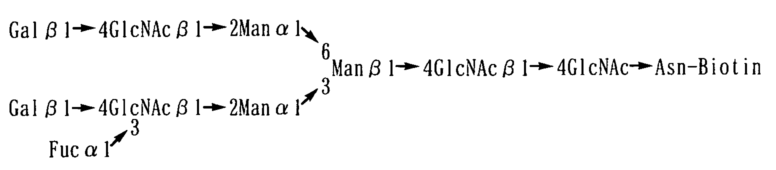

### Example 38

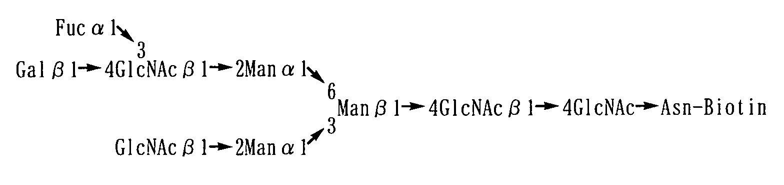

### Example 39

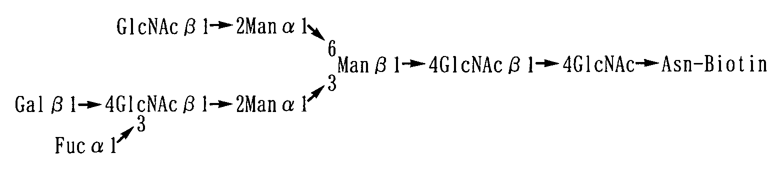

### Example 40

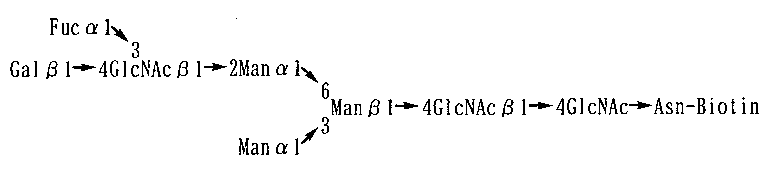

### Example 41

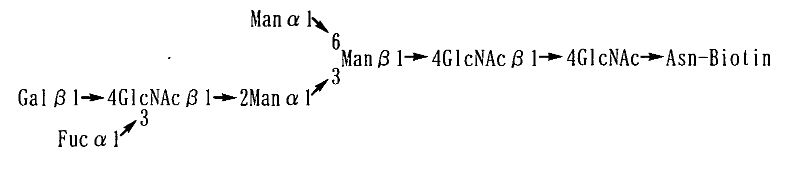

### Example 42

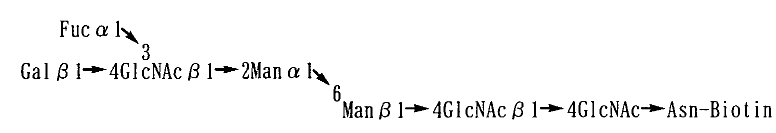

### Example 43

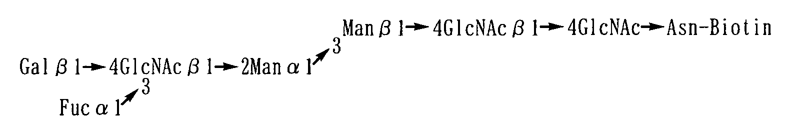

### Example 44

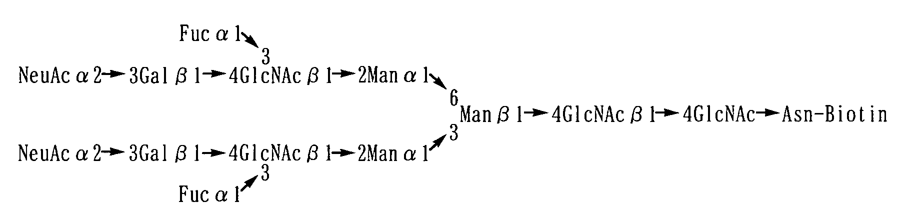

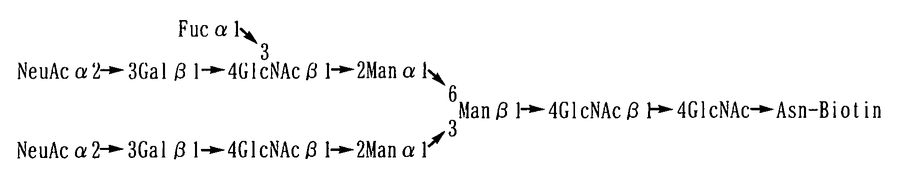

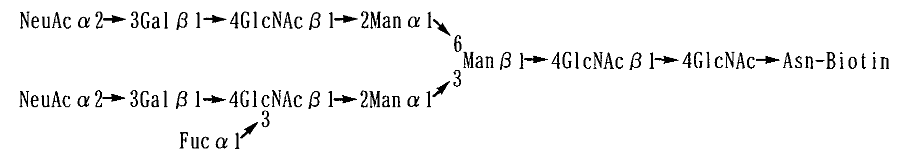

### Example 45

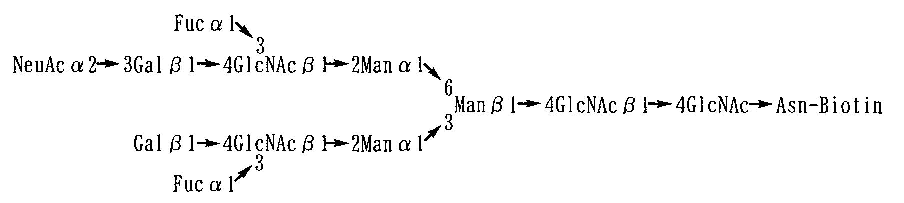

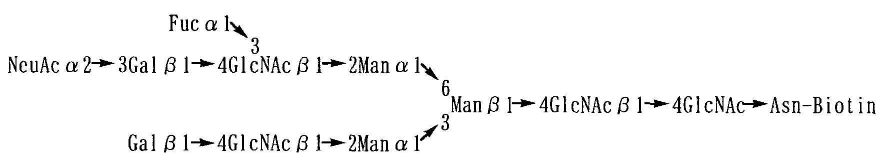

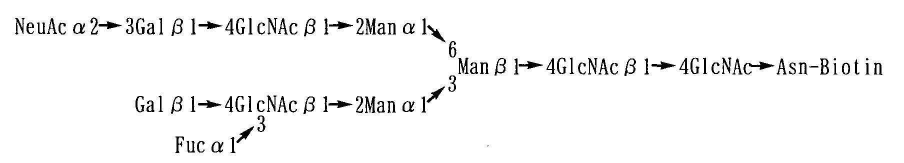

### Example 46

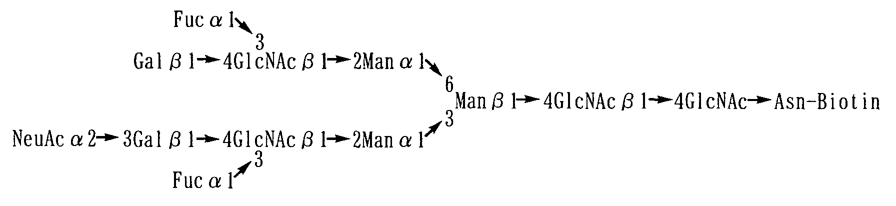

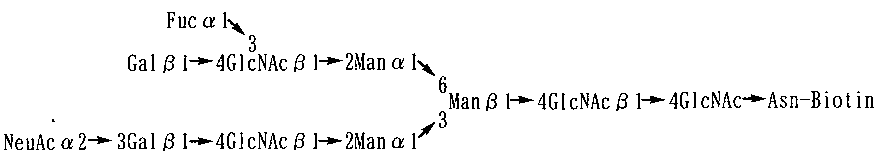

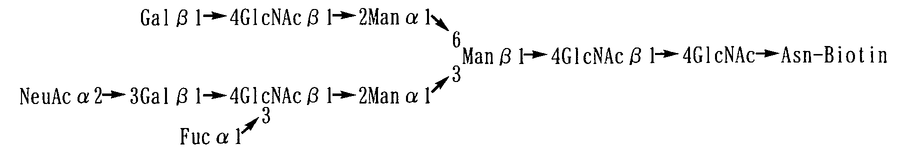

### Example 47

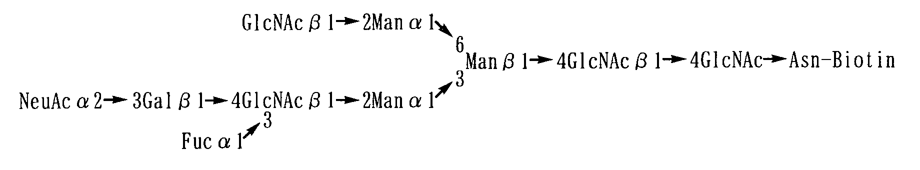

### Example 48

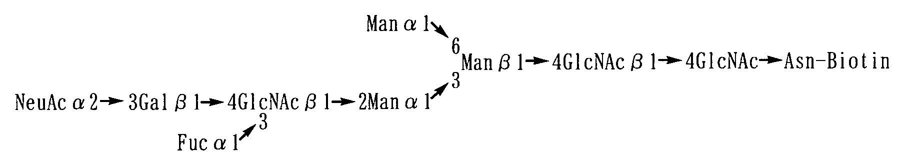

### Example 49

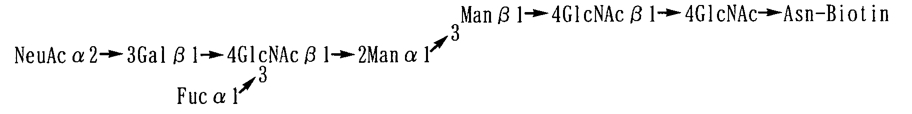

### Example 50

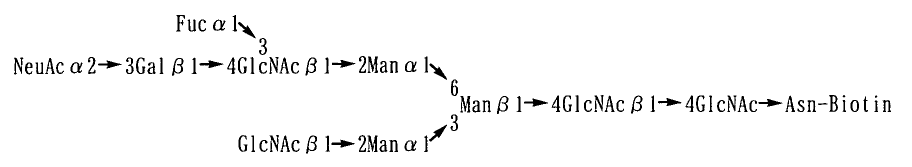

### Example 51

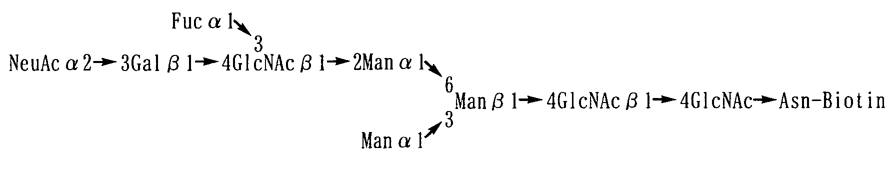

### Example 52

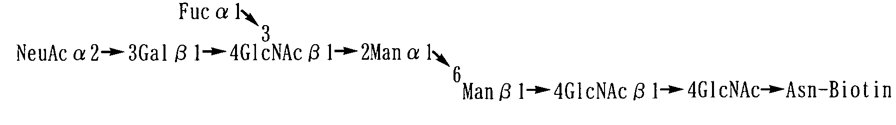

### Example 53

Asparagine-linked disialooligosaccharide (Compound 24)(undecasaccharide, 1.4 mg, 0.60 mmol) was dissolved in 70 ml of purified water, and 70 mL of acetone and NaHCO₃(0.7 mg, 9 mmoles) were added thereto and stirred at room temperature. Fluorescein isothiocyanate (FITC, 0.95 mg, 2.4 mmoles, product of Aldrich Corp.) was added to the solution and the mixture was stirred for about 2 hours. After 2 hours, the completion of the reaction was confirmed by TLC, acetone was removed at a reduced pressure and the remaining aqueous solution was purified by gel filtration column chromatography (Sephadex G-25, H₂O) to collect desired fraction. The fraction was concentrated and purified by HPLC (YMC-pack ODS=AM, SH-343-5AM, 20 × 250 mm, AN/25 mM AcONH₄ buffer=10/90, 7.5 ml/min., wave length: 274 nm). The fraction obtained was desalted by gel filtration column chromatography (Sephadex G-25, H₂O). The desired fraction was collected, concentrated and freeze-dried to obtain a desired FITC-bonded asparagine-linked disialooligosaccharide derivative (1.2 mg, 73.5% yield). 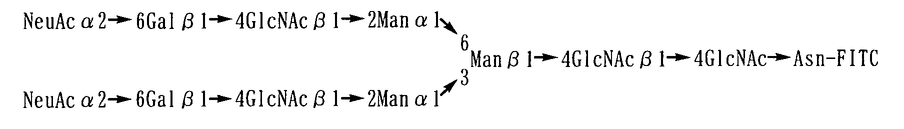 ¹H NMR (400MHz, D₂O, 30°C, HOD=4.81)
δ7.86(d, 1H, J=2.0, FITC), 7.75(dd, 1H, Ja=8.0, Jb=2.0, FITC), 7.46(d, 1H, J=8.0, FITC), 7.41(d, 1H, J=9.6, FITC), 6.71-6.83(bm, 4H, FITC), 5.22(s, 1H, Man4-H1), 5.10-5.20(bm, 2H, GlcNAc1-H1), 5.03(s, 1H, Man4'-H-1), 4.70-4.80(m, 3H), 4.53(d, 2H, J=8.0), 4.33(s, 1H, Man3-H-2), 4.27(bs, 1H, Man4-H-2), 4.18(s, 1H, Man4-H-2), 2.90-3.00(m, 1H, Asn-βCH), 2.85-2.95(m, 1H, Asn-βCH), 2.75(dd, 2H, Ja=12.0, Jb=2.8, NeuAc7-H3ex), 2.15, 2.14, 2.12, 2.09(eachs, 18H, Acx6), 1.80(dd, 2H, Ja=12.0, Jb=12.0, NeuAc7-H3ax).

### Example 54

FITC-bonded asparagine-linked oligosaccharide derivative was prepared in the same manner as in Example 53 using Compound 76 in place of Compound 24.

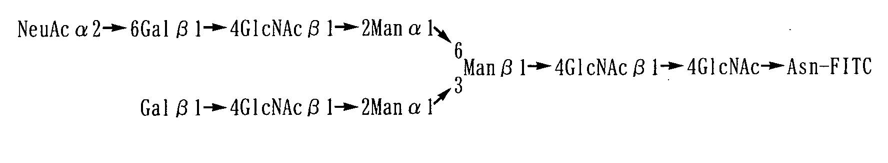

### Example 55

FITC-bonded asparagine-linked oligosaccharide derivative was prepared in the same manner as in Example 53 using Compound 77 in place of Compound 24.

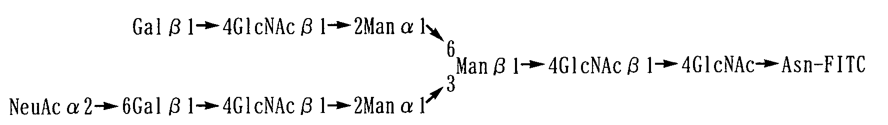

### Example 56

FITC-bonded asparagine-linked oligosaccharide derivative was prepared in the same manner as in Example 53 using Compound 33 in place of Compound 24.

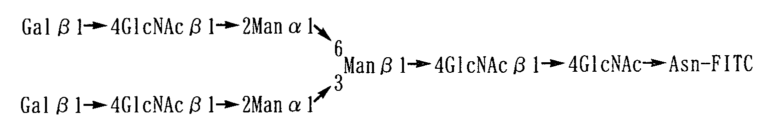

The NMR data of the obtained FITC-bonded asparagine-linked oligosaccharide is given below.
¹H-NMR (400MHz, D₂O, 30°C, HOD=4.81)
δ 7.88(d, 1H, J=2.0, FITC), 7.76(dd, 1H, Ja=8.0, Jb=2.0, FITC), 7.46(d, 1H, J=8.0, FITC), 7.45(d, 1H, J=8.8, FITC), 6.85-6.93(m, 4H, FITC), 5.21(s, 1H, Man4-H1), 5.05-5.20(bd, 2H, GlcNAc1-H1), 5.01(s, 1H, Man4'-H-1), 4.67(d, 3H, J=7.6), 4.55(d, 2H), 4.34(s, 1H, Man3-H-2), 4.28(bs, 1H, Man4-H-2), 4.20(s, 1H, Man4-H-2), 3.00-3.10(bm, 1H, Asn-βCH), 2.90-3.00(m, 1H, Asn-βCH), 2.75(dd, 2H, Ja=12.0, Jb=2.8, NeuAc7-H3ex), 2.14, 2.13, 2.12, 2.08(eachs, 12H, Acx4).

### Example 57

FITC-bonded asparagine-linked oligosaccharide derivative was prepared in the same manner as in Example 53 using Compound 26 in place of Compound 24.

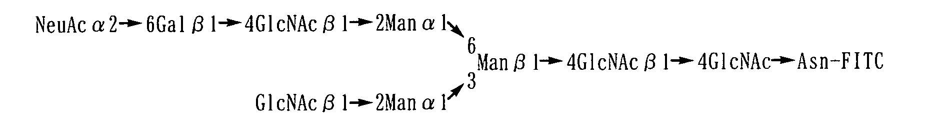

### Example 58

FITC-bonded asparagine-linked oligosaccharide derivative was prepared in the same manner as in Example 53 using Compound 27 in place of Compound 24.

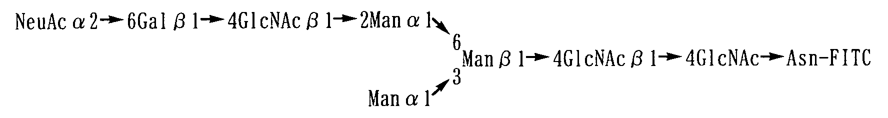

### Example 59

FITC-bonded asparagine-linked oligosaccharide derivative was prepared in the same manner as in Example 53 using Compound 28 in place of Compound 24.

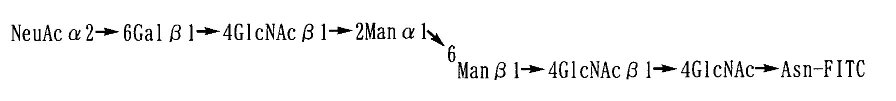

### Example 60

FITC-bonded asparagine-linked oligosaccharide derivative was prepared in the same manner as in Example 53 using Compound 30 in place of Compound 24.

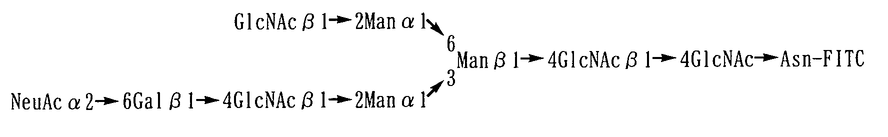

### Example 61

FITC-bonded asparagine-linked oligosaccharide derivative was prepared in the same manner as in Example 53 using Compound 31 in place of Compound 24.

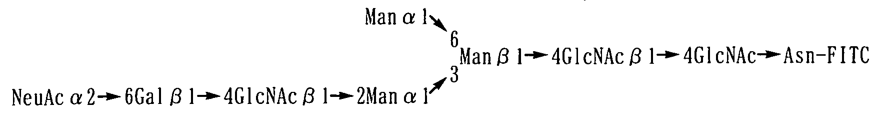

### Example 62

FITC-bonded asparagine-linked oligosaccharide derivative was prepared in the same manner as in Example 53 using Compound 32 in place of Compound 24.

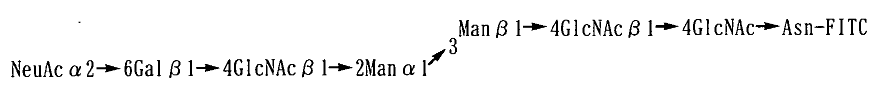

### Example 63

FITC-bonded asparagine-linked oligosaccharide derivative was prepared in the same manner as in Example 53 using Compound 37 in place of Compound 24.

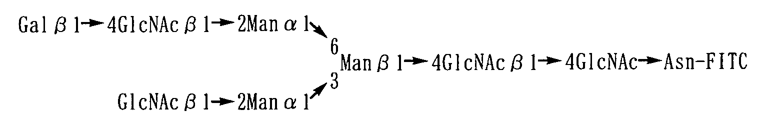

### Example 64

FITC-bonded asparagine-linked oligosaccharide derivative was prepared in the same manner as in Example 53 using Compound 42 in place of Compound 24.

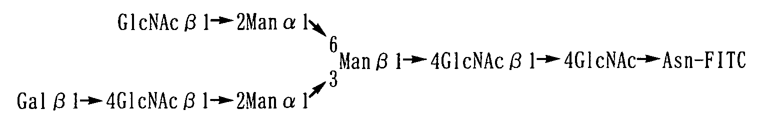

### Example 65

FITC-bonded asparagine-linked oligosaccharide derivative was prepared in the same manner as in Example 53 using Compound 38 in place of Compound 24.

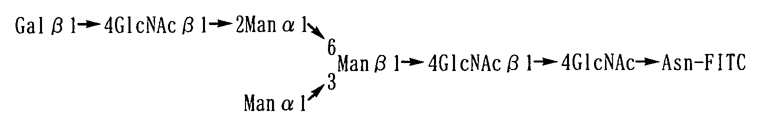

### Example 66

FITC-bonded asparagine-linked oligosaccharide derivative was prepared in the same manner as in Example 53 using Compound 72 in place of Compound 24.

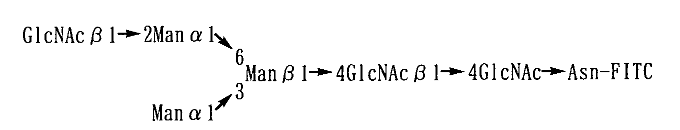

### Example 67

FITC-bonded asparagine-linked oligosaccharide derivative was prepared in the same manner as in Example 53 using Compound 43 in place of Compound 24.

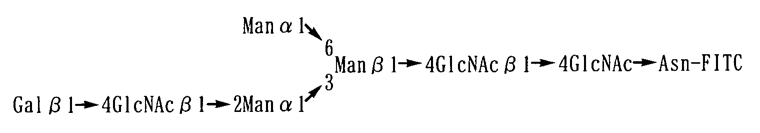

### Example 68

FITC-bonded asparagine-linked oligosaccharide derivative was prepared in the same manner as in Example 53 using Compound 73 in place of Compound 24.

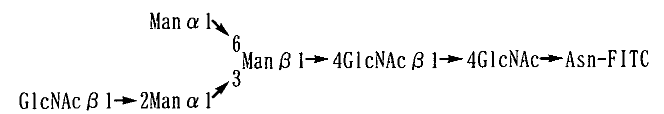

### Example 69

FITC-bonded asparagine-linked oligosaccharide derivative was prepared in the same manner as in Example 53 using Compound 39 in place of Compound 24.

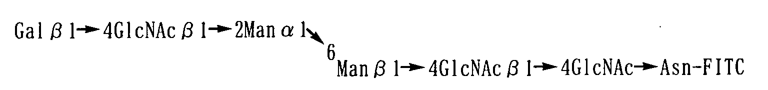

### Example 70

FITC-bonded asparagine-linked oligosaccharide derivative was prepared in the same manner as in Example 53 using Compound 40 in place of Compound 24.

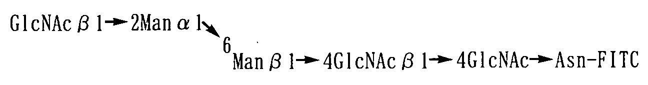

### Example 71

FITC-bonded asparagine-linked oligosaccharide derivative was prepared in the same manner as in Example 53 using Compound 41 in place of Compound 24.

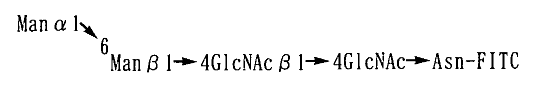

### Example 72

FITC-bonded asparagine-linked oligosaccharide derivative was prepared in the same manner as in Example 53 using Compound 44 in place of Compound 24.

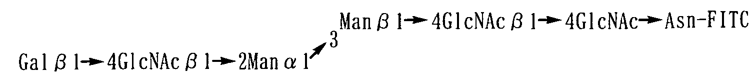

### Example 73

FITC-bonded asparagine-linked oligosaccharide derivative was prepared in the same manner as in Example 53 using Compound 45 in place of Compound 24.

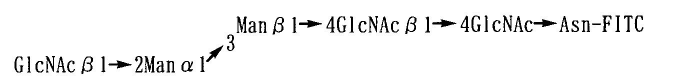

### Example 74

FITC-bonded asparagine-linked oligosaccharide derivative was prepared in the same manner as in Example 53 using Compound 46 in place of Compound 24.

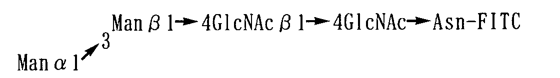

### Example 75

FITC-bonded asparagine-linked oligosaccharide derivative was prepared in the same manner as in Example 53 using Compound 34 in place of Compound 24.

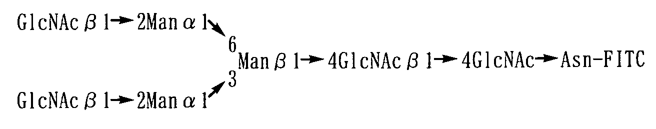

### Example 76

FITC-bonded asparagine-linked oligosaccharide derivative was prepared in the same manner as in Example 53 using Compound 35 in place of Compound 24.

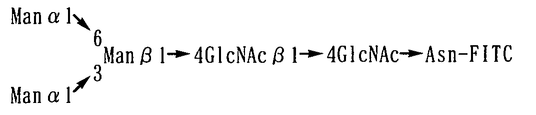

### Example 77

FITC-bonded asparagine-linked oligosaccharide derivative was prepared in the same manner as in Example 53 using Compound 36 in place of Compound 24.

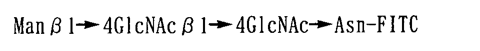

### Example 78

Each of Compounds (C-1)-(C-3) obtained in Example 26 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

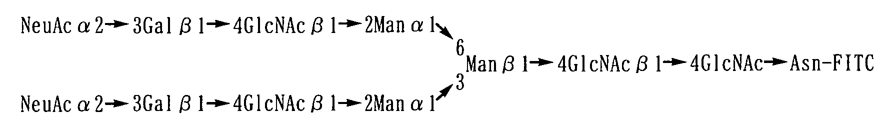

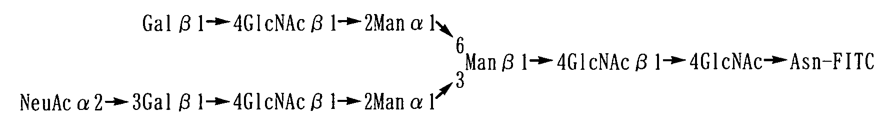

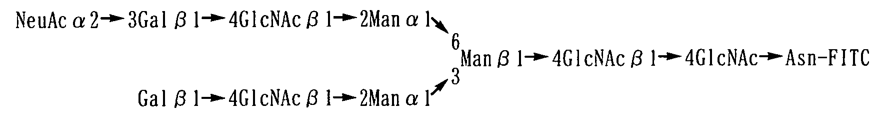

### Example 79

Compounds (C2) obtained in Example 27 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

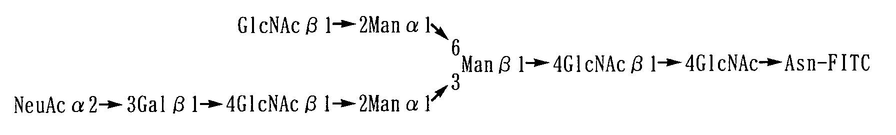

### Example 80

Compounds (C3) obtained in Example 28 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

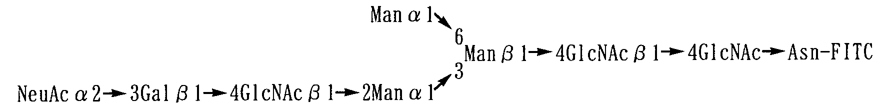

### Example 81

Compounds (C4) obtained in Example 29 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

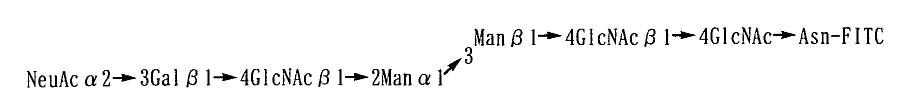

### Example 82

Compounds (C5) obtained in Example 30 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

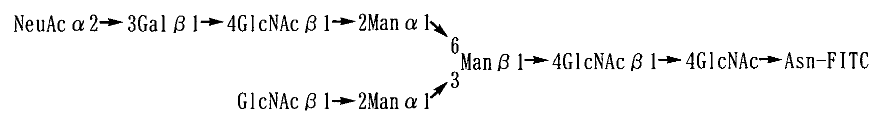

### Example 83

Compounds (C6) obtained in Example 31 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

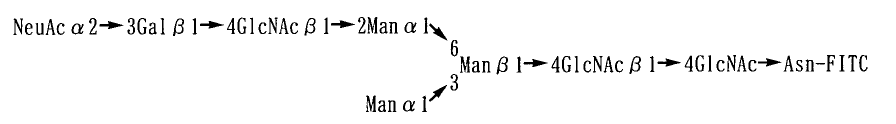

### Example 84

Compounds (C7) obtained in Example 32 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

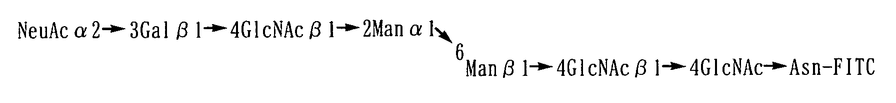

### Example 85

Compounds (C7A) obtained in Example 33 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

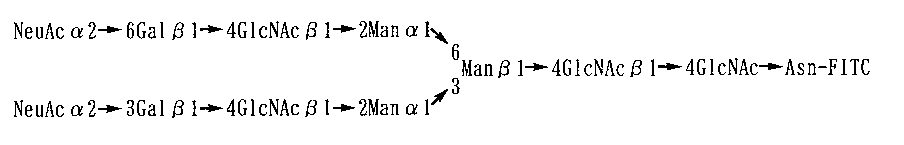

### Example 86

Compounds (C7B) obtained in Example 34 was treated in the same manner as in Reference Example 7 to remove Fmoc group to prepare an asparagine-linked oligosaccharide. The obtained asparagine-linked oligosaccharide was fluorescein isothiocyanated in the same manner as in Example 53.

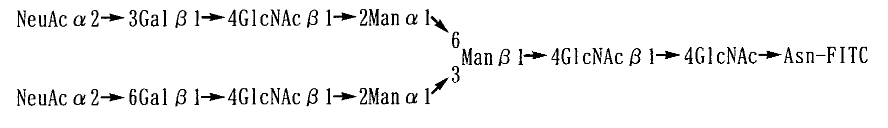

### Examples 87 to 104

Each of the asparagine-linked oligosaccharide derivatives containing fucose obtained in Examples 35 to 52 was fluorescein isothiocyanated in the same manner as in Example 53.

### Example 87

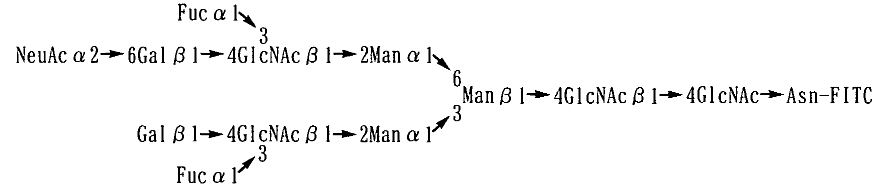

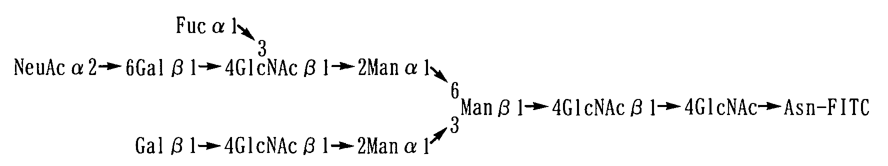

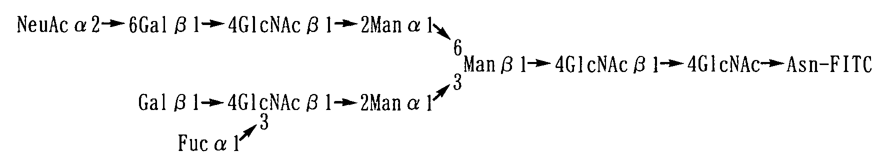

### Example 88

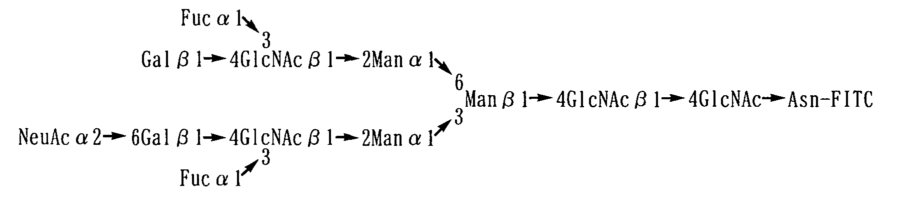

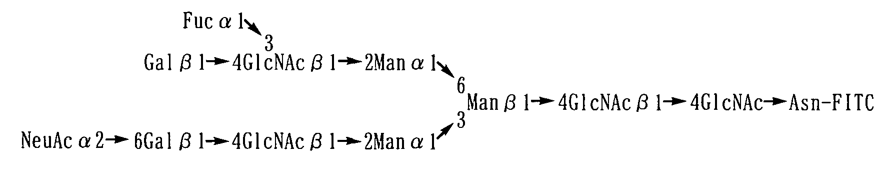

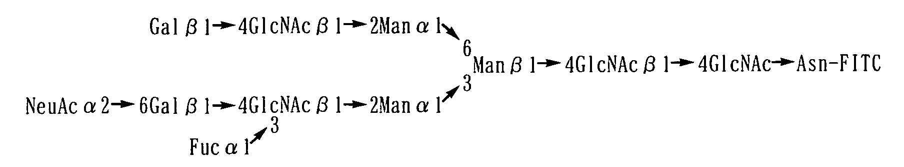

### Example 89

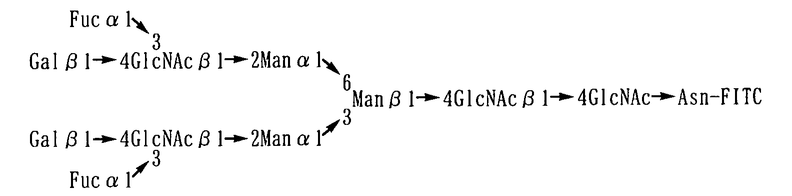

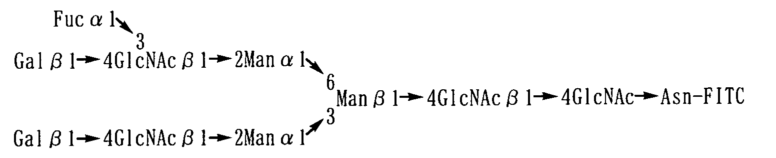

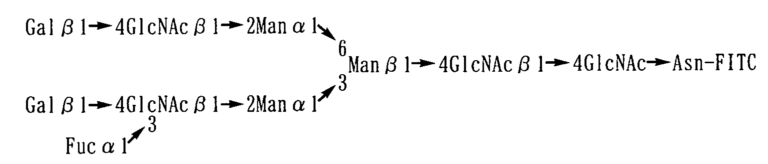

### Example 90

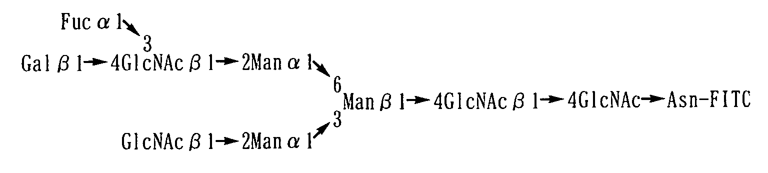

### Example 91

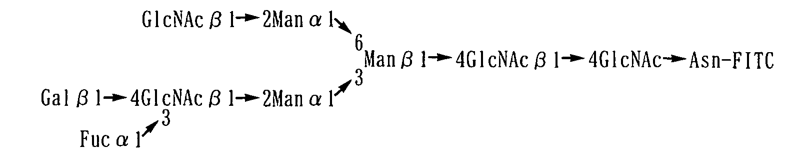

### Example 92

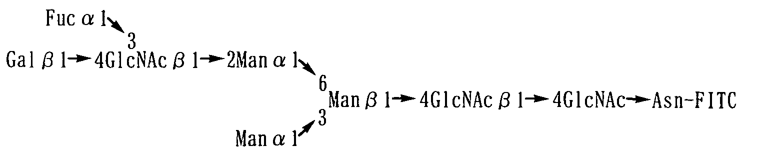

### Example 93

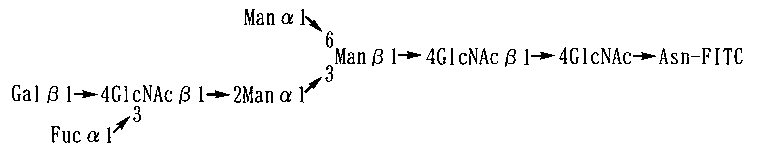

### Example 94

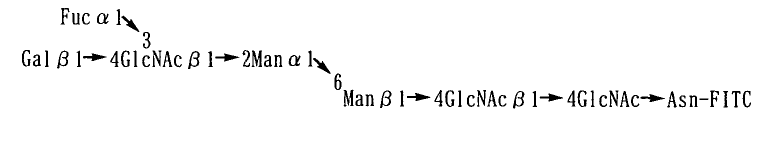

### Example 95

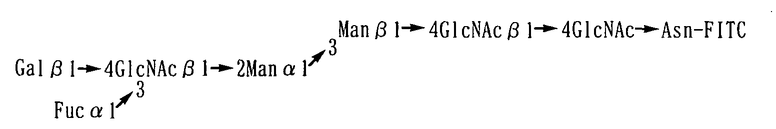

### Example 96

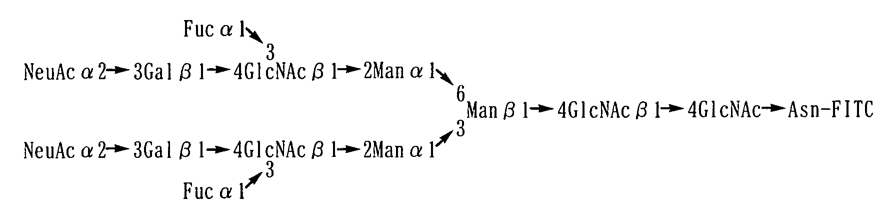

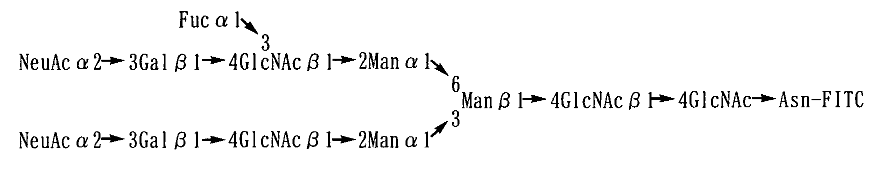

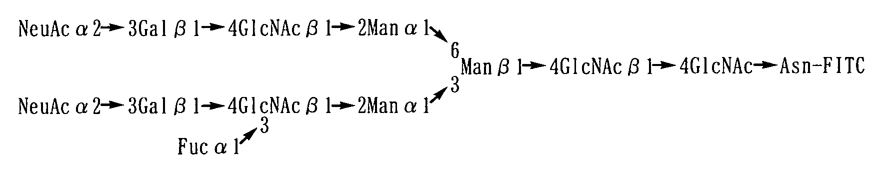

### Example 97

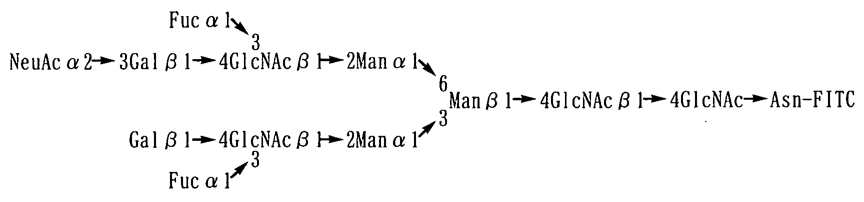

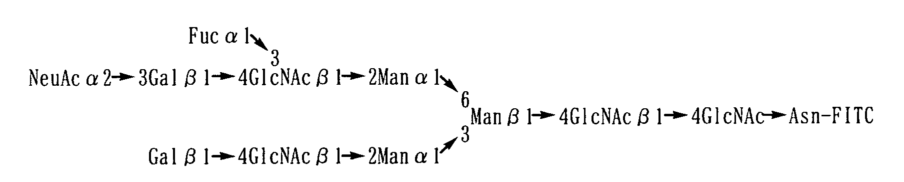

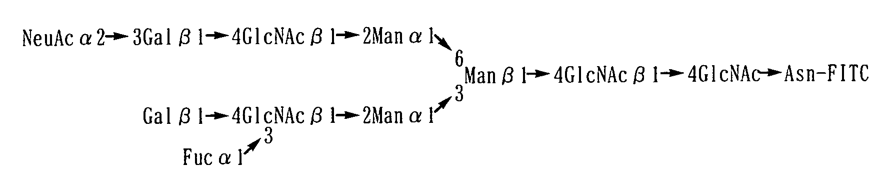

### Example 98

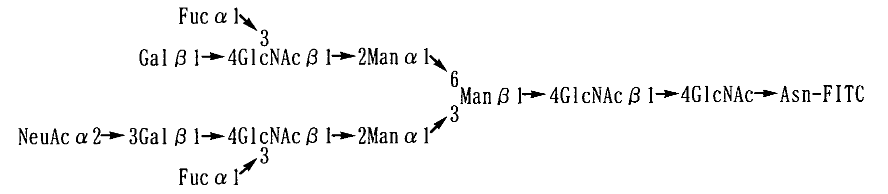

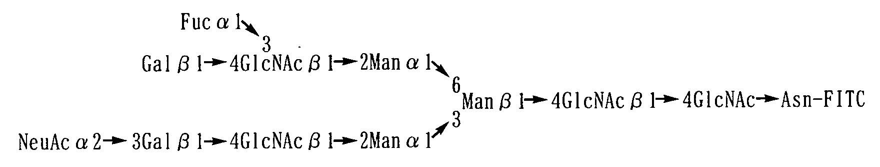

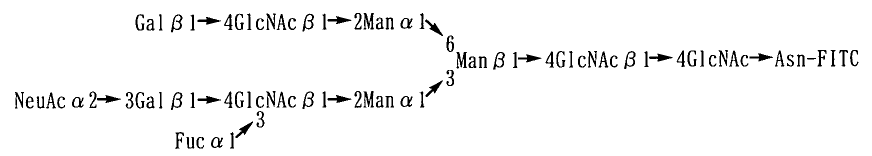

### Example 99

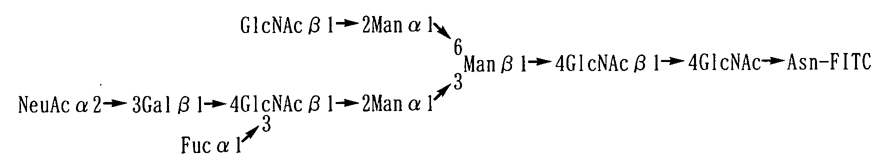

### Example 100

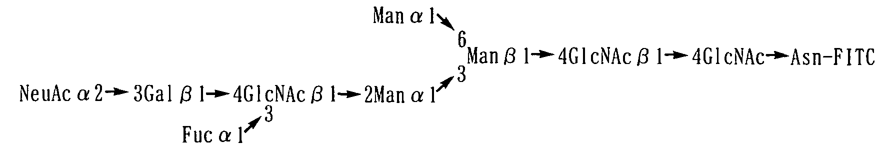

### Example 101

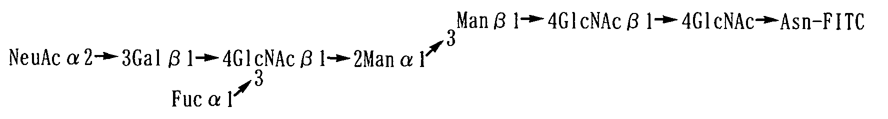

### Example 102

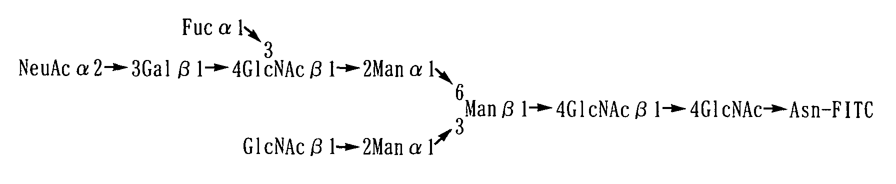

### Example 103

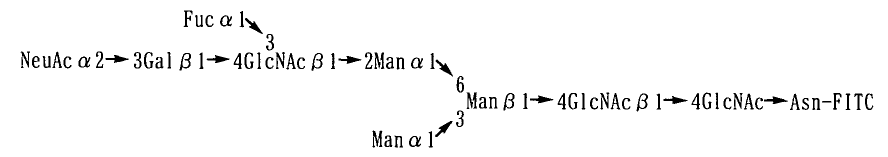

### Example 104

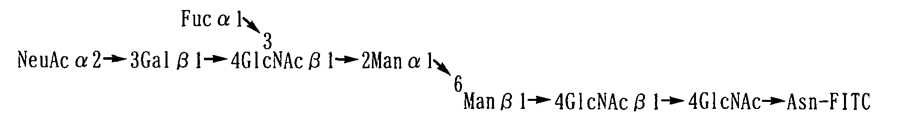

### Example 105 (Preparation of microplate)

The biotinated asparagine-linked oligosaccharide of Example 1 in an amount of 100 µg (corresponding to about 10 times the biotin bonding ability) was dissolved water to obtain 1000 µl solution. The solution thus adjusted was placed into the wells of 96-well BD BioCoat Streptavidin (product of BD Bioscience Corp. for bioassay, bonding ability: 5 ng/well) in an amount of 10 µl/well, and the wells were washed with distilled water three times to prepare a microplate. The bonding yield of the biotinated oligosaccharide was at least 95%. The fiwation rate was calculated from the quantity of oligosaccharide remaining unfixed.

### Example 106 (Preparation of affinity column)

Avidin-coated beads (product of Hitachi Software Enginearing Co., Ltd., xMAP LaumAvidin Development Microspheres 1 ml) in an amount of 10 ml and 30 mg of biotinated asparagine -linked oligosaccharide of Example 1 were stirred in the form of a slurry, and the beads were filtered off and washed. Washing was done using distilled water in an amount twice the volume of the beads, three times on filter paper. Fixation was confirmed from the amount of remaining biotinated oligosaccharide collected by washing. Subsequently, 10 ml of beads having the biotinated asparagine-linked oligosaccharide fixed thereto and 30 ml of distilled water were packed in the form of a slurry into an open chromatographic glass column (20 mm in diameter, 300 mm in length) to produce an affinity column.

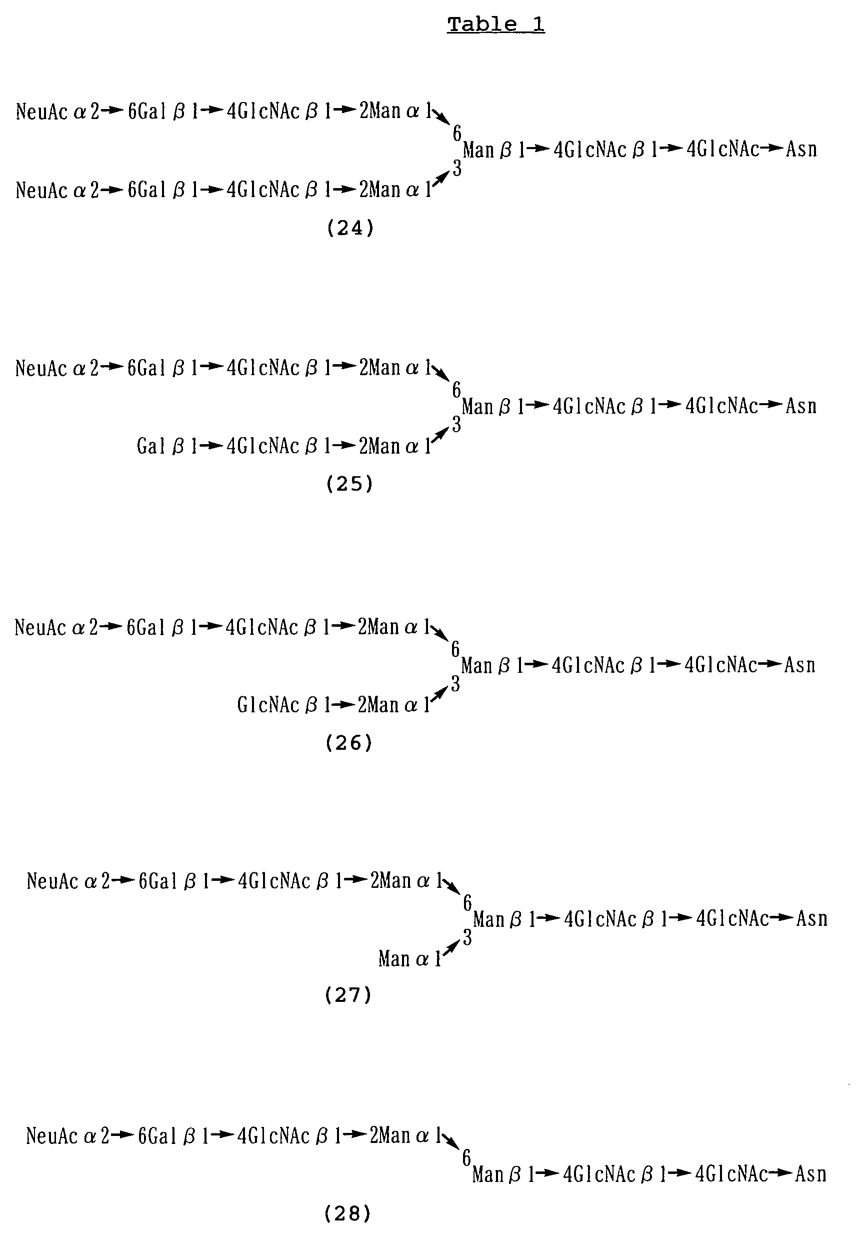

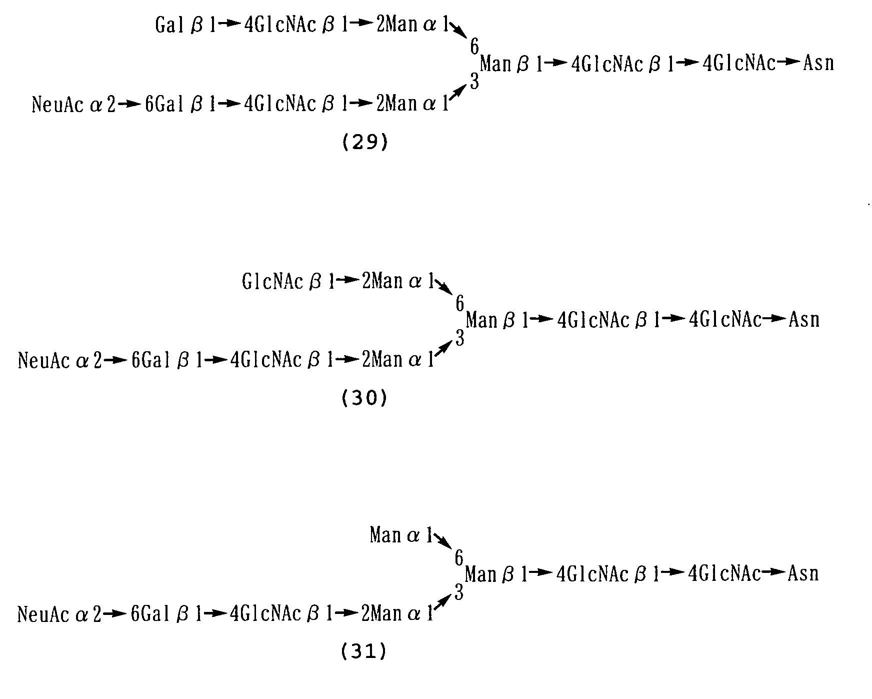

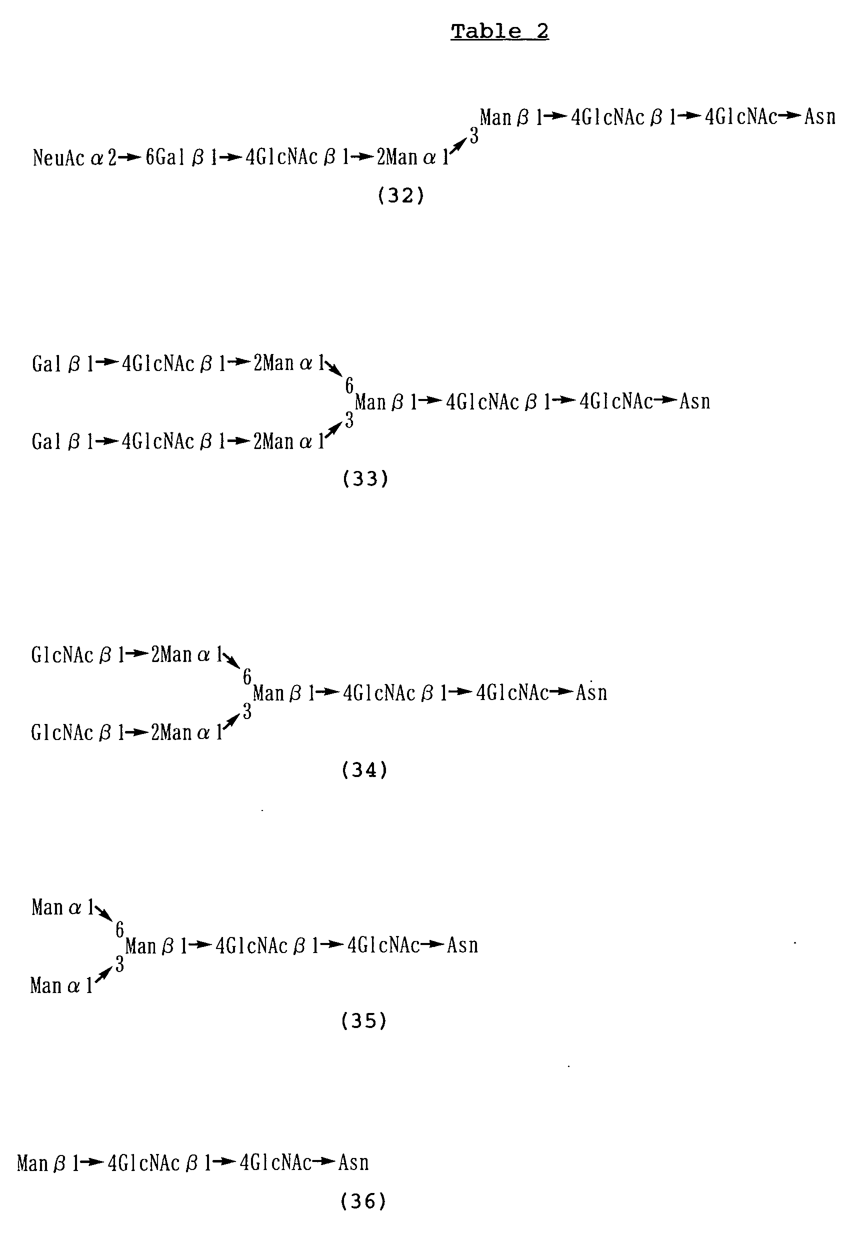

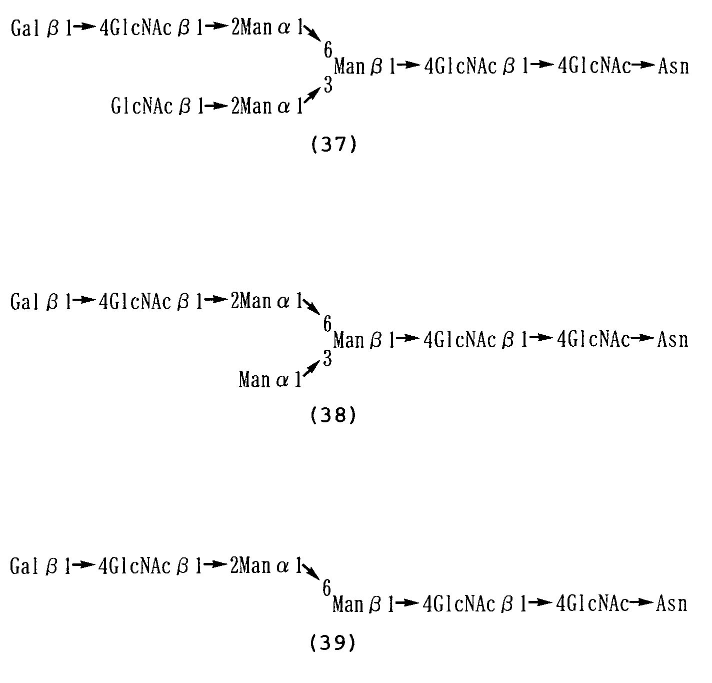

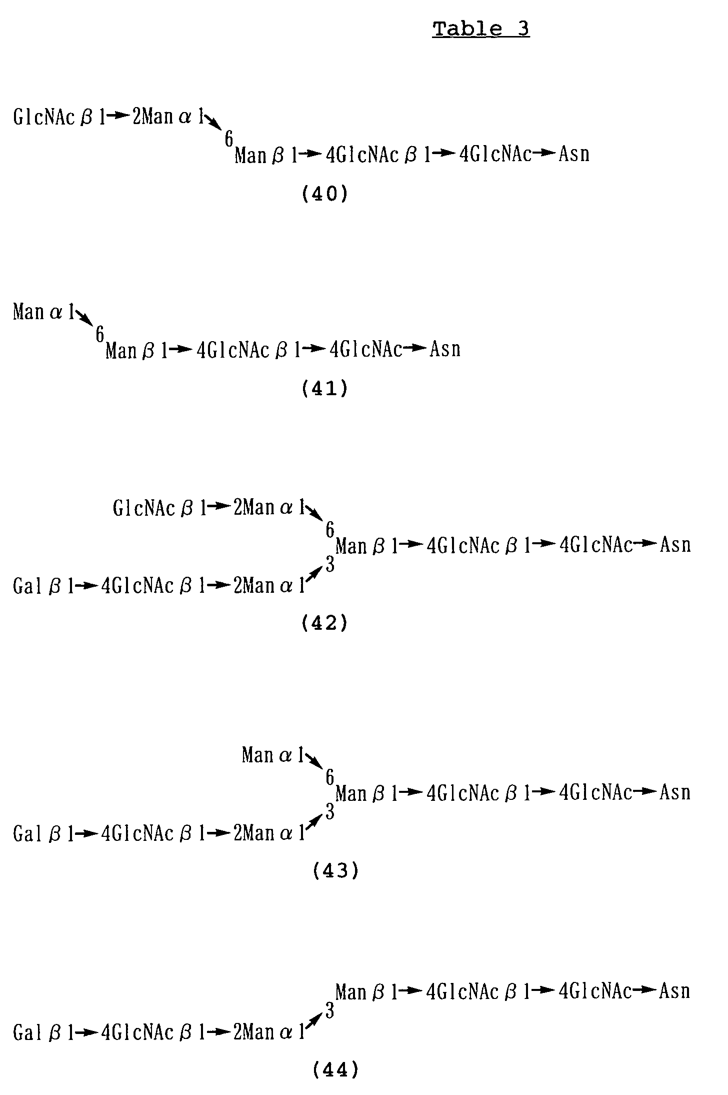

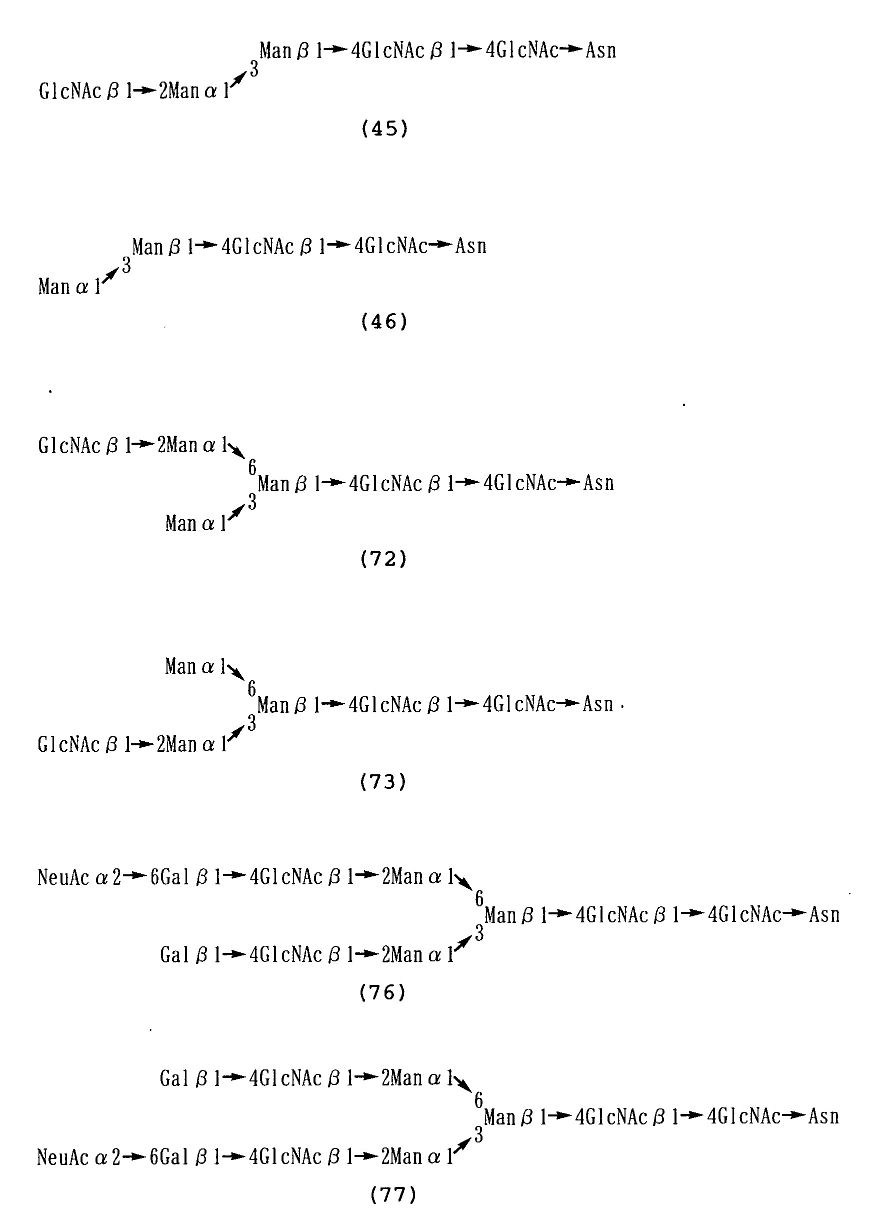

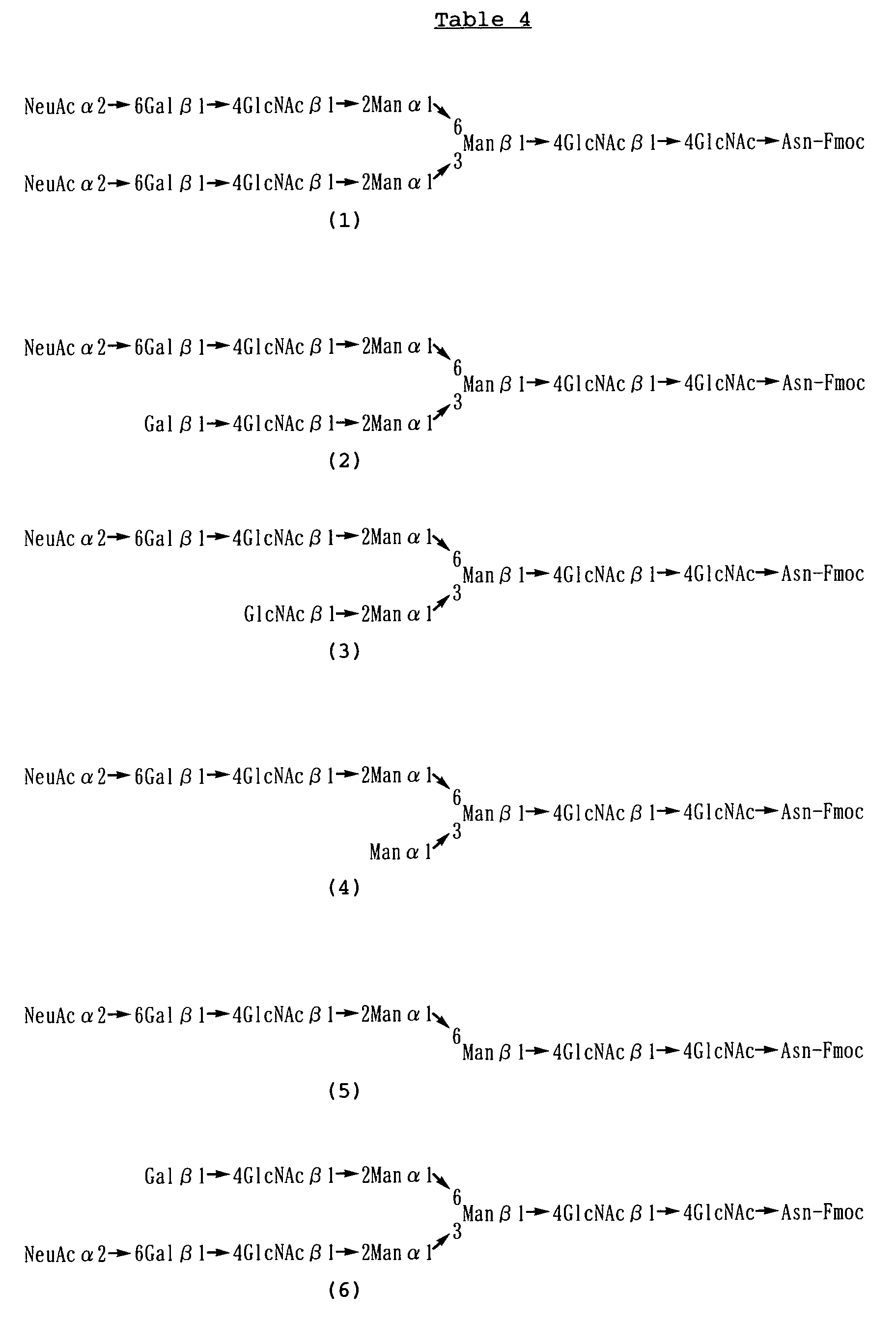

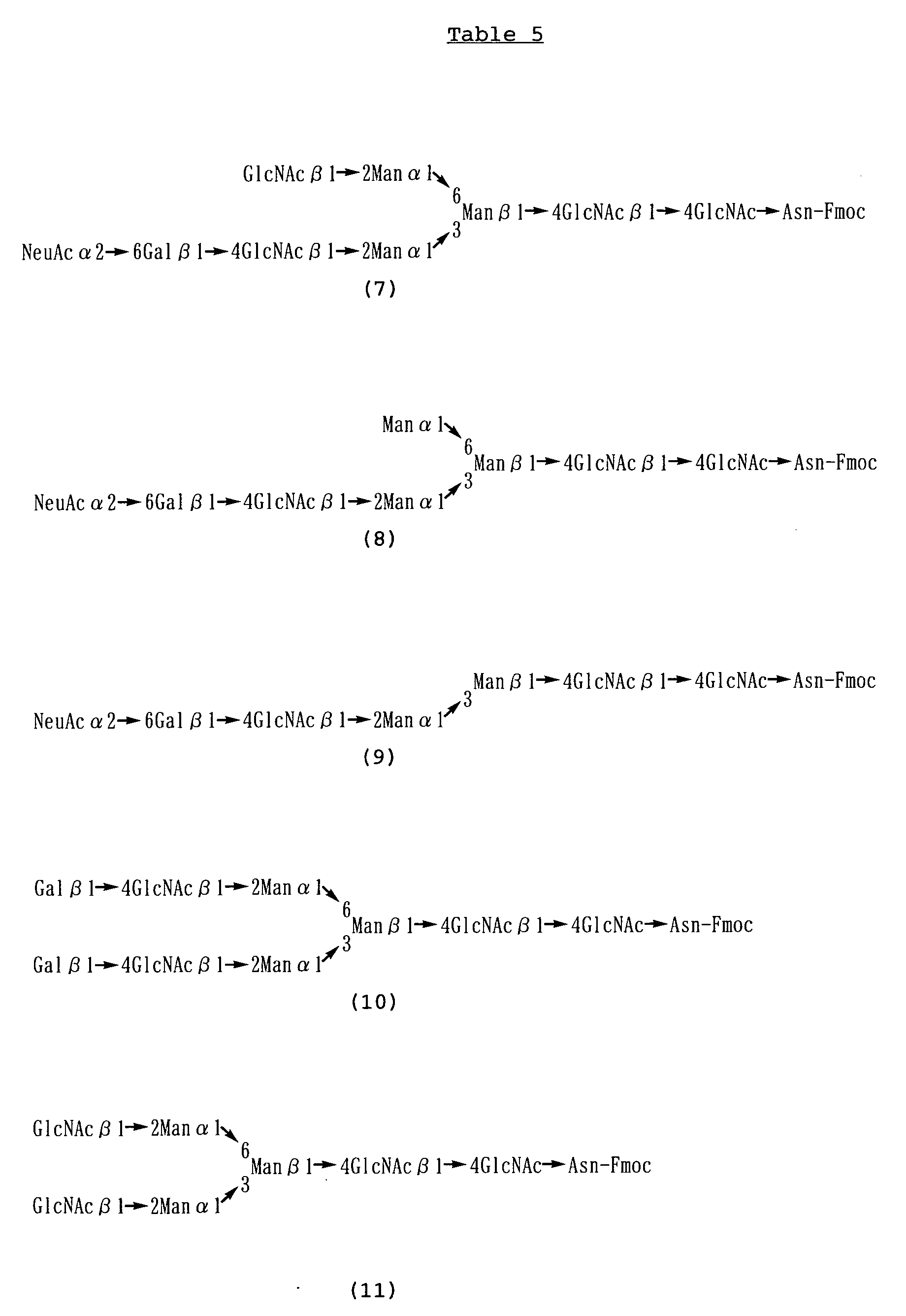

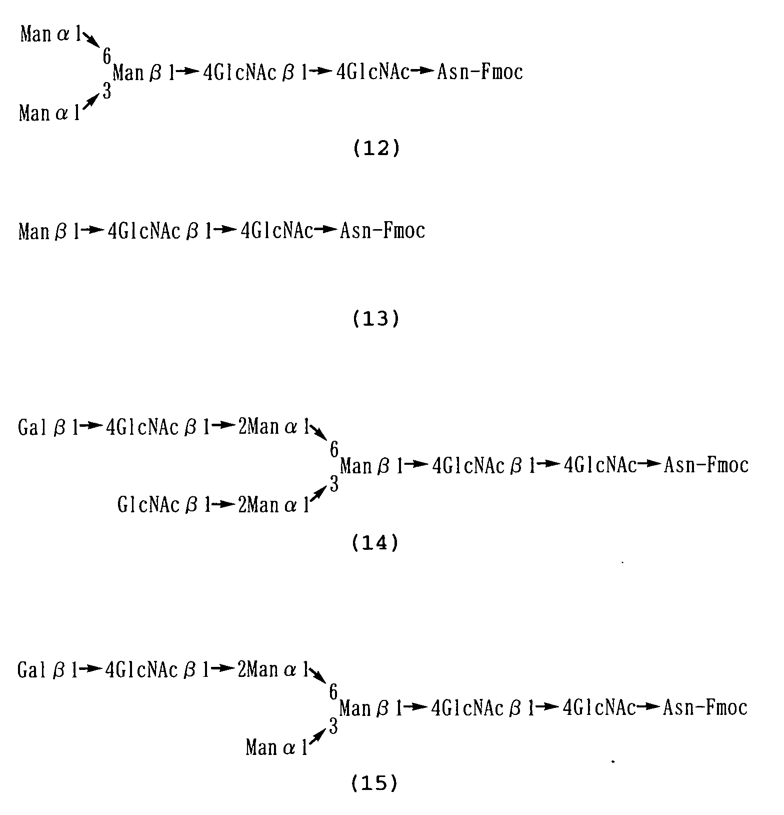

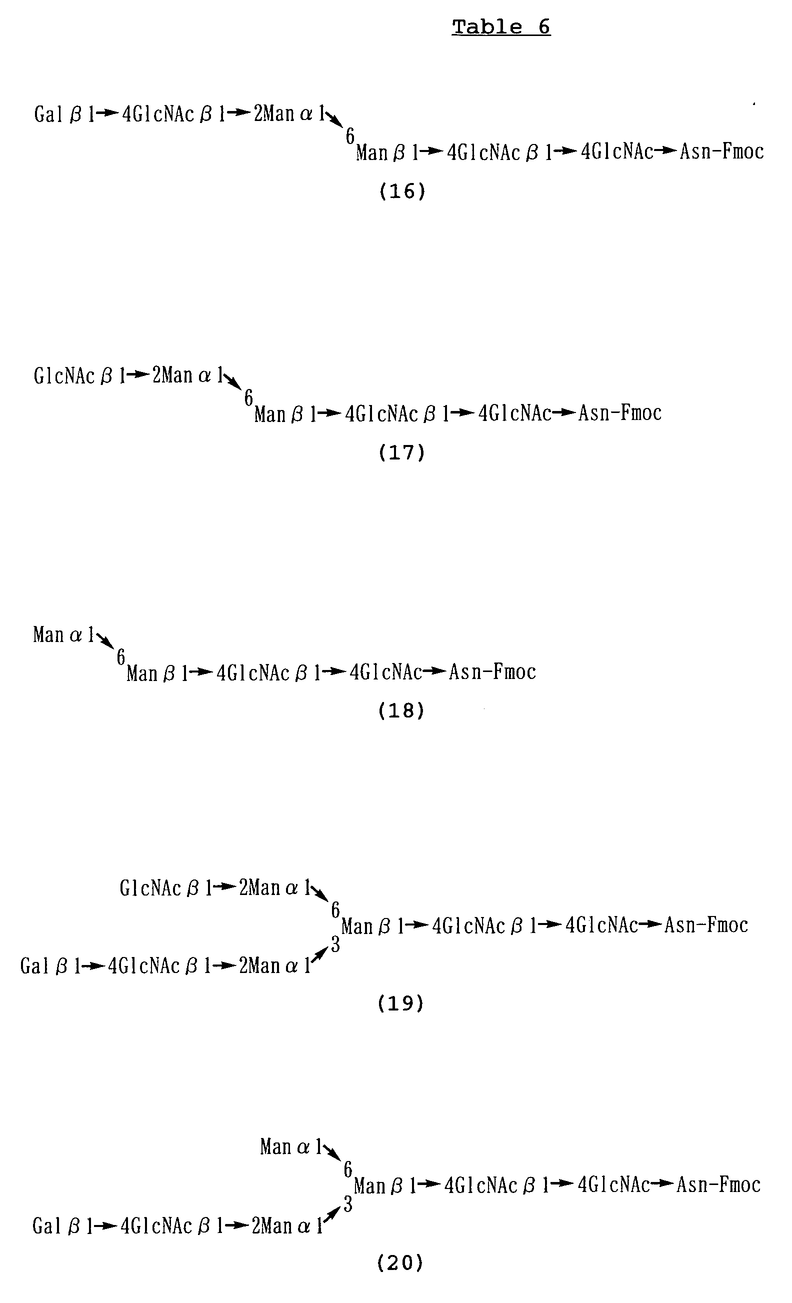

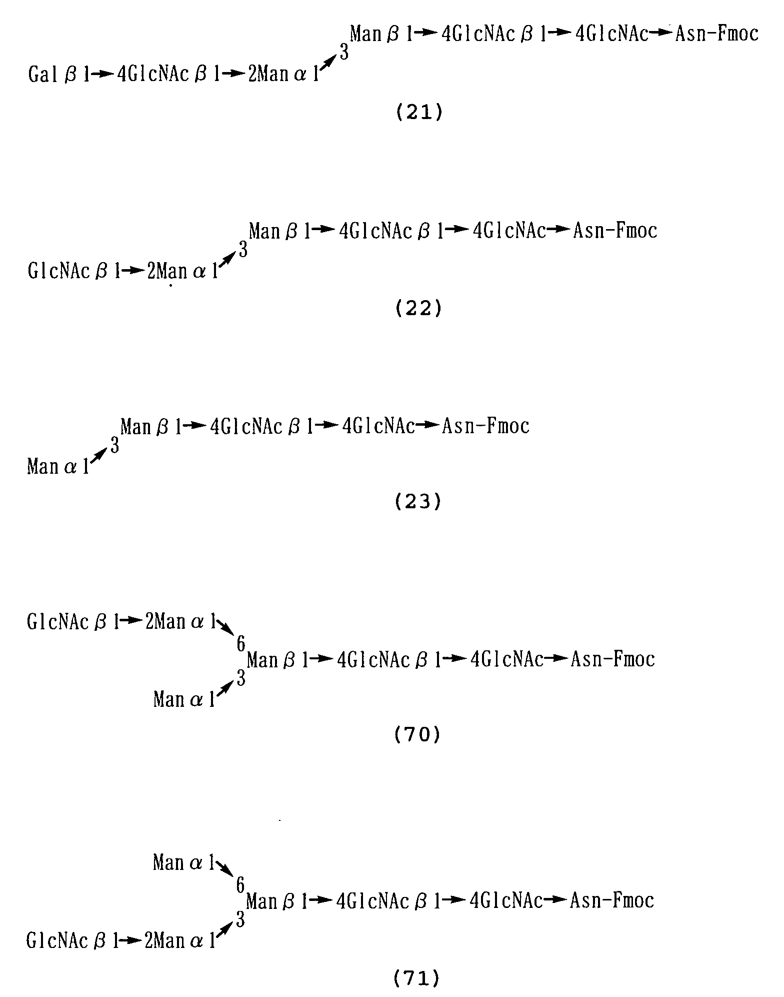

### INDUSTRIAL APPLICABILITY

The invention provides an asparagine-linked oligosaccharide wherein the amino group of asparagine is biotinated or bonded to FITC.

Utilizing the specificity of biotin-avidin bond, the invention further provides an oligosaccharide microchip easily merely by reacting a plurality of biotinated oligosaccharides on an avidinated microplate, whereby protens can be clarified which have ability to bond to a specific oligosaccharide.

In order to isolate and purify a specific protein, a specific biotinated oligosaccharide is bonded and immobilized to an avidinated affinity column, and a mixture containing a protein having ability to specifically bond to the biotinated oligosaccharide is passed through the column, whereby the desired protein only can be isolated.

The FITC-bonded asparagine-linked oligosaccharide obtained by the invention is useful for the research on acceptors of saccharides in the living body tissues and for the research on the sugar bond specificity of lectin.

## Claims

1. An asparagine-linked oligosaccharide of the formula (1) given below having undeca- to tri-saccharides 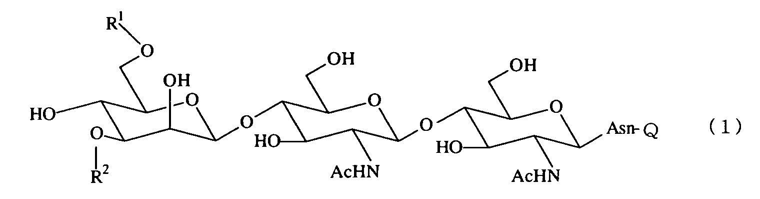 wherein R¹ and R² are each a hydrogen atom or a group of the formulae (2) to (6) and may be the same or different, and Q is a biotin group or FITC group.

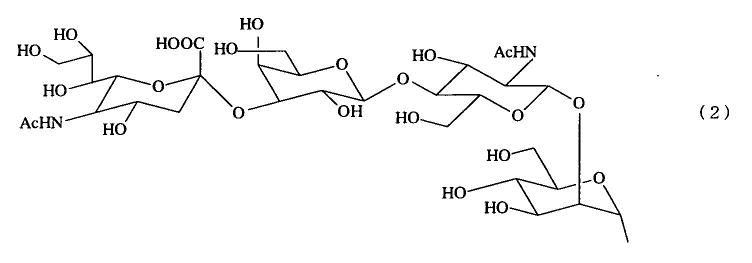

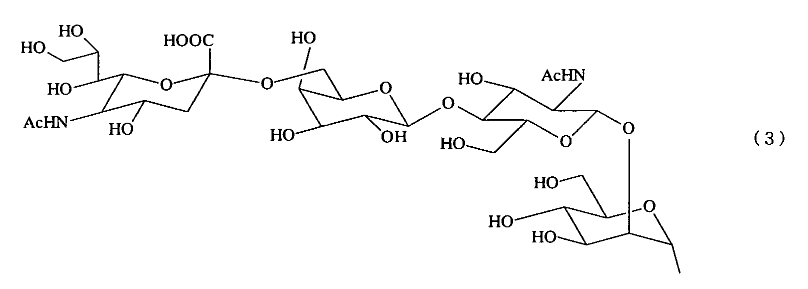

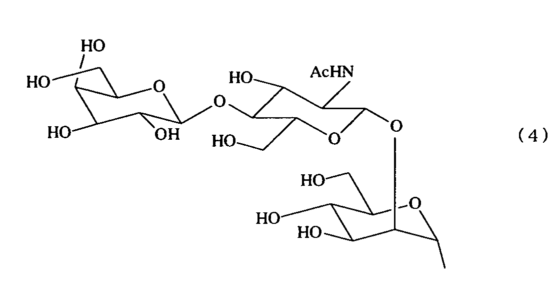

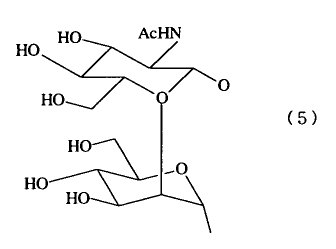

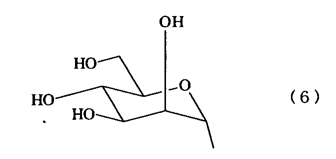

2. An asparagine-linked (α2,3) or (α2,6) oligosaccharide derivative having undeca- to hepta-saccharides and represented by the formula (1) wherein one of R¹ and R² is always a group of the formula (2) or (3).

3. An asparagine-linked (α2,3) (α2,6) oligosaccharide derivative having undecasaccharide and represented by the formula (1) wherein R¹ is a group of the formula (2), and R² is a group of the formula (3).

4. An asparagine-linked (α2,3) (α2,6) oligosaccharide derivative having undecasaccharide and represented by the formula (1) wherein R¹ is a group of the formula (3), and R² is a group of the formula (2).

5. An asparagine-linked oligosaccharide derivative containing at least one fucose in N-acetylglucosamine on the nonreducing terminal side of an asparagine-linked oligosaccharide wherein the amino group of asparagine is modified with a biotin group or FITC group.

6. An asparagine-linked oligosaccharide derivative containing fucose and according to claim 5 wherein the asparagine-linked oligosaccharide having a biotin group or FITC group modifying the amino group of asparagine is an asparagine-linked oligosaccharide derivative of the formula (1) having undeca- to tri-saccharides.

7. An asparagine-linked oligosaccharide derivative containing fucose and according to claim 5 wherein the asparagine-linked oligosaccharide having a biotin group or FITC group modifying the amino group of asparagine is an asparagine-linked (α 2,3) (α2,6) oligosaccharide derivative according to claim 3 and having undecasaccharide.

8. An asparagine-linked oligosaccharide derivative containing fucose and according to claim 5 wherein the asparagine-linked oligosaccharide having a biotin group or FITC group modifying the amino group of asparagine is an asparagine-linked (α 2,3) (α2,6) oligosaccharide derivative according to claim 4 and having undecasaccharide.

9. An asparagine-linked oligosaccharide derivative containing fucose and according to claim 5 wherein the asparagine-linked oligosaccharide having a biotin group or FITC group modifying the amino group of asparagine is an asparagine-linked α 2,3 oligosaccharide derivative having undeca- to hexa-saccharides and represented by the formula (1) wherein R¹ and R² are each a hydrogen atom, a group of the formula (2) or a group of the formulae (4) to (6), and one of R¹ and R² is always a group of the formula (2) or (4).

10. An asparagine-linked oligosaccharide derivative containing fucose and according to claim 5 wherein the asparagine-linked oligosaccharide having a biotin group or FITC group modifying the amino group of asparagine is an asparagine-linked α 2,6 oligosaccharide derivative having undeca- to hexa-saccharides and represented by the formula (1) wherein R¹ and R² are each a hydrogen atom, a group of the formula (3) or a group of the formulae (4) to (6), and one of R¹ and R² is always a group of the formula (3) or (4).

11. A process for preparing a biotinated asparagine-linked oligosaccharide **characterized in that** an asparagine-linked oligosaccharide of the formula (7) having undeca- to tri-saccharides is biotinated 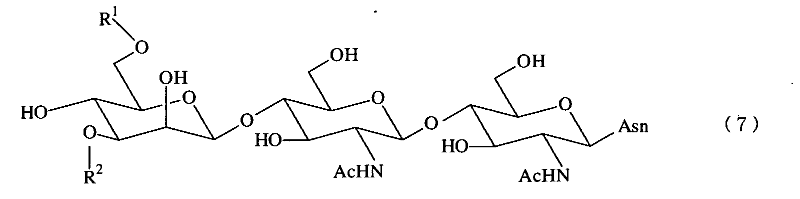 wherein R¹ and R² are as defined above.

12. A process for preparing a FITC-bonded asparagine-linked oligosaccharide **characterized in that** an asparagine-linked oligosaccharide of the formula (7) having undeca- to tri-saccharides is fluorescein isothiocyanated (FITC-bonded).

13. A microplate having immobilized thereto a biotinated asparagine-linked oligosaccharide according to claims 1 to 10.

14. An affinity column having immobilized thereto a biotinated asparagine-linked oligosaccharide according to claims 1 to 10.
